(19) Europäisches Patentamt
European Patent Office
Office européen des brevets

(11) Veröffentlichungsnummer: **0 450 566 B1**

(12) **EUROPÄISCHE PATENTSCHRIFT**

(45) Veröffentlichungstag der Patentschrift: **05.07.95**

(21) Anmeldenummer: **91105178.7**

(22) Anmeldetag: **02.04.91**

(51) Int. Cl.$^6$: **C07D 409/06**, C07D 403/06, C07D 471/04, C07D 487/04, C07D 413/06, A61K 31/40, A61K 31/415, A61K 31/41

# (54) Substituierte Azole, Verfahren zu deren Herstellung, diese enthaltende Mittel und deren Verwendung.

(30) Priorität: **04.04.90 DE 4010797**

(43) Veröffentlichungstag der Anmeldung: **09.10.91 Patentblatt 91/41**

(45) Bekanntmachung des Hinweises auf die Patenterteilung: **05.07.95 Patentblatt 95/27**

(84) Benannte Vertragsstaaten: **AT BE CH DE DK ES FR GB GR IT LI LU NL SE**

(56) Entgegenhaltungen:
**EP-A- 0 028 834**
**EP-A- 0 033 094**
**EP-A- 0 054 233**
**EP-A- 0 107 618**
**EP-A- 0 253 310**
**EP-A- 0 266 890**
**EP-A- 0 293 978**
**EP-A- 0 298 921**
**EP-A- 0 313 397**
**EP-A- 0 323 841**
**EP-A- 0 324 377**
**EP-A- 0 409 332**
**EP-B- 0 036 713**
**AT-B- 44 957**
**AT-B- 53 390**

(73) Patentinhaber: **HOECHST AKTIENGESELL-SCHAFT**
**D-65926 Frankfurt (DE)**

(72) Erfinder: **Wagner, Adalbert, Dr.**
**Kleiststrasse 9**
**W-6238 Hofheim am Taunus (DE)**
Erfinder: **Henning, Rainer, Dr.**
**Lorsbacher Strasse 4g**
**W-6234 Hattersheim am Main (DE)**
Erfinder: **Gerhards, Hermann, Dr.**
**Wacholderweg 4**
**W-6238 Hofheim am Taunus (DE)**
Erfinder: **Schölkens, Bernward, Dr.**
**Hölderlinstrasse 62**
**W-6233 Kelkheim/Taunus (DE)**
Erfinder: **Jean-Claude Caille**
**57 rue Michelet**
**F-49000 Angers (FR)**
Erfinder: **Jean-Paul VEVERT**
**51, rue Rouget De l'Isle**
**93500 PANTIN (FR)**

**EP 0 450 566 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus EP-A-324377, EP-A-253310, EP-A-28834 und EP-A 323841 sind Derivate von Imidazol, Pyrrol, Pyrazol bzw. Triazol und deren Verwendung als Antagonisten von Angiotensin-II-Rezeptoren bekannt.

Es wurden nun neue Verbindungen vom Azol-Typ gefunden, die überraschenderweise hochwirksame Antagonisten von Angiotensin-II-Rezeptoren sowohl in vitro als in vivo sind.

Die Erfindung betrifft Verbindungen der Formel I,

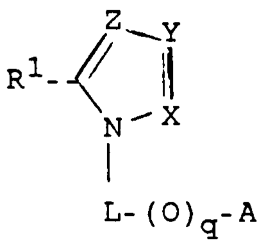

(I)

in welcher

a) X, Y und Z gleich oder verschieden sind und N oder $CR^2$ bedeuten,

b) $R^1$

1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

bedeutet;

c) $R^2$

1. Wasserstoff,
2. Halogen,
3. Nitro,
4. $C_vF_{2v+1}$,
5. Pentafluorphenyl,
6. Cyano,
7. Phenyl,
8. Phenyl-$(C_1-C_3)$-alkyl,
9. $(C_1-C_{10})$-Alkyl,
10. $(C_3-C_{10})$-Alkenyl,
11. Phenyl-$(C_2-C_6)$-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,

21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,
25.

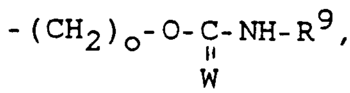

26.

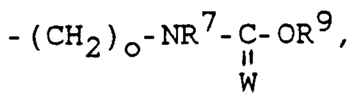

27. $-(CH_2)_o-NR^7-CO-NHR^9$,
28. $-(CH_2)_o-NR^7-SO_2R^9$,
29.

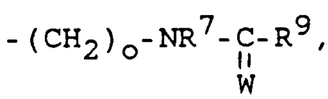

30. $-(CH_2)_nF$,
31. $-(CH_2)_n-O-NO_2$,
32. $-CH_2-N_3$,
33. $-(CH_2)_n-NO_2$,
34. $-CH=N-NR^5R^7$,
35. Phthalimido-$(CH_2)_n$-,
36.

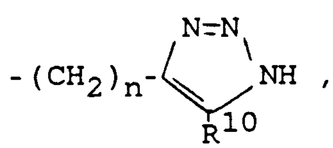

37.

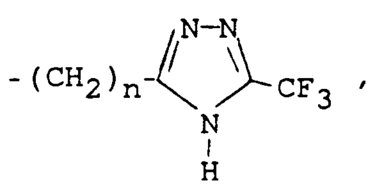

38.

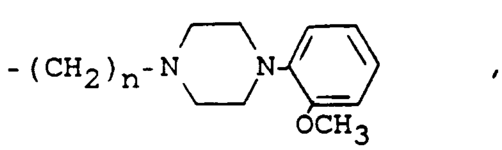

39.

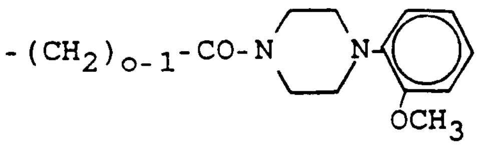
,

40. Phenyl-$SO_2$-NH-N = CH-,

41.

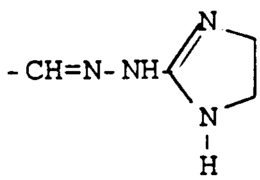
,

42. -$(CH^2)_n$-$SO_2$-$NR^7$-CO-$NR^6R^9$,

43. -$(CH_2)_o$-$SO_2R^9$,

44. einem wie unter c) 7. oder 8. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,

45. einem wie unter c) 9., 10. oder 18 definierten Rest, worin 1 bis alle H-Atome durch Fluor substituiert sind, oder

46. den unter c) 13. definierten Rest, der mit 1 oder 2 gleichen oder verschiedenen Resten aus der Reihe Methoxycarbonyl und ($C_1$-$C_4$)-Alkyl substituiert ist,

bedeutet;

d) $R^3$

1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,

bedeuten;

e) $R^4$

1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl

bedeutet;

f) $R^5$

1. Wasserstoff,
2. ($C_1$-$C_6$)-Alkyl,
3. ($C_3$-$C_8$)-Cycloalkyl,
4. Phenyl oder
5. Benzyl

bedeutet;

g) $R^6$

1. Wasserstoff,
2. ($C_1$-$C_6$)-Alkyl,
3. ($C_3$-$C_8$)-Cycloalkyl,
4. ($C_6$-$C_{12}$)-Aryl,
5. Benzyl,

6. ($C_1$-$C_9$)-Heteroaryl, der teilweise oder vollständig hydriert sein kann und der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können.

7. ($C_1$-$C_4$)-Alkanoyl,

8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ Trifluormethyl, $NR^{11}R^{12}$ oder

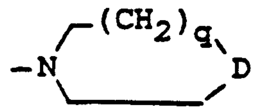

bedeutet;

9. ($C_1$-$C_9$)-Heteroaryl-($C_1$-$C_3$)-Alkyl, wobei der Heteroarylteil wie unter g) 6 definiert ist;

h) $R^7$

1. Wasserstoff,
2. ($C_1$-$C_6$)-Alkyl,
3. ($C_3$-$C_8$)-Cycloalkyl,
4. ($C_6$-$C_{12}$)-Aryl-($C_1$-$C_6$)-alkyl,
5. Phenyl oder
6. ($C_1$-$C_9$)-Heteroaryl der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können , bedeutet

i) $R^8$

1. Wasserstoff,
2. ($C_1$-$C_6$)-Alkyl,
3. ($C_3$-$C_8$)-Cycloalkyl,
4. Phenyl-$(CH_2)_q$-,
5. $OR^5$,
6. $NR^{11}R^{12}$ oder
7.

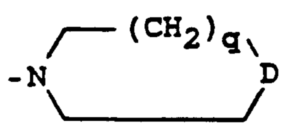

bedeutet;

j) $R^9$

1. ($C_1$-$C_6$)-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind, bedeutet;

k) $R^{10}$ Cyano, Nitro oder $CO_2R^7$ bedeutet;

l) $R^{11}$ und $R^{12}$ gleich oder verschieden sind und

1. Wasserstoff,
2. ($C_1$-$C_4$)-Alkyl,
3. Phenyl,
4. Benzyl oder

5. α-Methylbenzyl
bedeuten;

m) D $NR^{13}$, O oder $CH_2$ bedeutet;

n) $R^{13}$ Wasserstoff, $(C_1-C_4)$Alkyl oder Phenyl bedeutet;

o) A den Rest eines anellierten Heterobicyclus mit 8 bis 10 Ringatomen, wovon bis zu 9 Ringatome C-Atome sind, worin zwei nebeneinanderliegende Atome gemeinsame Bestandteile beider Ringe sind, wobei einer oder beide dieser Ringe sich formal vom Benzol ableiten, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind, bedeutet;
der mit bis zu 6 gleichen oder
verschiedenen Resten $R^{14}$ oder
$-(CH_2)_{n-1}-(CHR^6-CH_2)_{o-1}-R^{15}$ substituiert sein kann,

(p) $R^{14}$

1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R^3$,
12. Methansulfonylamino,
13. Trifluormethansulfonylamino,
14. $-CO-NH-OR^9$,
15. $-SO_2-NR^6R^7$,
16. $-CH_2-OR^7$,
17. $(C_1-C_9)$-Heteroaryl-$(CH_2)_q$-, wobei der Heteroarylteil wie unter h) 6. definiert ist,
18. $(C_7-C_{13})$-Aroyl,
19.

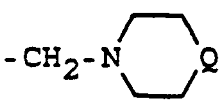

,

20.

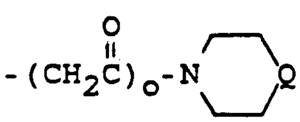

oder

21. $(C_6-C_{12})$-Aryl

bedeutet;

q) $R^{15}$

1. Wasserstoff,
2. $(C_1-C_6)$-Alkyl,
3. $(C_3-C_8)$-Cycloalkyl,
4. $(C_6-C_{12})$-Aryl,
5. $(C_7-C_{13})$-Aroyl,
6. $(C_1-C_4)$-Alkoxy,
7. $(C_1-C_4)$-Alkanoyloxy,
8. $(C_1-C_9)$-Heteroaryl, der wie unter h) 6. definiert ist,
9. $CO_2R^3$,

10. Halogen,
11. Cyano
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. Phosphonooxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. den unter p) 20. definierten Rest,
29.

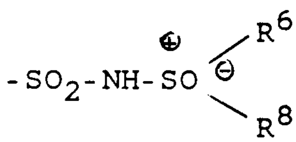

,

30.

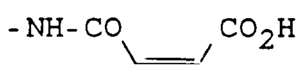

,

31.

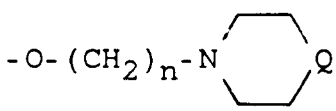

,

32. 5-Tetrazolyl-NH-CO-,
33. $-CO-NH-NH-SO_2CF_3$,
34.

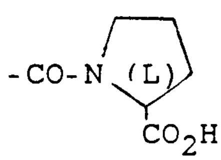

,

35.

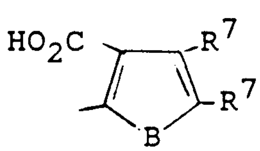

,

36.

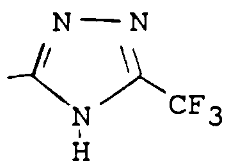

,

37.

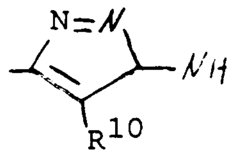

,

38.

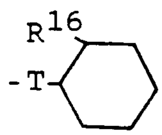

,

39.

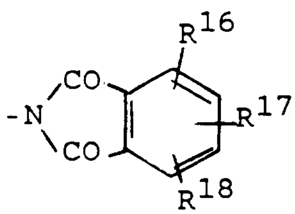

,

40. $-CO-NH-SO_2-R^{19}$ oder

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r) B O, $NR^7$ oder S bedeutet;

s) W O oder S bedeutet;

t) L $(C_1-C_3)$-Alkandiyl bedeutet;

u) $R^{16}$ $CO_2R^3$ oder $CH_2CO_2R^3$ bedeutet;

v) $R^{17}$ Wasserstoff, Halogen, $(C_1-C_4)$-Alkyl oder $(C_1-C_4)$-Alkoxy bedeutet;

w) $R^{18}$ Wasserstoff, $(C_1-C_4)$-Alkyl oder Phenyl bedeutet;

x) $R^{19}$

1. $(C_1-C_8)$-Alkyl,
2. $(C_3-C_8)$-Cycloalkyl
3. Phenyl
4. Benzyl oder
5. den unter x) 1. definierten Rest, worin 1 bis alle H-Atome durch Fluor oder Chlor substituiert sind, bedeutet;

y) T

1. eine Einfachbindung,
2. -CO-
3. $-CH_2-$
4. -O-
5. -S-

6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. $-CH=CH-$
18. $-CF=CF-$,
19. $-CH=CF-$,
20. $-CF=CH-$,
21. $-CH_2-CH_2-$,
22. $-CF_2-CF_2-$,
23. $-CH(OR^3)-$
24. $-CH(OCOR^5)-$
25.

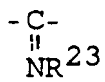

oder
26.

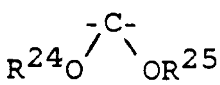

bedeutet;

z) $R^{20}$ und $R^{22}$ gleich oder verschieden sind und Wasserstoff, $(C_1-C_5)$-Alkyl, Phenyl, Allyl oder Benzyl bedeutet;

a') $R^{21}$ Wasserstoff, $(C_1-C_6)$Alkyl, Benzyl oder Allyl bedeutet;

b') $R^{23}$
1. $NR^{20}R^{21}$
2. Ureido,
3. Thioureido,
4. Toluol-4-sulfonyl oder
5. Benzolsulfonylamino

c') $R^{24}$ und $R^{25}$ gleich oder verschieden sind und $(C_1-C_4)$-Alkyl bedeuten oder gemeinsam für $-(CH_2)_q-$ stehen;

d') Q $CH_2$, NH, O oder S bedeutet;

e') m eine ganze Zahl von 0 bis 5 ist,

f') n eine ganze Zahl von 1 bis 5 ist;

g') o eine ganze Zahl von 1 bis 10 ist;

h') q 0 oder 1 ist,

i') r 0, 1 oder 2 ist, oder

j') v eine ganze Zahl von 1 bis 6 ist;

sowie deren physiologisch verträglichen Salze.

Alkyl, Alkenyl und Alkinyl können geradkettig oder verzweigt sein. Entsprechendes gilt für davon abgeleitete Reste, wie Alkanoyl oder Alkoxy.

$(C_6-C_{12})$-Aryl ist beispielsweise Phenyl, Naphthyl oder Biphenylyl vorzugsweise Phenyl. Entsprechendes gilt für davon abgeleitete Reste, wie Aroyl oder Aralkyl.

Unter ($C_1$-$C_9$)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Weiter kann auch 1 oder beide Atome der Kondensationsstelle bicyclischer Reste (wie im Indolizinyl) ein N-Atom sein.

Dies sind z.B. Euranyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Unter dem anelliertem Heterobicyclus AH, von dem der Rest A abgeleitet ist, versteht man insbesondere ein bicyclisches Ringsystem mit 8 bis 10 Ringatomen, wovon bis zu 9 Ringatome C-Atome sind, worin zwei nebeneinanderliegende Atome gemeinsame Bestandteile beider Ringe sind. Einer oder beide dieser Ringe leiten sich formal vom Benzol ab, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind.
A ist beispielsweise ein Rest von Benzothiophen, Benzofuran, Indol, Isoindol, Indazol, Benzimidazol, Chinolin, Isochinolin, Phthalazin, Chinoxalin, Chinazolin, Cinnolin, Benzthiazol, Benzothiazol-1,1-dioxid, Cumarin, Chroman, Benzoxazol, Benzisothiazol, Benzodiazine, Benztriazol, Benztriazin, Benzoxazin. Imidazo-pyridin, Imidazo-pyrimidin, Imidazo-pyrazin, Imidazo-pyridazin, Imidazo-thiazol, Pyrazolopyridin, Thienopyridin und Pyrrolopyrimidin. Der genannte Heterobicyclus AH kann auch teilweise oder ganz hydriert sein. Vorzugsweise bleibt aber ein Ring von AH aromatisch, wobei ein benzanellierter Heterobicyclus AH besonders bevorzugt ist.

Im Falle von S-haltigen und/oder teilgesättigten Resten kann der Bicyclus z.B. auch oxosubstituiert sein, wie es beim Rest des Benz-1,2,3-triazinon der Fall ist.

Die Verknüpfung von A erfolgt vom isocyclischen oder vom heterocyclischen Teil aus über eine Alkandiyl-Brücke L.

Unter physiologisch verträglichen Salzen von Verbindungen der Formel I versteht man sowohl deren organische als auch anorganische Salze, wie sie in Remington's Pharmaceutical Sciences, 17. Auflage, Seite 1418 (1985) beschrieben sind. Aufgrund von physikalischer und chemischer Stabilität und Löslichkeit sind für saure Gruppen unter anderem Natrium-, Kalium-, Calcium- und Ammoniumsalze bevorzugt; für basiche Gruppen unter anderen Salze mit Salzsäure, Schwefelsäure, Phosphorsäure, Carbonsäuren oder Sulfonsäuren, wie Essigsäure, Zitronensäure, Benzoesäure, Maleinsäure, Fumarsäure, Weinsäure, p-Toluolsulfonsäure.

Bevorzugt sind Verbindungen der Formel I, worin
a) X N, Y $CR^2$ und Z $CR^2$ bedeuten;
b) X $CR^2$, Y N und Z $CR^2$ bedeuten;
c) X $CR^2$, Y $CR^2$ und Z N bedeuten
oder
d) X, Y und Z jeweils N bedeuten.

Weiter sind Verbindungen der Formel I bevorzugt in welcher
a) $R^1$
1. ($C_3$-$C_{10}$)-Alkyl,
2. ($C_3$-$C_{10}$)-Alkenyl,
3. ($C_3$-$C_{10}$)-Alkinyl,
4. ($C_3$-$C_8$)-Cycloalkyl,
5. Benzyl oder
6. Benzyl, das wie im Anspruch 1 definiert substituiert ist,
bedeutet;
b) $R^2$
1. Wasserstoff,
2. Halogen,
3. Nitro,
4. $C_v F_{2v+1}$
5. Pentafluorphenyl
6. Cyano
7. Phenyl,
8. Phenyl-($C_1$-$C_3$)-alkyl,
9. ($C_1$-$C_{10}$)-Alkyl,
10. ($C_3$-$C_{10}$)-Alkenyl,

11. Phenyl-($C_2$-$C_6$)-alkenyl,
12. 1-Imidazolyl-$(CH_2)_m$-
13. 1,2,3-Triazolyl-$(CH_2)_o$-
14. Tetrazolyl-$(CH_2)_m$-
15. -$(CH_2)_{o-1}$-$CHR^7$-$OR^5$
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ oder
27. einem wie unter b) 7., 8., 9., 10. oder 13. definierten Rest, der wie oben unter c) 46., 45. oder 44. jeweils wie für einen solchen Rest beschrieben definiert substituiert ist,

bedeutet;

c) $R^8$ Wasserstoff; ($C_1$-$C_5$)-Alkyl, $OR^5$, $NR^{11}R^{12}$ oder Morpholino bedeutet;

d) T
1. eine Einfachbindung,
2. -CO-
3. -$CONR^{21}$-
4. -$CH_2$-$CH_2$-
5. -$NR^{21}$-CO-
6. -O-$CH_2$-
7. -$CH_2$-O-
8. -S-$CH_2$-
9. -$CH_2$-S-
10. -NH-$CH_2$-
11. -$CH_2$-NH- oder
12. -CH = CH-

bedeutet und die übrigen Reste und Variablen wie oben definiert sind sowie deren physiologisch verträglichen Salze.

Besonders bevorzugt sind Verbindungen der Formel I in welcher

a) $R^1$ ($C_3$-$C_7$)-Alkyl, ($C_3$-$C_7$)-Alkenyl oder ($C_3$-$C_7$)-Alkinyl bedeutet;

b) $R^2$
1. Chlor,
2. Brom,
3. $C_vF_{2v+1}$ mit v = 1,2 oder 3,
4. Pentafluorphenyl,
5. -$S(O)_rR^6$,
6. $(CH_2)_{o-1}$-$CHR^7$-$OR^5$,
7. $(CH_2)_o$-O-CO-$R^3$,
8. -$COR^8$,
9. -$(CH_2)_o$-CO-$R^8$,
10. -$CH_2$-NH-CO-$R^8$,
11. -$(CH_2)_o$-NH-$SO_2$-$R^9$,
12. -CH = CH-$CHR^3$-$OR^6$,
13. Tetrazolyl-$(CH_2)_m$-
14. -$(CH_2)_nSO_2$-NH-CO-$NR^6R^9$,
15. -$(CH_2)_o$-$SO_3R^9$ oder

gegebenenfalls durch Hydroxy substituiertes ($C_1$-$C_6$)-Alkyl,

bedeutet;

c) $R^3$ Wasserstoff oder ($C_1$-$C_4$)-Alkyl

bedeutet;

d) $R^6$ Wasserstoff, $(C_1-C_4)$-Alkyl, $(C_1-C_4)$-Alkanoyl, einen wie oben unter g) 4., 6. oder 9. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, oder $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, bedeutet;

e) $R^7$ Wasserstoff, $(C_1-C_4)$-Alkyl, $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, oder $(C_6-C_{12})$-Aryl-$(C_1-C_4)$-Alkyl bedeutet;

f) $R^8$ Wasserstoff, $(C_1-C_4)$-Alkyl, $OR^5$ oder Morpholino bedeutet;

g) $R^9$ $CF_3$, $(C_1-C_6)$-Alkyl oder Phenyl bedeutet;

h) $R^{14}$

1. $(C_1-C_4)$-Alkyl,
2. $(C_1-C_4)$-Alkoxy,
3. Cyano,
4. Amino,
5. Nitroso,
6. Nitro,
7. Fluor,
8. Chlor,
9. Brom,
10. Hydroxy,
11. $CH_2OR^7$,
12. $(C_1-C_9)$-Heteroaryl-$CH_2$-, wobei der Heteroarylteil sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
13. $(C_1-C_4)$-Alkanoyloxy,
14. $(C_1-C_4)$Alkanoyl,
15. Benzoyl,
16.

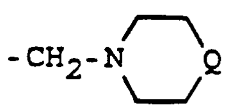

,

17. -NH-CO-$R^7$ oder
18. Tetrazolyl

bedeutet;

i) $R^{15}$

1. $(C_1-C_4)$-Alkyl,
2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
6. Cyano,
7. Nitro,
8. Hydroxy,
9. -S(O)$_r$$R^6$
10. -$SO_3R^3$

11. Chlor,
12. Brom,
13. Benzoyl,
14. $-CO_2R^3$,
15. $-CO-NH-R^6$,
16. $-NR^6R^7$,
17. $-CO-R^8$,
18. $-SO_2-NR^6R^7$,
19.

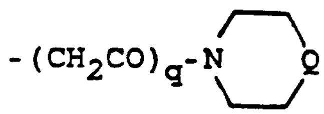
,

20.

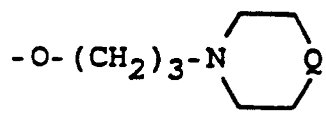
,

21. $-SO_2-NH-CO-NR^6R^9$,
22. $-PO_3H$
23. $-CO-CHR^5-CO_2H$,
24. $-NH-CO-NH-SO_2-CH_2-R^5$,
25. 5-Tetrazolyl-NH-CO-,
26.

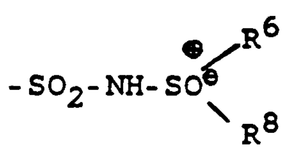
,

27.

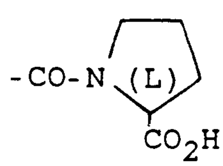
,

28.

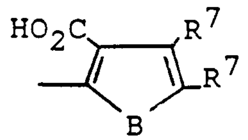
,

29.

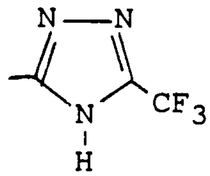

,

30.

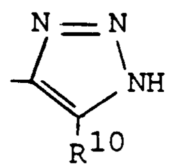

,

31.

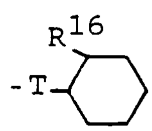

,

32.

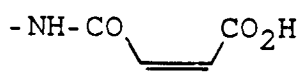

,

33.

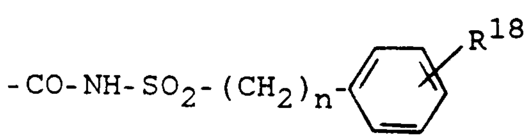

oder

34. den unter i) 2) definierten Rest, substituiert wie oben definiert,

bedeutet;

j) Q $CH_2$, NH oder O bedeutet;

k) $R^{18}$ Wasserstoff, Methyl oder Ethyl bedeutet;

l) T eine Einfachbindung, -O-, -CO-, -NHCO- oder $-OCH_2$-bedeutet

und die übrigen Reste und Variablen wie oben definiert sind.

Ganz besonders bevorzugt sind Verbindungen der Formel (I), wobei die Symbole $R^2$, $R^9$, $R^{14}$, $R^{15}$, Z, X, Y, L, q folgende Bedeutungen haben:

a) $R^2$ Chlor, Brom, $-S(O)_rR^6$, $-COR^8$ oder $-(CH_2)_nSO_2-NH-CO-NR^6R^9$;

b) $R^9$ $(C_1-C_6)$-Alkyl;

c) $R^{14}$ Tetrazolyl;

d) $R^{15}$ $-CO_2-R^3$, $-SO_2-NR^6R^7$, $-SO_2-NH-CO-NR^6R^9$ oder $-NH-CO-NH-SO_2-CH_2-R^5$;

e) Z gleich N;

f) X, Y beide $CR_2$ bedeuten;

g) q Null;

h) L $CH_2$;

Die Erfindung betrifft auch ein Verfahren zur Herstellung von Verbindungen der Formel I, das dadurch gekennzeichnet ist, daß man Verbindungen der Formel II

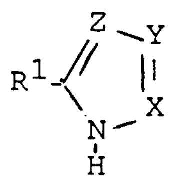

(II)

worin R[1], X, Y und Z wie oben definiert sind, alkyliert mit Verbindungen der Formel III

U-L-$(O)_q$-A (III)

worin L, A und q wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

Geeignete Fluchtgruppen U sind vorzugsweise eine nucleofuge Gruppe (vgl. Angew. Chem. 72 [1960] 71) wie Halogen, o-Toluolsulfonat, Mesylat oder Triflat.

Verfahren zur Herstellung der Vorstufen der Formel II sind u.a. bekannt aus US-Patent 4 355 044, EP-A-324 377 und EP-A-323 841.

Weitere Verfahren sind beschrieben in G. L'abbe, Chem. Rev. 69, 345 (1969); T. Srodsky in "The Chemistry of the Azido Group", Wiley, New York, 1971, S. 331; H. Wamhoff in "Comprehensive Heterocyclic Chemistry, S. Katritzky Ed., Pergamon Press, New York (1984).

Zur Alkylierung der Azole der Formel II sind z.B. entsprechnede Benzylhalogenide, -tosylate, -mesylate oder Triflate oder ensprechende Alkylhalogenide, -tosylate, mesylate oder-triflate geeignet.

Die Synthese dieser Derivate wie Benzofurane, Benzthiophene und Indole mit benzylischer $-CH_3$-Gruppe wurde unter anderem vom R.P. Dickson; et al. in J. Med. Chem. 29, 1637 (1986), und ibid. 29, 1643 (1986) beschrieben. Geeignete Hydroxybenzotriazole können nach R. Geiger et. al, Chem. Ber. 103, 788 (1970) hergestellt werden. Die Darstellung von Benzimidazolen, Benzothiazolen, Benzodiazinen, Benzopyronen, Benzothiazolonen, Benzotriazinen, Benzoxazinen, Benzoxazolen ist in der oben zitierten Ausgabe "Comprehensive Heterocyclic Chemistry", S. Katritzky Ed. Pergamon Press, New York (1984) skiziert. Weitere Heterocyclen waren nach E, Abignente et al. in J. Heterocyclic Chem. 26, 1875 (1989), A. Krubsack et al. in J. Org. Chem. 41, 3399, (1976) und nach F. Santer et al. in Mh. Chem. 99, 715 (1968) zugänglich.

Die Alkylierung erfolgt nach prinzipiell bekannten Verfahren in analoger Weise.

Das Azol-Derivat der Formel II wird z.B. in Gegenwart einer Base metalliert. Bevorzugte Basen sind Metallhydride der Formel MH wie Z.B. Lithium-, Natrium- oder Kaliumhydrid in beispielsweise DMF oder DMSO als Lösungsmittel oder Metallalkoxide der Formel MOR, wobei R für Methyl, Ethyl, t-Butyl steht und die Reaktion in dem entsprechenden Alkohol, DMF oder DMSO durchgeführt wird. Die so gebildeten Salze der Azole werden in einem aprotischen Lösungsmittel wie DMF oder DMSO gelöst und mit einer geeigneten Menge Alkylierungsreagenz versetzt.

Eine alternative Möglichkeit zur Deprotonierung der Azol-Derivate stellt z.B. die Umsetzung mit Kaliumcarbonat in DMF oder DMSO dar.

Die Umsetzungen werden bei Temperaturen unterhalb Raumtemperatur bis zum Siedepunkt des Reaktionsgemisches, vorzugsweise zwischen +20 °C und dem Siedepunkt des Reaktionsgemisches während etwa 1 bis 10 Stunden durchgeführt.

Die erfindungsgemäßen Verbindungen der Formel I haben antagonistische Wirkung auf Angiotensin-II-Rezeptoren und können deshalb zur Behandlung der Angiotensin-II-abhängige Hypertension verwendet werden. Anwendungsmöglichkeiten bestehen weiterhin bei Herzinsuffizienz, Kardioprotektion, Myocardinfarkt, Herz-Hypertrophie, Arteriosklerosis, Nephropathie, Nierenversagen sowie vasculären Erkrankungen des Gehirn wie transitorischen ischämischen Attacken und Gehirnschlag.

Renin ist ein proteolytisches Enzym aus der Klasse der Aspartyl-proteasen, welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem au der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die

Sekretion von Aldosteron aus der Nebenniere und erhöht au diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderen Stimulation des des Phosphoinositol-Umsatzes ($Ca^{2+}$-Freisetzung), Aktivier der Proteinkinase C) und Facilitation von cAMP abhängigen Hormon-Rezeptoren.

Die Affinität der Verbindungen der Formel I zum Angiotensin-II-Rezeptor kann durch Messung der $^{125}$J-Angiotensin-II- oder $^{3}$H-Angiotensin-II-Verdrängung von Rezeptoren an Zona glomerulosa Membranen von Rindernebennieren bestimmt werden. Dazu werden die präparierten Membranen in Puffer bei pH 7.4 suspendiert. Um die Degradierung des Radioliganden während der Inkubierung zu verhindern, wird Aprotinin, ein Peptidase Inhibitor, zugesetzt. Zusätzlich werden ca. 14000 cpm ein Tracers mit spezifischer Aktivität von 74 TBq/mmol (käuflich bei Amersham Buchler) und eine Menge Rezeptorprotein, die 50 % des Tracers bindet, verwendet. Die Reaktion wird durch Zugabe von 50 µl Membransuspensio zu einer Mischung von 100 µl Puffer + Aprotinin; 50 µl Puffer mit oder ohne Angiotensin-II oder Rezeptorantagoni und 50 µl Tracer gestartet. Nach einer Inkubationszeit von 60 Minuten bei 25 °C werden gebundener und freier Radioligand durch einen Filtrationsassay mit Whatmann® GFIC Filtern auf einem Skatron®-Zellsammler getrennt.

Unspezifische Bindungen werden durch Behandlung der Filter mit 0,3 % Polyethylenimino pH = 10 verhindert (Sigma, Nr. 3143). Durch Messung der Radioaktivität in einem Gamma-Szintillationszähler wird die Stärke der Verdrängung des Radioliganden vom Rezeptor bestimmt. Die $IC_{50}$-Werte, welche die Konzentration des Inhibitors bedeuten, um 50 % des Liganden zu verdrängen, werden nach Chem. et. al. J. Theor. Biol. 59, 253 (1970) bestimmt. Sie liegen für die Verbindungen der Formel (I) im Bereich von $1.10^{-4}$-$1.10^{-9}$ M.

Zur Bestimmung der antagonistischen Wirkung der Verbindungen der Formel (I) kann ihr Effekt auf den Angiotensin-II-induzierten Blutdruckanstieg an narkotisierten Sprague-Dawley Ratten gemessen werden. Als Narkotikum dient Na-Thiobarbital (Trapanal®, Byk Gulden) in der Dosierung 100 mg/kg i.p. Die i.v.-Applikation erfolgt in die Vena Jugularis. Der Blutdruck wird in der A. carotis gemessen. Zunächst werden die Tiere mit Pentoliniumtartrat (10 mg/kg i.m.) vorbehandelt, so daß ein niedrigerer Blutdrucklevel erreicht wird (Ganglienblockade). ANG II (Hypertensin® CIBA) wird im Volumen 0,1 ml/100 g in 10-minütigen Intervallen i.v. appliziert. Die Dosis beträgt 0,5 µg/kg. Die Verbindungen der Formel (I) werden in Aqua. dest. gelöst und in den Dosierungen 0,1-1; 10 und 100 mg/kg intravenös oder intraduodenal appliziert.

Die Verbindungen der Formel (I) sind im Bereich von 0,1-100 mg/kg wirksam.

Die Erfindung bezieht sich ebenso auf pharmazeutische Zusammensetzungen bestehend aus einer Verbindung der Formel I und anderen Wirkstoffen, wie z.B. Diuretika oder nichtsteroidalen antiinflammatorischen Wirkstoffen. Die Verbindungen der Formel I können auch als Diagnostika für das Renin-Angiotensin-System verwendet werden.

Pharmazeutische Präparate enthalten eine wirksame Menge des Wirkstoffs der Formel I und eventuell andere Wirkstoffe zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht; wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Apllikation werden die aktiven Verbindungen oder deren physiologisch verträgliche Salze, gewünschtenfalls mit den dafür üblichen Substanzen wie Lösungsvermittlern, Emulgatoren oder weiterem Hilfsstoffen in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

Liste der Abkürzungen:

| | |
|---|---|
| DMF | N,N-Dimethylformamid |
| NBS | N-Bromsuccinimid |
| AIBN | α,α-Azobis-isobutyronitril |

EI electron impact
DCI Desorption Chemical Ionisation
RT Raumtemperatur
EE Ethylacetat (EtOAc)
DIP Diisopropylether
H n-Heptan

**Beispiel 1**

1-[(2-Carboxy-benzo[b])furan-5-yl)methyl]-2-butyl-4-chloro-5-hydroxymethyl-imidazol

a) 5-Methyl-benzo[b]furan-2-carbonsäure-ethylester
12 g (0,088 Mol) 2-Hydroxy-5-methyl-benzaldehyd werden in 150 ml abs. DMF gelöst. 14,6 g Kaliumcarbonat (0,105 Mol) werden zugefügt, anschließend tropfenweise 9,5 g (0,088 Mol) Chloressigsäureethylester. Nach 3 Stunden am Rückfluß wird mit 500 ml $H_2O$ verdünnt, mit Ethylacetat 3x extrahiert, 4x mit Wasser gewaschen, mit Magnesiumsulfat getrocknet und eingeengt.
Chromatographie an $SiO_2$ mit Ethylacetat/Cyclohexan (1:5) ergibt 7,6 g Öl. MS (EI) = 204 ($M^+$)
$R_f$ [$SiO_2$; EtOAc/Cyclohexan (1:4)] = 0,41
b) 5-Bromomethyl-benzo[b]furan-2-carbonsäure-ethylester
7,6 g (37 mmol) der Verbindung aus a) werden mit 6,6 g NBS und 300 mg AIBN 10 Std. in 200 ml $CCl_4$ am Rückfluß gekocht. Nach Abkühlen wird mit $H_2O$ versetzt. Die Phasen werden getrennt, die organischen Phasen mit $MgSO_4$ getrocknet und eingeengt. Chromatographie an $SiO_2$ mit EtOAc/Cyclohexan (1:4) ergibt 5,4 g Öl.
MS (EI) = 282, 284 ($M^+$)
$R_f$ [$SiO_2$, EtOAc/Cyclohexan (1:4)] = 0,38
c) 1-[(2-Ethoxycarbonyl-benzo[b]furan-5-yl)methyl]-2-butyl-4-chloro-5-hydroxymethyl-imidazol
1 g (5,3 mmol) 2-Butyl-4-chloro-5-hydroxymethyl-imidazol (hergestellt nach EP-A 253 310) werden in 20 ml Methanol gelöst und mit einer Lösung von 127 mg Natrium in 5 ml Methanol versetzt. Nach 30 min. wird eingeengt und in 40 ml DMF gelöst. 1,5 g (5,5 mmol) der Verbindung aus b) werden zugesetzt und 14 Std. bei 25 °C gerührt. Nach Eingießen in 200 ml $H_2O$ wird mit EtOAc extrahiert, die organische Phase mit $H_2O$ gewaschen, mit $MgSO_4$ getrocknet und eingeengt. Chromatographie an $SiO_2$ mit EtOAc/Cyclohexan (1:1) ergibt 0,73 g Öl
MS (DCI) = 391 ($M^+ + H$)
$R_f$[$SiO_2$; EtOAc/Cyclohexan (1:2)] = 0,29
d) 1-[2-Carboxy-benzo[b]furan-5-yl)methyl]-2-butyl-4-chloro-5-hydroxymethyl-imidazol
0,79 g (2,5 mmol) der Verbindung aus c) werden in 20 ml Ethanol mit 20 ml 2 N NaOH am Rückfluß gekocht. Nach 2 Std. wird in Wasser aufgenommen, mit 2 N HCl auf pH 3,5 gestellt und mit Dichlormethan extrahiert. Nach Trocknen und Einengen erhält man 0,31 g der Titelverbindung als amorphes Pulver.
MS (DCI) = 363 ($M^+$ + H)
$R_f$ ($SiO_2$ , EtOAc/Cyclohexan (1:1)] = 0,03

**Beispiel 2**

1-[2-Carboxy-benzo[b]thiophen-5-yl)methyl]-2-butyl-4-chloro-5-hydroxymethyl-imidazol

a) 4-Tolyl-thio-acetaldehyd-diethylacetal
In einer Lösung von 12,5 g Natrium in 250 ml Ethanol werden 62 g p-Thiokresol in 100 ml Ethanol bei 0 °C getropft. Nach Zugabe von 15 g Natriumiodid tropft man 98,5 g Bromacetaldehyddiethylacetal zu und kocht 4 Std. am Rückfluß. Nach Abkühlen wird auf 1 l Wasser gegossen und mit EtOAc extrahiert. Nach Trocknen mit $MgSO_4$ wird eingeengt und destilliert.
Sdp. 118 ° (0,01 Torr), Ausbeute 92,9 g
b) 5-Methylbenzo[b]thiophen
320 g $P_2O_5$ werden mit 246 ml Orthophosphorsäure versetzt und auf 130 °C erhitzt (1 Std.). Bei 180 °C werden 92,9 g der Verbindung aus a) bei 5 mm Hg mittels Kapillarrohr unter die Oberfläche der Polyphosphorsäure gegeben. Das Produkt destilliert ab. Man erhält 26 g als Öl.
MS (EI) = 135 ($M^+$)

c) 2-Acetyl-5-methylbenzo[b]thiophen
Eine Lösung von 26 g der Verbindung aus b) und 13,5 g Acetylchlorid in 250 ml $CH_2Cl_2$ wird zu einer Suspension von 23,4 g $AlCl_3$ in 780 ml $CH_2Cl_2$ getropft. Nach 90 min wird die dunkelgrüne Lösung in eiskalte verdünnte HCl gegossen, die organische Phase abgetrennt, mit $MgSO_4$ getrocknet und eingeengt. Man erhält 25,5 g Produkt als Öl.
MS (EI) = 178 ($M^+$)
d) 5-Methyl-benzo[b]thiophen-2-carbonsäure
11,7 g NaOH werden in 58 ml $H_2O$ gelöst und bei 10 °C mit 14,1 g Brom versetzt. Bei 0 °C gibt man 5,2 g 2-Acetyl-5-methyl-benzo[b]thiophen in 50 ml Dioxan zu. Nach 90 min wird mit 5 N HCl sauer gestellt und mit $CH_2Cl_2$ extrahiert. Nach Trocknen mit $Na_2SO_4$ wird eingeengt. Nach Verreiben mit EtOAc wird abgesaugt. Man erhält 3 g Kristalle.
MS (EI) = 192 ($M^+$)
$R_f$ [$SiO_2$; $CH_2Cl_2$/MeOH (10:1)] = 0,37
e) 5-Methyl-benzo[b]thiophen-2-carbonsäureethylester
3 g der Verbindung aus d) wird in 2,5 N ethanolischer HCl (50 ml) 2,5 Std. am Rückfluß gekocht. Nach Einengen erhält man 2,27 g Öl.
MS (EI) = 220 ($M^+$)
$R_f$ [$SiO_2$; $CH_2Cl_2$/MeOH (10:1)] = 0,82
f) 5-Brommethyl-benzo[b]thiophen-2-carbonsäureethylester
Aus 2,27 g der Verbindung aus e) und 1,83 g NBS erhält man nach der Vorschrift von Beispiel 1b) 1,77 g Öl.
MS (EI) = 298 + 300 ($M^+$)
g) 1-[(2'-Ethoxycarbonyl-benzo[b]thiophen-5'-yl)methyl]-2-butyl-4-chloro-5-hydroxymethylimidazol
Aus 1,1 g 2-Butyl-4-chloro-5-hydroxymethyl-imidazol und 1,77 g der Verbindung aus f) erhält man analog der Vorschrift von Beispiel 1c) 0,5 g der Titelverbindung als Öl.
MS (DCI) = 407 (M + H)
$R_f$ ($SiO_2$; EtOAc/Cyclohexan (1:1)] = 0,2
h) 1-[(2-Carboxyl-benzo[b]thiophen-5-yl)methyl]-2-butyl-4-chloro-5-hydroxymethyl-imidazol
Analog der Vorschrift aus Beispiel 1d), erhält man aus der Verbindung aus g) 0,26 g der Titelverbindung, Schmp. 195 °C (Zers.)

**Beispiel 3**

1-[(2-Carboxy-indol-5-yl)-methyl]-2-butyl-4-chloro-5-hydroxymethyl-imidazol

a) 30 g (0,189 Mol) p-Tolylhydrazin werden in 600 ml 1N HCl gelöst und auf 40-50 °C erwärmt. 23 g Brenztraubensäureethylester werden zugegeben. Nach 2 Std. wird der Niederschlag abgesaugt und getrocknet (Vakuum). Man erhält 37 g Produkt.
MS (EI) = 220 ($M^+$)
b) 5-Methyl-indol-2-carbonsäurethylester
130 g $P_2O_5$ werden zu 111 ml Orthophosphorsäure gegeben. Nach Abkühlen gibt man 37 g der Verbindung aus a) zu und erwärmt auf 80 °C. Nach Einsetzen der Reaktion steigt die Temperatur auf 130 °C an. Nach Abkühlen wird auf Eiswasser gegossen und mit Essigester extrahiert. Die organische Phase wird mit $NaHCO_3$ (1 N), $H_2O$ und gesättigter NaCl-Lösung gewaschen, mit $Na_2SO_4$ getrocknet und eingeengt. Das Produkt wird aus n-Hexan/EtOAc umkristallisiert.
Man erhält 11,7 g Produkt von Schmp. 158 °C.
c) 1-Acetyl-5-methyl-indol-2-carbonsäureethylester
11,7 g der Verbindung aus b) werden in 100 ml wasserfreiem DMF mit 2,6 g Natriumhydrid (50 % in Öl) versetzt. Anschließend gibt man 4,5 g Acetylchlorid zu und rührt 3,5 Std. Nach Eingießen in $H_2O$ wird mit EtOAc extrahiert. Die organische Phase wird 3x mit $H_2O$ und 1x mit gesättigter NaCl-Lösung gewaschen, mit $MgSO_4$ getrocknet und eingeengt. Nach Chromatographie an $SiO_2$ erhält man 6 g der Titelverbindung als Öl.
MS (EI) 245 ($M^+$)
$R_f$ ($SiO_2$; EtOAc/Cyclohexan (1:4)] = 0,23
d) 1-Acetyl-5-brommethyl-indol-2-carbonsäurethylester
Analog der Vorschrift von Beispiel 1b), erhält man aus 6 g der Verbindung aus Beispiel 3c) und 4,3 g NBS 3,6 g der Titelverbindung als Öl.
MS (EI) 323 + 325 ($M^+$)

$R_f$ [($SiO_2$); EtOAc/Cyclohexan (1:0) = 0,21]

e) 1-[(2-Ethoxycarbonyl-indol-5-yl)methyl]-2-butyl-4-chlor-5-hydroxymethylimidazol

Analog der Vorschrift in Beispiel 1c) erhielt man aus 0,6 g 2-Butyl-4-chloro-5-hydroxymethylimidazol und 1,1 g der Verbindung aus Beispiel 3d) 160 mg der Titelverbindung als Öl.

MS (DCI): 390 ($M+H^+$)

$R_f$ [$SiO_2$; EtOAc/Cyclohexan (1:1)] = 0,2

f) 1-[(2-Carboxy-indol-5-yl)-methyl]-2-butyl-4-chloro-5-hydroxymethylimidazol

Analog der Vorschrift in Beispiel 1d) erhielt man aus 160 mg der Verbindung aus Beispiel 3e) 40 mg der Titelverbindung als Harz.

MS (DCI) 362 ($M+H^+$)

$R_f$ [$SiO_2$; $CH_2Cl_2$/MeOH (2:1)] = 0,27.

Die Verbindungen der Beispiele 4-11 wurden analog zu Beispiel 1 synthetisiert.

Diese Verbindungen besitzen folgende allgemeine Formel:

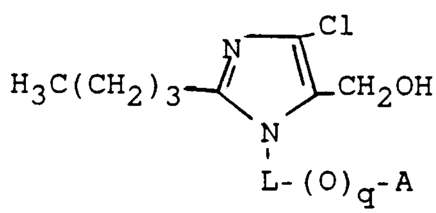

| | MS (DCI; M+H) | A-(O)$_q$-L- |
|---|---|---|
| Beispiel 4 | 413 | 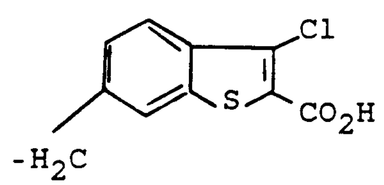 |
| Beispiel 5 | 423 | 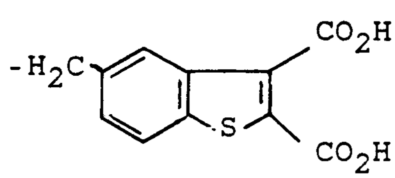 |

| | MS (DCI; M+H) | A-$(O)_q$-L- |
|---|---|---|
| Beispiel 6 | 379 | 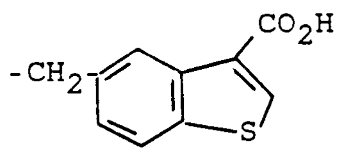 |
| Beispiel 7 | 407 | 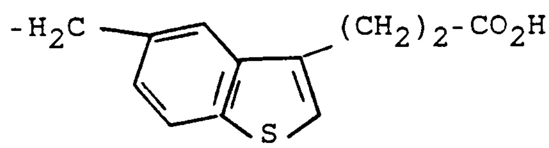 |
| Beispiel 8 | 409 | 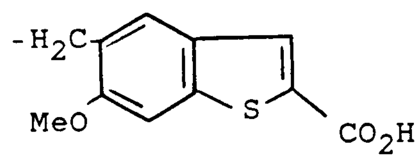 |
| Beispiel 9 | 371 | 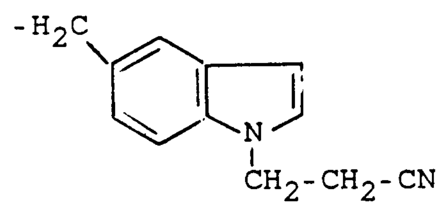 |
| Beispiel 10 | 439 | 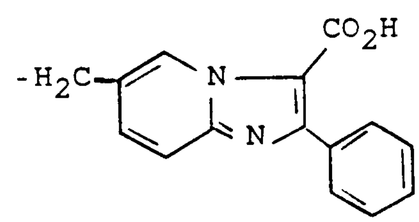 |
| Beispiel 11 | 440 | 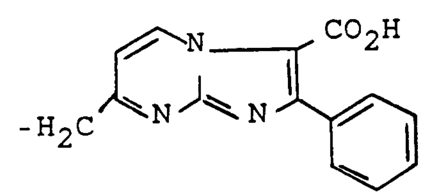 |

3-(5-Methyl-Indol-1-yl)-propionitril (s. Beispiel 9)

Zu 4 ml Trimethylbenzylammoniumhydroxyd und 10 ml Acrylnitril in 100 ml Dioxan gibt man unter Rühren 13 g (0,1 mol) 5-Methylindol. Nun erhitzt man 2 h auf 80 °C, dann läßt man 3 Tage bei Raumtemperatur stehen. Stark verdünnte Essigsäure wird nun zugegeben, um die Base zu neutralisieren. Nun wird mit EE (3x30 ml) extrahiert. Die vereinten organischen Phasen werden mit $Na_2SO_4$ getrocknet und im Vakuum eingeengt. Der Rückstand wurde an $SiO_2$ mit DIP als Eluens chromatographiert. $R_f$ [DIP] = 0,3

Bedingungen für die Veresterung der Reste A in den Beispielen 5, 6 und 7.

20 g 5-Methyl-2,3-dicarboxy-benzo[b]thiophen (Beispiel 5) werden 6 h in 400 ml absolutem Methanol, das 3 % Schwefelsäure enthält unter Rückfluß gekocht. Nach Abkühlen auf Raumtemperatur wird auf Eis gegossen. Der Niederschlag wird abfiltriert und die $H_2O$-Phase 3x mit Ether extrahiert. nach Trocknen mit

$Na_2SO_4$ wird eingeengt, wobei der Ester als Öl anfällt.

**Beispiel 10a)**

Alternative zur Verseifung des Ethylesters von Beispiel 10 zur Titelverbindung 10

180 mg (0,38 mmol) Ethylester in 2 ml Ethanol werden mit 0,5 ml wässriger 1 N NaOH versetzt und 20-60 h bei 25 °C gerührt. Nun wird eingeengt und der Rückstand an $SiO_2$ mit $CH_2Cl_2$/MeOH 8/2 als Laufmittel gereinigt, wobei die Säure als amorphes Pulver anfällt.

**Beispiel 12**

1-[(3-Carboxy-2-phenylimidazo[1,2-a]pyridin-6-yl)methyl]-2-butyl-4-chloro-5-formyl-imidazol

a) 2-Butyl-4-chloro-5-formylimidazol
Zu 20 g (0,106 Mol) 2-Butyl-4-chloro-5-hydroxymethylimidazol in 350 ml Eisessig gibt man bei 10-15 °C langsam 305 ml einer 1 M $(NH_4)_2Ce(NO_3)_6$-Lösung in $H_2O$ zu. Nach 2,5 h bei RT wird der pH-Wert mit 2 n KOH auf 4 eingestellt (20 °C während der Zugabe der Base). Nun wird 4 x mit je 500 ml $CH_2Cl_2$ extrahiert und die vereinigten organischen Extrakte 3x mit je 300 ml gesättigter wäßriger $NaHCO_3$-Lösung gewaschen, mit $Na_2SO_4$ getrocknet und eingeengt, wobei die Titelverbindung als farbloser Feststoff anfällt (18 g). Smp = 90 °C.
b) 1-[3-Ethoxycarbonyl-2-phenylimidazo[1,2-a]pyridin-6-yl) methyl]-2-butyl-4-chloro-5-formyl-imidazol
0,2 g (1,07 mmol) 2-Butyl-4-chloro-5-formyl-imidazol, 0,38 g (1,07 mmol) 6-Brommethyl-3-ethoxycarbonyl-2-phenylimidazo[1,2-a]pyridin, 0,15 g $K_2CO_3$ (1,07 mmol) und 0,5 g pulverisiertes Molsieb werden 5 h bei 60 °C in 5 ml DMF gerührt. Nun werden 100 ml EE zugesetzt und 3x mit $H_2O$ gewaschen. Nach Trocknen mit $Na_2SO_4$ wird eingeengt und der Rückstand an $SiO_2$ mit EE/H 1/1 als Eluens chromatographiert.
MS (DCI) 465 ($M^+H^+$)
Rf [$SiO_2$; EE/H(1:1)] = 0,18)
c) Die Herstellung der Brommethylverbindung aus b) und die Verseifung der Titelverbindung b) wird analog Beispiel 1 oder 10a durchgeführt.

Beispiel 13 wird analog Beispiel 12 hergestellt. Diese Verbindungen besitzen die folgende allgemeine Formel

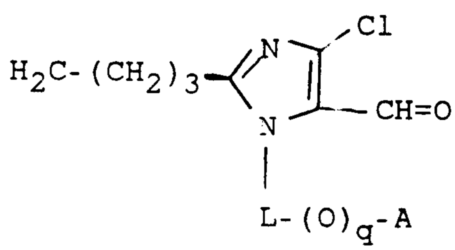

| | MS (DCI; M+H) | A-(O)$_q$-L- |
|---|---|---|
| Beispiel 12 | 437 | 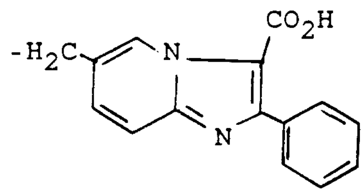 |
| Beispiel 13 | 438 | 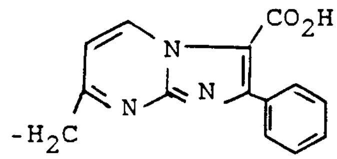 |
| Beispiel 14 | 437 | 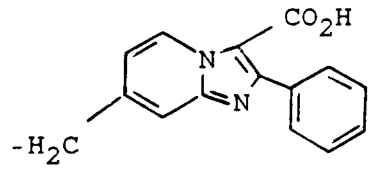 |
| Beispiel 15 | 437 | 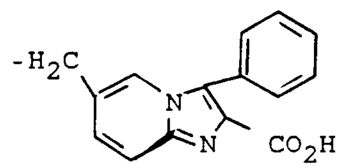 |
| Beispiel 16 | 411 | 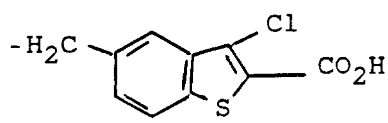 |
| Beispiel 17 | 377 | 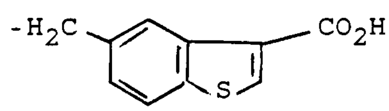 |
| Beispiel 18 | 405 | 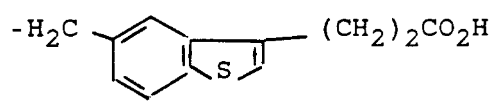 |
| Beispiel 19 | 449 | 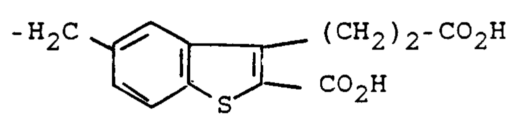 |
| Beispiel 20 | 455 | 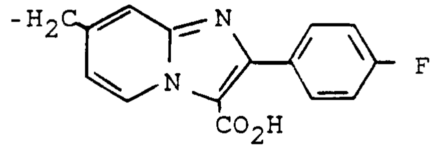 |

| | MS (DCI; M+H) | $A-(O)_q-L-$ |
|---|---|---|
| Beispiel 21 | 455 | 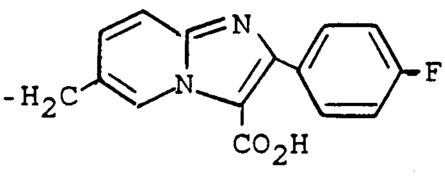 |

zu Beispiel 15: Herstellung des heterocyclischen Grundkörpers

2-Ethoxycarbonyl-3-phenyl-6-methyl-imidazo[3,2-a]pyridin

a) Brenztraubensäureethylester
Zu 10 g (0,061 mol) Phenylbrenztraubensäure, 8,6 g (0,073 mol) Diisopropylethylamin in 200 ml Dichlormethan gibt man 13,9 g (0,073 mol) Triethyloxoniumtetrafluoroborat. Nach 4 h bei RT wird die organische Phase 2mal mit 10 %iger Zitronensäurelösung und 2mal mit gesättigter Natriumhydrogencarbonat-Lösung gewaschen. Nach Trocknen mit $MgSO_4$ wird eingeengt. Nach Chromatographie an $SiO_2$ mit EE/H 1/5 als Eluens erhält man 7 g eines Öles.
$R_f$ (EE/H 1/5) = 0,3
b) 2-Brombrenztraubensäureethylester
7 g (0,036 mol) Brenztraubensäureethylester (a) 50 ml Tetrachlorkohlenstoff werden bei 5 °C mit 2,6 ml Brom versetzt. Danach wird 1 h zum Sieden erhitzt, eingeengt und der Rückstand in 100 ml EE aufgenommen. Die EE-Phase wird 2mal mit 10 %iger $Na_2SO_3$-Lösung und 1mal mit $H_2O$ gewaschen und mit $MgSO_4$ getrocknet. Entfernen des Lösungsmittels im Vakuum liefert 9 g der Verbindung als Öl.
MS (DCI) = 271 (M + H)
c) 2-Ethoxycarbonyl-3-phenyl-6-methyl-imidazo[3,2-a]pyridin
3 g 2-Brombrenztraubensäureethylester (b) und 1,2 g 2-amino-5-methylpyridin in 50 ml Ethanol werden 2 h zum Sieden erhitzt. Nach Abziehen des Alkohols wird der Rückstand in 100 ml EE aufgenommen. Die organische Phase wird 1mal mit gesättigter $Na_2CO_3$-Lösung gewaschen und anschließend mit $MgSO_4$ getrocknet. Nach Einengen wird an $SiO_2$ mit EE/H 1/1 als Eluens chromatographiert.
Ausbeute: 1,7 g
$R_f$ (EE/H 1/1) = 0,2 MS (DCI) = 281

Beispiel 22

1-[2-(Tetrazol-5-yl)-3-chlor-benzo[b]thiophen-6-yl)-methyl]-2-n-butyl-4-chlor-5-hydroxymethyl-imidazol

a) 3-Chlor-6-methyl-benzo[b]thiophen-2-carbonsäureamid
Zu 30 g (12,3 mmol) 3-Chlor-6-methyl-benzo[b]thiophen-2-carbonsäurechlorid (J. Org. Chem. 41, 3399, (1976) in Dimethoxyethan werden bei 0 °C 100 ml 4 n $NH_3$ in DME zugetropft. Nach 30 min wird eingeengt und aus Ethanol kristalissiert, wobei 14,5 g der Titelverbindung anfallen. Schmp.: = 193 °C
$R_f$ (EE/H 1/1) = 0,3 MS (DCI) = 226 (M + H)
b) 3-Chlor-2-cyano-6-methyl-benzo[b]thiophen
3 g (13,3 mmol) Titelverbindung a) in 40 ml Pyridin werden bei 0 °C mit 6,2 g (53,2 mmol) Thionylchlorid versetzt. Nach 2 h wird auf eisgekühlte 4 n HCl gegossen und 3mal mit je 50 ml EE extrahiert. Die vereinigten organischen Phasen werden mit $MgSO_4$ getrocknet und eingeengt. Chromatographie an $SiO_2$ mit EE/H 1/2 liefert die Titelverbindung (1,5 g). $R_f$ (EE/H 1/1) = 0,75 MS (DCI) = 208 (M + H)
c) 1-[(2-Hetrazol-5-yl)-3-chlor-benzo[b]-thiophen-6-yl)-methly]-2-n-butyl-4-chlor-5-hydroxymethyl-imidazol
0,4 g (1,0 mmol) 1-[(2-cyano-3-chlor-benzo[b]-thiophen-6-yl) methyl]-2-n-butyl-4-chlor-5-hydroxymethyl-imidazol (hergestellt aus 22 b analog Beispiel 1b, c) und 0,42 (2,0 mmol) Trimethylzinnazid werden in 50 ml Toluol 24 h zum Sieden erhitzt. Danach wird eingeengt und der Rückstand 20 ml EE aufgenommen

und bei RT mit 0,6 ml $HBF_4$ und 15 ml gesättigter KF-Lösung versetzt. Nach 12 h werden nochmals 20 ml EE zugegeben und die Phasen getrennt. Die organische Phase wird mit $MgSO_4$ getrocknet und eingeengt.
Chromatographie an $SiO_2$ mit Dichlormethan/Methanol 5/1 liefert die Titelverbindung.
$R_f$ ($CH_2Cl_2$/MeOH 5/1) = 0,4 MS (FAB) = 437 (M+H)

Beispiel 23

1-[(2-phenyl-3-carboxy-imidazo[1,2-a]pyrimidin-7-yl)-methyl]-2-n-butyl-4-methylthio-imidazol-5-carbonsäure

a) 2-Amino-2-cyano-essigsäureethylester
Zu 35 g (0,246 mol) 1-Cyanoglyoxyalsäureethylester-2-oxim in 350 ml $H_2O$ und 280 ml gesättigter Natriumhydrogencarbonat-Lösung werden bei Raumtemperatur portionsweise (15 min) 119 g Natriumdithionit zugegeben. Anschließend wird 1 Stunde auf 35 °C erwärmt; dannmit NaCl gesättigt und 5 x mit Dichlormethan extrahiert. Nach Trocknen mit Calciumchlorid wird die organische Phase eingeengt. Man erhält 11,8 g der Titelverbindung als Öl.
$R_f$ ($CH_2Cl_2$/$CH_3OH$ 9/1) = 0,6
b) 2-Cyano-2-n-butylcarbonyaminoölessigsäureethylester
Zu 3,6 g (28,09 mmol) Verbindung 23a) in 50 ml trockenem $CH_2Cl_2$ und 2,3 ml (28,09 mmol) Pyridin tropft man bei -5 °C bis 0 °C 3,39 ml (28,09 mmol) Valeroylchlorid in 5 ml $CH_2Cl_2$ zu. Anschließend wird 1 Stunde bei Raumtemperatur gerührt. Die organische Phase wird dann 3mal mit $H_2O$ 1mal mit gesättigter NaCl-Lösung gewaschen, mit Calciumchlorid getrocknet und eingeengt. Kristallisation aus DIP liefert 1,7 g der Titelverbindung.
$R_f$ ($CH_2Cl_2$/$CH_3OH$ 9/1) = 0,35
Schmp.: 87 °C
c) 3-Amino-2-n-butylcarbonylamino-methylthioacrylsäureethylester
Zu 2,9 g (13,67 mmol) Verbindung 23b) und 0,19 ml (1,36 mmol) Triethylamin in 60 ml absolutem Ethanol gibt man bei Raumtemperatur 2 ml (27,26 mmol) kondensiertes Methylmercaptan. Nach 3 Tagen gibt man nochmals 0,5 ml Methylmercaptan zu. Nach weiteren 24 Stunden bei Raumtemperatur werden nochmals 0,5 ml Methylenmercaptan und 0,19 ml Triethylamin zugespritzt und weitere 24 Stunden bei Raumtemperatur gerührt. Anschließend wird das Lösungsmittel entfernt und der Rückstand aus DIP kristallisiert, wobei 2,4 g der Titelverbindung anfallen.
$R_f$ ($CH_2Cl_2$/EE 4/1) = 0,3
Schmp.: 120 °C
d) 2-n-Butyl-4-methylthio-imidazol-5-carbonsäureethylester
Zu 4,17 g (20,0 mmol) Phosphorpentachlorid in 20 ml $CH_2Cl_2$ tropft man bei -78 °C 2,44 g (20,0 mmol) 4-Dimethylaminopyridin in 12 ml $CH_2Cl_2$. Nach 5 min werdne 2,42 g (10,0 mmol) Verbindung 23c) in 25 ml $CH_2Cl_2$ zugetropft. Nun läßt man auf Raumtemperatur kommen und verdünnt mit 30 ml $CH_2Cl_2$. Nach 2 Stunden werden unter Eiskühlung 300 ml 1n Natriumhydrogencarbonat-Lösung zugegeben und 1 Stunde gerührt. Nun werden die Phasen getrennt, die wäßrige Phase 3mal mit EE extrahiert und die vereinigten organischen Phasen mit Calciumchlorid getrocknet. Chromatographie an $SiO_2$ mit $CH_2Cl_2$/EE (9/1) liefert 1,4 g der Titelverbindung als Öl.
$R_f$ ($CH_2Cl_2$/EE 9/1) = 0,6 MS (DCI) = 243 ($M^+$+H)

Die übrigen Reaktionsschritte zur Titelverbindung 23 werden analog Beispiel 12b) und 10a durchgeführt.
$R_f$ (EE/MeOH 5/1) = 0,2 MS (FAB) = 466 (M+H)

Beispiel 24

1-[(2-phenyl-3-carboxy-imidazo(1,2-a)pyrimidin-7-yl)-methyl]-2-n-butyl-4-methylsulfoxyl-imidazol-5-carbonsäure

a) 1-[(2-phenyl-3-carboxyethyl-imidazo(1,2-a)pyrimidin-7-yl)-methyl]-2-n-butyl-4-methylsulfoxyl-imidazol-5-carbonsäureethylester
Zu 80 mg (0,15 mmol) 1-[(2-phenyl-3-carboxyethyl-imidazo [1,2-a]pyrimidin-7-yl)-methyl]-2-n-butyl-4-methylthio-imidazol-5-carbonsäureethylester in 5 ml Dichlormethan gibt man 52 g (0,15 mmol) 50 %ige Metachlorperbenzoesäure bei RT. Nach 1 h wird 1mal mit gesättigter $Na_2CO_3$-Lösung und 1mal mit $H_2O$ gewaschen. Die organische Phase wird eingeengt, der Rückstand liefert nach Chromatographie auf $SiO_2$

mit EE/H 1/1 als Eluens die Titelverbindung.
$R_f$ (EE/H 1/1) = 0,15 MS (FAB) = 538 (M + H)45
b) Die Titelverbindung 24 wird analog Beispiel 10a) erhalten.
$R_f$ ($CH_2Cl_2$/MeOH 5/1) = 0,2 MS (FAB) = 482 (M + H)

Beispiel 25

1-[(2-phenyl-3-carboxy-imidazo(1,2-a)pyrimidin-7-yl)-methyl]-2-n-butyl-4-methylsulfonyl-imidazol-5-carbonsäure

a) Zu 120 mg (0,23 mmol) 1-[(2-phenyl-3-carboxyethyl-imidazo (1,2-a)pyrimidin-7-yl)-methyl]-2n-butyl-4-methylthioimidazol-5-carbonsäureethylester in 10 ml Dichlormethan gibt man bei RT 158 mg (0,46 mmol) 50 %iger Metachlorperbenzoesäure. Weiterer Reaktionsverlauf und Aufarbeitung wie 24a).
$R_f$ (EE) = 0,6 MS (FAB) = 554 (M + H)
b) Die Verseifung zur Titelverbindung erfolgt analog Beispiel 10a).
$R_f$ ($CH_2Cl_2$/MeOH 5/1) = 0,2 MS (FAB) = 498 (M + H)

Die Beispiele 26 und 27 werden analog zu den Beispielen 24 und 25 hergestellt.

Beispiel 28-33 werden analog Beispiel 22 hergestellt.

Die Herstellung von 2-Phenyl-3-amido-7-methyl-imidazo (1,2-a)pyrimidin (für Bsp. 28) 500 mg (1,78 mmol) 2-Phenyl-3-carbethoxy-7-methyl-imidazo(1,2-a)pyridin, 213 µl (5,35 mmol) Formamid und 120 mg Kaliumtertiärbutylat werden in 10 ml DMF 1 h auf 100 °C erhitzt. Nun wird auf $H_2O$ gegossen, der pH mit $NaHCO_3$ auf 8-9 gestellt und abgesaugt.
$R_f$ (EE) = 0,3 MS (DCI) = 252 (M + H)

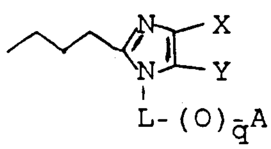

| | MS (FAB; M+H) | X | Y | $A-(O)_q-L$ |
|---|---|---|---|---|
| Beispiel 22 | 437 | Cl | $CH_2-OH$ | 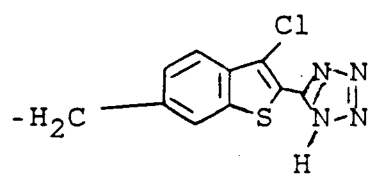 |

| | MS (FAB; M+H) | X | Y | $A-(O)_q-L$ |
|---|---|---|---|---|
| Beispiel 23 | 466 | $-S-CH_3$ | $CO_2H$ | 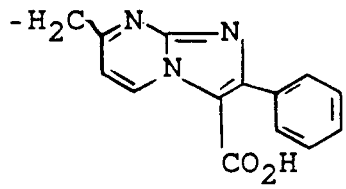 |
| Beispiel 24 | 482 | $-SOCH_3$ | $CO_2H$ | |
| Beispiel 25 | 498 | $-SO_2CH_3$ | $CO_2H$ | |
| Beispiel 26 | 455 | $-SOCH_3$ | $CO_2H$ | 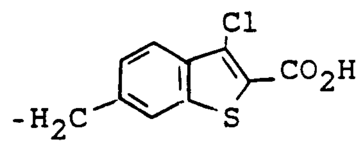 |
| Beispiel 27 | 471 | $-SO_2CH_3$ | $CO_2H$ | |
| Beispiel 28 | 517 | $-S-CH_3$ | $CO_2Et$ | 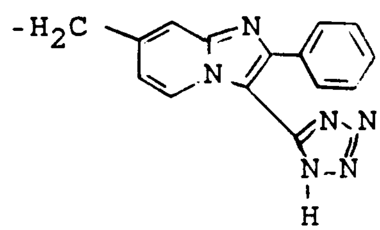 |
| Beispiel 29 | 488 | $-S-CH_3$ | $CO_2H$ | |
| Beispiel 30 | 497 | $-S-CH_3$ | $CO_2Et$ | 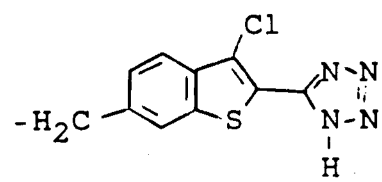 |
| Beispiel 31 | 463 | $-SCH_3$ | $CO_2H$ | |
| Beispiel 32 | 523 | $-SO_2CH_3$ | $CO_2Et$ | |
| Beispiel 33 | 495 | $-SO_2CH_3$ | $CO_2H$ | |

Beispiel 34

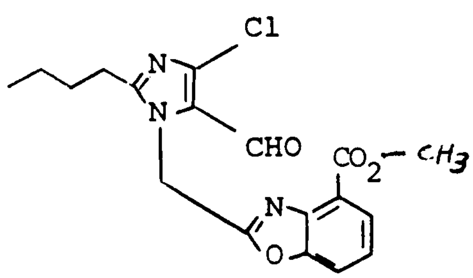

1-[(2-(4-Methoxycarbonyl)-benzoxazolyl)methyl]-2-n-butyl-4-chloro-5-formyl-imidazol

a) 2-Brommethyl-benzoazol-4-carbonsäuremethylester

453 mg (2,37 mmol)

2-Methyl-benzoxazol-4-carbonsäuremethylester (hergestellt nach H. Heterocyclic Chem., 27, 335 (1990) werden in 40 ml Chlorbenzol gelöst, 421 mg (2,37 mmol) NBS und 20 mg Benzoylperoxid zugefügt und die erhaltene Mischung 2 Stunden am Rückfluß erhitzt. Es wird eingeengt, der Rückstand in Ethylacetat aufgenommen, mit ges. $NaHCO_3$-, 10 %iger $Na_2SO_3$-und ges. NaCl-Lösung ausgeschüttelt und über

$Na_2SO_4$ getrocknet. Einengung und Chromatographie an $SiO_2$ mit n-Heptan/Ethylacetat (2:1) ergibt 128 mg der Titelverbindung.
F.p.: 110-113 °C
MS (CI) = 271 (M+H)
b) 1-[(2-(4-Methoxycarbonyl)-benzoxazolyl)methyl-2-n-butyl-4-chloro-formyl-imidazol
Zu einer Suspension von 176 mg (0, 944 mmol) 2-n-Butyl-4-chloro-5-formyl-imidazol und 130 mg (0,944 mmol) $K_2CO_3$ in 2 ml absolutem DMF wird eine Lösung von 255 mg (0,944 mmol) der Verbindung aus a) in 1 ml absolutem DMF getropft und die erhaltene Suspension 1 Stunde bei RT gerührt. Es wird zur Trockene eingeengt, der Rückstand in Ethylacetat aufgenommen, mit Wasser und gesättigter NaCl-Lösung gewaschen, getrocknet und eingeengt. Chromatographie an $SiO_2$ mit $CH_2Cl_2$/Ethylacetat (9:1) ergibt 103 mg der gewünschten Verbindung.
MS (CI) = 376 (M+H)

Beispiel 35

1-[(2-(5-Methoxycarbonyl)-benzoxazolyl)-methyl]-2-n-butyl-4-chloro-5-formyl-imidazol

a) 2-Brommethyl-benzoxazol-5-carbonsäuremethylester
Die Titelverbindung wird aus 2-Methyl-benzoxazol-5-carbonsäuremethylester nach dem in Beispiel 14a) angeführten Verfahren hergestellt.
MS (CI) = 271 (M+H)
b) 1-[(2-(5-Methoxycarbonyl)-benzoxazolyl)methyl]-2-n-butyl-4-chloro-5-formyl-imidazol
Diese Verbindung wird aus der Verbindung aus a) und 2-n-Butyl-4-chloro-5-formyl-imidazol nach dem Verfahren des Beispiels 34b) dargestellt.
MS (CI) = 376 (M+H)

**Patentansprüche**
**Patentansprüche für folgende Vertragsstaaten : AT, BE, CH, DE, DK, FR, GB, IT, LI, LU, NL, SE**

**1.** Verbindung der Formel I,

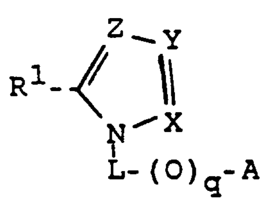

(I)

in welcher

a) X, Y und Z gleich oder verschieden sind und N oder $CR^2$ bedeuten,

b) $R^1$

1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

bedeutet;

c) $R^2$

1. Wasserstoff,
2. Halogen,
3. Nitro,
4. $C_vF_{2v+1}$,
5. Pentafluorphenyl,
6. Cyano,
7. Phenyl,
8. Phenyl-($C_1$-$C_3$)-alkyl,
9. ($C_1$-$C_{10}$)-Alkyl,
10. ($C_3$-$C_{10}$)-Alkenyl,
11. Phenyl-($C_2$-$C_6$)-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. -$(CH_2)_{o-1}$-$CHR^7$-$OR^5$,
16. -$(CH_2)_o$-O-CO-$R^3$,
17. -$(CH_2)_o$-S-$R^6$,
18. -$S(O)_r$-$R^6$,
19. -CH = CH-$(CH_2)_m$-$CHR^3$-$OR^6$,
20. -$CH_2$ = CH-$(CH_2)_m$-CO-$R^8$,
21. -CO-$R^8$,
22. -CH = CH-$(CH_2)_m$-O-CO-$R^7$,
23. -$(CH_2)_m$-$CH(CH_3)$-CO-$R^8$,
24. -$(CH_2)_o$-CO-$R^8$,
25.

$$-(CH_2)_o-O-\underset{\underset{W}{\|}}{C}-NH-R^9,$$

26.

$$-(CH_2)_o-NR^7-\underset{\underset{W}{\|}}{C}-OR^9,$$

27. -$(CH_2)_o$-$NR^7$-CO-$NHR^9$,
28. -$(CH_2)_o$-$NR^7$-$SO_2R^9$,
29.

$$-(CH_2)_o-NR^7-\underset{\underset{W}{\|}}{C}-R^9,$$

30. -$(CH_2)_n$F,
31. -$(CH_2)_n$-O-$NO_2$,
33. -$CH_2$-$N_3$,
34. -$(CH_2)_n$-$NO_2$,
35. -CH = N-$NR^5R^7$,
36. Phthalimido-$(CH_2)_n$-,

37.

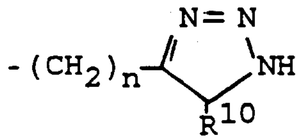

,

38.

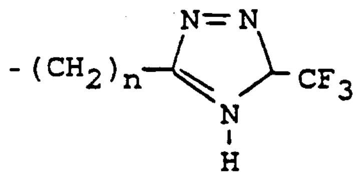

,

39.

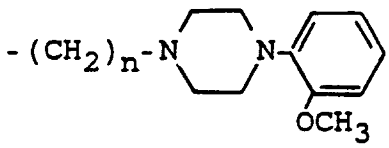

,

40.

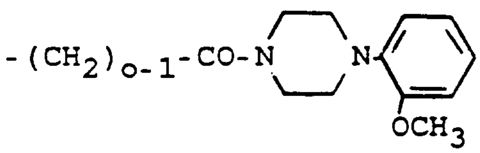

,

41. Phenyl-$SO_2$-NH-N = CH-,

42.

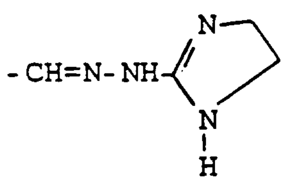

,

43. -$(CH_2)_n$-$SO_2$-$NR^7$-CO-$NR^6R^9$,

44. -$(CH_2)_o$-$SO_2R^9$,

44. einem wie unter c) 7. oder 8. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,

45. einem wie unter c) 9., 10., oder 18. definierten Rest, worin 1 bis alle H-Atome durch Fluor substituiert sind, oder

46. den unter c) 13. definierten Rest, der mit 1 oder 2 gleichen oder verschiedenen Resten aus der Reihe Methoxycarbonyl und $(C_1$-$C_4)$-Alkyl substituiert ist,

bedeutet;

d) $R^3$

1. Wasserstoff,

2. $(C_1$-$C_8)$-Alkyl,

3. $(C_3-C_8)$-Cycloalkyl,
4. Phenyl,
5. Benzyl oder
6. den unter d) 2. definierten Rest, worin 1 bis alle H-Atome durch Fluor substituiert sind,

bedeuten;

e) $R^4$

1. Wasserstoff,
2. $(C_1-C_6)$-Alkyl,
3. $(C_3-C_8)$-Cycloalkyl,
4. $(C_2-C_4)$-Alkenyl oder
5. $(C_2-C_4)$-Alkinyl

bedeutet;

f) $R^5$

1. Wasserstoff,
2. $(C_1-C_6)$-Alkyl,
3. $(C_3-C_8)$-Cycloalkyl,
4. Phenyl oder
5. Benzyl

bedeutet;

g) $R^6$

1. Wasserstoff,
2. $(C_1-C_6)$-Alkyl,
3. $(C_3-C_8)$-Cycloalkyl,
4. $(C_6-C_{12})$-Aryl,
5. Benzyl,
6. $(C_1-C_9)$-Heteroaryl, der teilweise oder vollständig hydriert sein kann, und der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können.
7. $(C_1-C_4)$-Alkanoyl,
8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, $NR^{11}R^{12}$ oder

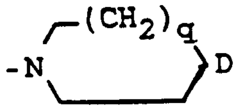

bedeutet;

9. $(C_1-C_9)$-Heteroaryl-$(C_1-C_3)$-Alkyl, wobei der Heteroarylteil wie unter g) 6. definiert ist

h) $R^7$

1. Wasserstoff,
2. $(C_1-C_6)$-Alkyl,
3. $(C_3-C_8)$-Cycloalkyl,
4. $(C_6-C_{12})$-Aryl-$(C_1-C_6)$-alkyl,
5. Phenyl oder
6. $(C_1-C_9)$-Heteroaryl der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie in Indolizinyl) ein N-Atom sein können,

bedeutet;

i) $R^8$

1. Wasserstoff,
2. $(C_1-C_6)$-Alkyl,

3. $(C_3-C_8)$-Cycloalkyl,
4. Phenyl-$(CH_2)_q$-,
5. $OR^5$,
6. $NR^{11}R^{12}$ oder
7.

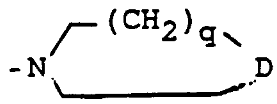

bedeutet;

j) $R^9$
1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

k) $R^{10}$ Cyano, Nitro oder $CO_2R^7$ bedeutet;

l) $R^{11}$ und $R^{12}$ gleich oder verschieden sind und
1. Wasserstoff,
2. $(C_1-C_4)$-Alkyl,
3. Phenyl,
4. Benzyl oder
5. $\alpha$-Methylbenzyl

bedeuten;

m) D $NR^{13}$, O oder $CH_2$ bedeutet;

n) $R^{13}$ Wasserstoff, $(C_1-C_4)$Alkyl oder Phenyl bedeutet;

o) A den Rest eines anellierten Heterobicyclus mit 8 bis 10 Ringatomen, wovon bis zu 9 Ringatome C-Atome sind, worin zwei nebeneinanderliegende Atome gemeinsame Bestandteile beider Ringe sind, wobei einer oder beide dieser Ringe sich formal vom Benzol ableiten, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind, bedeutet;

der mit bis zu 6 gleichen oder verschiedenen Resten $R^{14}$ oder $-(CH_2)_{n-1}-(CHR^6-CH_2)_{o-1}-R^{15}$ substituiert sein kann,

p) $R^{14}$
1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R^3$,
12. Methansulfonylamino,
13. Trifluormethansulfonylamino,
14. $-CO-NH-OR^9$,
15. $-SO_2-NR^6R^7$,
16. $-CH_2OR^7$,
17. $(C_1-C_9)$-Heteroaryl-$(CH_2)_q$-, wobei der Heteroarylteil wie unter h) 6. definiert ist,
18. $(C_7-C_{13})$-Aroyl,

19.

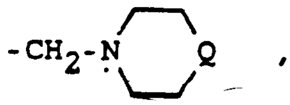

20.

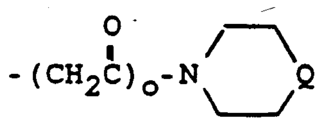

oder

21. $(C_6-C_{12})$-Aryl

bedeutet;

q) $R^{15}$

1. Wasserstoff,
2. $(C_1-C_6)$-Alkyl,
3. $(C_3-C_8)$-Cycloalkyl,
4. $(C_6-C_{12})$-Aryl,
5. $(C_7-C_{13})$-Aroyl,
6. $(C_1-C_4)$-Alkoxy,
7. $(C_1-C_4)$-Alkanoyloxy,
8. $(C_1-C_9)$-Heteroaryl, der wie unter h) 6. definiert ist
9. $CO_2R^3$,
10. Halogen,
11. Cyano
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. Phosphonooxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. den unter p) 20. definierten Rest,
29.

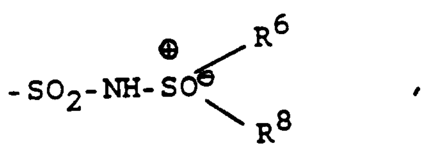

30.

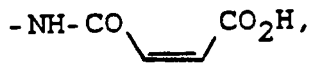

31.

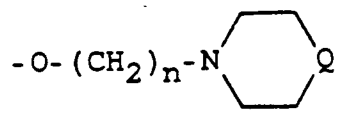 ,

32. 5-Tetrazolyl-NH-CO-,
33. $-CO-NH-NH-SO_2CF_3$,
34.

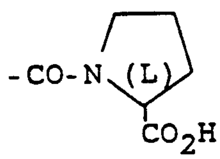 ,

35.

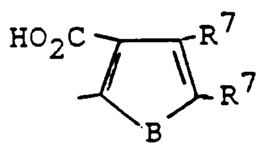 ,

36.

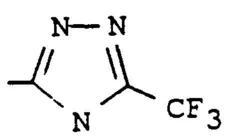 ,

37.

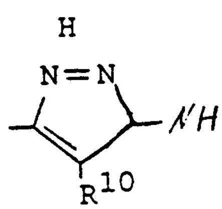 ,

38.

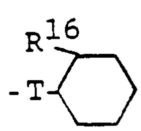 ,

39.

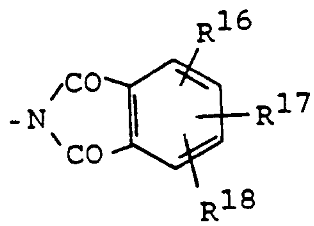

,

40. $-CO-NH-SO_2-R^{19}$

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r) B O, $NR^7$ oder S bedeutet;

s) W O oder S bedeutet;

t) L $(C_1-C_3)$-Alkandiyl bedeutet;

u) $R^{16}$ $CO_2R^3$ oder $CH_2CO_2R^3$ bedeutet;

v) $R^{17}$ Wasserstoff, Halogen, $(C_1-C_4)$-Alkyl oder $(C_1-C_4)$-Alkoxy bedeutet;

w) $R^{18}$ Wasserstoff, $(C_1-C_4)$-Alkyl oder Phenyl bedeutet;

x) $R^{19}$

1. $(C_1-C_8)$-Alkyl
2. $(C_3-C_8)$-Cycloalkyl
3. Phenyl
4. Benzyl oder
5. den unter x) 1. definierten Rest, worin 1 bis alle H-Atome durch Fluor oder Chlor substituiert sind,

bedeutet;

y) T

1. eine Einfachbindung,
2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. -CH = CH-
18. -CF = CF-,
19. -CH = CF-,
20. -CF = CH-,
21. $-CH_2-CH_2-$,
22. $-CF_2-CF_2-$,
23. $-CH(OR^3)-$
24. $-CH(OCOR^5)-$

25.

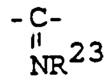

oder

26.

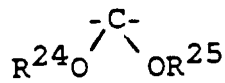

bedeutet;

z) $R^{20}$ und $R^{22}$ gleich oder verschieden sind und Wasserstoff, $(C_1$-$C_5)$-Alkyl, Phenyl, Allyl oder Benzyl bedeutet;

a') $R^{21}$ Wasserstoff, $(C_1$-$C_6)$Alkyl, Benzyl oder Allyl bedeutet;

b') $R^{23}$

1. $NR^{20}R^{21}$
2. Ureido,
3. Thioureido,
4. Toluol-4-sulfonyl oder
5. Benzolsulfonylamino

c') $R^{24}$ und $R^{25}$ gleich oder verschieden sind und $(C_1$-$C_4)$-Alkyl bedeuten oder gemeinsam für $-(CH_2)_q$-stehen;

d') Q $CH_2$, NH, O oder S bedeutet;

e') m eine ganze Zahl von 0 bis 5 ist,

f') n eine ganze Zahl von 1 bis 5 ist;

g') o eine ganze Zahl von 1 bis 10 ist;

h') q 0 oder 1 ist;

i') r 0, 1 oder 2 ist, oder

j') v eine ganze Zahl von 1 bis 6 ist;

sowie deren physiologisch verträglichen Salze.

**2.** Verbindung der Formel I gemäß Anspruch 1, in welcher

a) $R^1$

1. $(C_3$-$C_{10})$-Alkyl,
2. $(C_3$-$C_{10})$-Alkenyl,
3. $(C_3$-$C_{10})$-Alkinyl,
4. $(C_3$-$C_8)$-Cycloalkyl,
5. Benzyl oder
6. Benzyl, das wie im Anspruch 1 definiert substituiert ist,

bedeutet;

b) $R^2$

1. Wasserstoff,
2. Halogen,
3. Nitro,
4. $C_v F_{2v+1}$
5. Pentafluorphenyl
6. Cyano
7. Phenyl,
8. Phenyl-$(C_1$-$C_3)$-alkyl,
9. $(C_1$-$C_{10})$-Alkyl,
10. $(C_3$-$C_{10})$-Alkenyl,
11. Phenyl-$(C_2$-$C_6)$-alkenyl,
12. 1-Imidazolyl-$(CH_2)_m$-

13. 1,2,3-Triazolyl-$(CH_2)_O$-
14. Tetrazolyl-$(CH_2)_m$-
15. $-(CH_2)_{o-1}-CHR^7-OR^5$
16. $-(CH_2)_o-O-COR^3$,
17. $-COR^8$,
18. $-(CH_2)_o-CO-R^8$,
19. $-S(O)_rR^6$,
20. $-CH=CH-(CH_2)_m-CHR^3-OR^6$,
21. $-CH_2=CH-(CH_2)_m-CO-R^8$,
22. $-(CH_2)_o-NH-CO-OR^9$,
23. $-(CH_2)_o-NH-SO_2-R^9$,
24. $-(CH_2)_nF$,
25. $-(CH_2)_o-SO_3R^9$,
26. $-(CH_2)_n-SO_2-NH-CO-NR^6R^9$ oder
27. einem wie unter b) 7., 8., 9., 10. oder 13. definierten Rest, der wie in Anspruch 1 unter c) 46., 45. oder 44. jeweils wie für einen solchen Rest beschrieben substituiert ist,

bedeutet;

c) $R^8$ Wasserstoff; $(C_1-C_5)$-Alkyl, $OR^5$ oder $NR^{11}R^{12}$ oder Morpholino bedeutet;

d) T

1. eine Einfachbindung,
2. -CO-
3. $-CONR^{21}-$
4. $-CH_2-CH_2-$
5. $-NR^{21}-CO-$
6. $-O-CH_2-$
7. $-CH_2-O-$
8. $-S-CH_2-$
9. $-CH_2-S-$
10. $-NH-CH_2-$
11. $-CH_2-NH-$ oder
12. -CH=CH-

bedeutet und die übrigen Reste und Variablen wie im Anspruch 1 definiert sind, sowie deren physiologisch verträglichen Salze.

**3.** Verbindung der Formel I gemäß Anspruch 1 oder 2, in welcher

a) $R^1$ $(C_3-C_7)$-Alkyl, $(C_3-C_7)$-Alkenyl oder $(C_3-C_7)$-Alkinyl bedeutet;

b) $R^2$

1. Chlor,
2. Brom,
3. $C_v F_{2v+1}$ mit v = 1,2 oder 3,
4. Pentafluorphenyl,
5. $-S(O)_rR^6$,
6. $(CH_2)_{o-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. Tetrazolyl-$(CH_2)_m$-
14. $-(CH_2)_n$ $SO_2-NH-CO-NR^6R^9$,
15. $-(CH_2)_o-SO_3R^9$ oder

gegebenenfalls durch Hydroxy substituiertes $(C_1-C_6)$-Alkyl bedeutet;

c) $R^3$ Wasserstoff oder $(C_1-C_4)$-Alkyl bedeutet;

d) $R^6$ Wasserstoff, $(C_1-C_4)$-Alkyl, $(C_1-C_4)$-Alkanoyl, einen wie oben im Anspruch 1 unter g) 4., 6. oder 9. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, oder $(C_1-C_9)$-Heteroaryl, das sich von Phenyl oder Naphthyl ableitet, in welchem eine oder mehrere CH-Gruppen durch N

ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, bedeutet;

e) $R^7$ Wasserstoff, $(C_1-C_4)$-Alkyl, $(C_1-C_9)$-Heteroaryl,

das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,

oder

$(C_6-C_{12})$-Aryl-$(C_1-C_4)$-alkyl bedeutet;

f) $R^8$ Wasserstoff, $(C_1-C_4)$-Alkyl, $OR^5$ oder Morpholino bedeutet;

g) $R^9$ $CF_3$, $(C_1-C_6)$-Alkyl oder Phenyl bedeutet;

h) $R^{14}$

1. $(C_1-C_4)$-Alkyl,
2. $(C_1-C_4)$-Alkoxy,
3. Cyano,
4. Amino,
5. Nitroso,
6. Nitro,
7. Fluor,
8. Chlor,
9. Brom,
10. Hydroxy,
11. $CH_2OR^7$,
12. $(C_1-C_9)$-Heteroaryl-$CH_2$-, wobei der Heteroarylteil sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
13. $(C_1-C_4)$-Alkanoyloxy,
14. $(C_1-C_4)$Alkanoyl,
15. Benzoyl,
16.

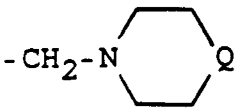

,

17. -NH-CO-$R^7$ oder
18. Tetrazolyl

bedeutet;

i) $R^{15}$

1. $(C_1-C_4)$-Alkyl,
2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
6. Cyano,
7. Nitro,
8. Hydroxy,
9. $-S(O)_rR^6$

10. $-SO_3R^3$
11. Chlor,
12. Brom,
13. Benzoyl,
14. $-CO_2R^3$,
15. $-CO-NH-R^6$,
16. $-NR^6R^7$,
17. $-CO-R^8$,
18. $-SO_2-NR^6R^7$,
19.

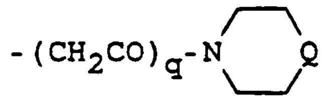

,

20.

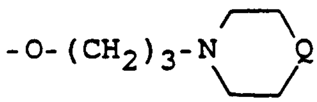

,

21. $-SO_2-NH-CO-NR^6R^9$,
22. $-PO_3H$
23. $-CO-CHR^5-CO_2H$,
24. $-NH-CO-NH-SO_2-CH_2-R^5$,
25. 5-Tetrazolyl-NH-CO-,
26.

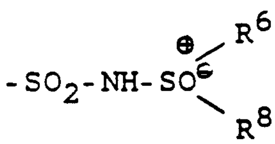

,

27.

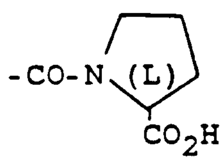

,

28.

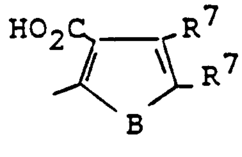

,

29.

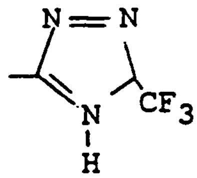

,

30.

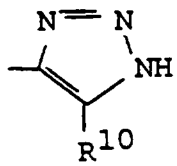

,

31.

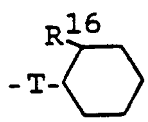

,

32.

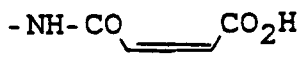

,

33.

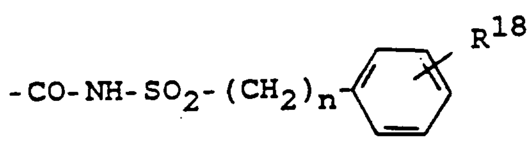

oder

34. den unter i) 2 definierten Rest, substituiert wie in Anspruch 1 definiert,

bedeutet;

j) Q $CH_2$, NH oder O bedeutet;

k) $R^{18}$ Wasserstoff, Methyl oder Ethyl bedeutet;

l) T eine Einfachbindung, -O-, -CO-, -NHCO- oder $-OCH_2$-bedeutet

und die übrigen Reste und Variablen wie im Anspruch 1 definiert sind, sowie deren physiologisch verträglichen Salze.

**4.** Verbindung gemäß einem der Ansprüche 1 bis 3, worin

a) X N, Y $CR^2$ und Z $CR^2$ bedeuten;

b) X $CR^2$, Y N und Z $CR^2$ bedeuten;

c) X $CR^2$, Y $CR^2$ und Z N bedeuten

oder

d) X, Y und Z jeweils N bedeuten, sowie deren physiologisch verträglichen Salze.

**5.** Verbindung gemäß einem oder mehreren der Ansprüche 1 bis 4, worin

a) $R^2$ Chlor, Brom, $-S(O)_rR^6$, $-COR^8$ oder $-(CH_2)_nSO_2-NH-CO-NR^6R^9$ bedeutet;
b) $R^9$ $(C_1-C_6)$-Alkyl bedeutet;
c) $R^{14}$ Tetrazolyl bedeutet;
d) $R^{15}$ $-CO_2-R^3$, $-SO_2-NR^6R^7$, $-SO_2-NH-CO-NR^6R^9$ oder $NH-CO-NH-SO_2-CH_2-R^5$ bedeutet;
e) Z gleich N ist;
f) X und Y beide $CR_2$ bedeuten;
g) q Null ist;
h) L $CH_2$ bedeutet;
i) A ein Rest von Benzothiophen, Benzofuran, Indol, Isoindol, Indazol, Benzimidazol, Chinolin, Isochinolin, Phthalazin, Chinoxalin, Chinazolin, Cinnolin, Benzthiazol, Benzothiazol-1,1-dioxid, Cumarin, Chroman, Benzoxazol, Benzisothiazol, Benzodiazine, Benztriazol, Benztriazin, Benzoxazin, Imidazo-pyridin, Imidazo-pyrimidin, Imidazo-pyrazin, Imidazo-pyridazin, Imidazo-thiazol, Pyrazolopyridin, Thienopyridin und Pyrrolopyrimidin bedeutet
der mit bis zu 6 gleichen oder verschiedenen Resten $R^{14}$ oder
$-(CH_2)_{n-1}-(CHR^6-CH_2)_{o-1}-R^{15}$ substituiert sein kann,

und die übrigen Reste und Variablen wie im Anspruch 3 definiert sind, sowie deren physiologisch verträglichen Salze.

**6.** Verfahren zur Herstellung einer Verbindung der Formel I gemäß einem der Ansprüche 1 bis 4, dadurch gekennzeichnet, daß man eine Verbindung der Formel II

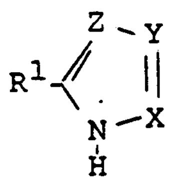

(II)

worin $R^1$, X, Y und Z wie im Anspruch 1 definiert sind, umsetzt mit einer Verbindung der Formel III

$U-L-(O)_q-A$ (III)

worin L, A und q wie im Anspruch 1 definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

**7.** Verbindung gemäß einem der Ansprüche 1-5 zur Anwendung als Heilmittel.

**8.** Verbindung gemäß einem der Ansprüche 1-5 zur Anwendung als Heilmittel zur Behandlung des Bluthochdrucks.

**9.** Pharmazeutische Zubereitung enthaltend eine Verbindung gemäß einem der Ansprüche 1-5.

**10.** Verfahren zur Herstellung einer Zubereitung gemäß Anspruch 9, dadurch gekennzeichnet, daß man eine Verbindung gemäß einem der Ansprüche 1-5 zusammen mit einem physiologisch unbedenklichen Träger und gegebenenfalls weiteren Zusatz- oder Hilfsstoffen in eine geeignete Darreichungsform bringt.

**11.** Verbindung der Formel

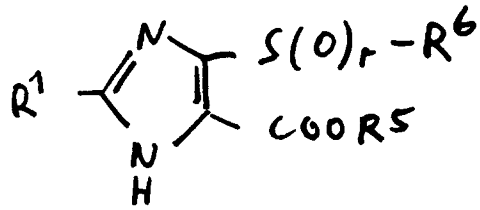

worin

a) $R^1$ n-Butyl bedeutet;
b) $R^6$ Methyl bedeutet;
c) $R^5$ Wasserstoff oder Ethyl bedeutet und
d) r 0, 1 oder 2 ist.

**12.** Verbindung der Formel gemäß Anspruch 11 wobei r gleich 0 ist und $R^5$ Ethyl bedeutet.

**Patentansprüche für folgenden Vertragsstaat : ES**

**1.** Verfahren zur Herstellung einer Verbindung der Formel I

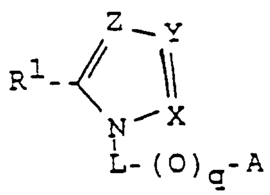

(I)

in welcher

a) X, Y und Z gleich oder verschieden sind und N oder $CR^2$ bedeuten,
b) $R^1$
1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,
bedeutet;
c) $R^2$
1. Wasserstoff,
2. Halogen,
3. Nitro,
4. $C_vF_{2v+1}$,
5. Pentafluorphenyl,
6. Cyano,

7. Phenyl,
8. Phenyl-($C_1$-$C_3$)-alkyl,
9. ($C_1$-$C_{10}$)-Alkyl,
10. ($C_3$-$C_{10}$)-Alkenyl,
11. Phenyl-($C_2$-$C_6$)-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. -$(CH_2)_{o-1}$-$CHR^7$-$OR^5$,
16. -$(CH_2)_o$-O-CO-$R^3$,
17. -$(CH_2)_o$-S-$R^6$,
18. -$S(O)_r$-$R^6$,
19. -CH = CH-$(CH_2)_m$-$CHR^3$-$OR^6$,
20. -$CH_2$ = CH-$(CH_2)_m$-CO-$R^8$,
21. -CO-$R^8$,
22. -CH = CH-$(CH_2)_m$-O-CO-$R^7$,
23. -$(CH_2)_m$-CH($CH_3$)-CO-$R^8$,
24. -$(CH_2)_o$-CO-$R^8$,
25.

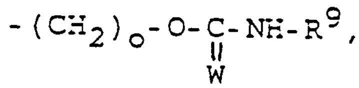

26.

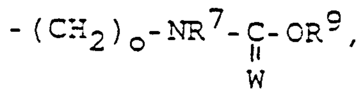

27. -$(CH_2)_o$-$NR^7$-CO-$NHR^9$,
28. -$(CH_2)_o$-$NR^7$-$SO_2R^9$,
29.

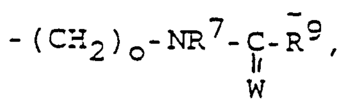

30. -$(CH_2)_n$F,
31. -$(CH_2)_n$-O-$NO_2$,
33. -$CH_2$-$N_3$,
34. -$(CH_2)_n$-$NO_2$,
35. -CH = N-$NR^5R^7$,
36. Phthalimido-$(CH_2)_n$-,
37.

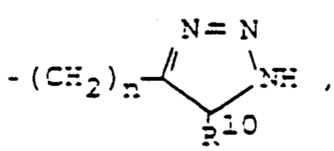

38.

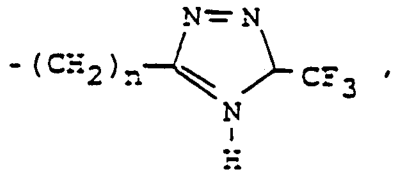

39

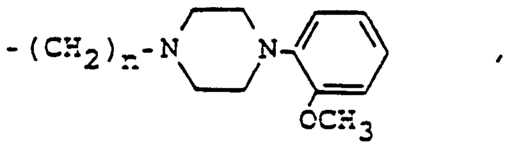

.
40.

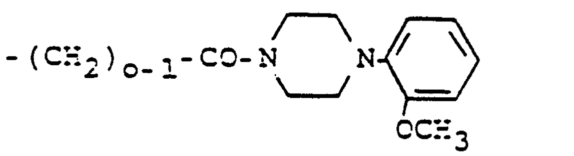

41. Phenyl-$SO_2$-NH-N = CH-,
42.

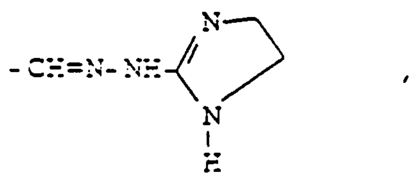

43. -$(CH_2)_n$-$SO_2$-$NR^7$-CO-$NR^6R^9$,
44. -$(CH_2)_o$-$SO_2R^9$,
44. einem wie unter c) 7. oder 8. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
45. einem wie unter c) 9., 10., oder 18. definierten Rest, worin 1 bis alle H-Atome durch Fluor substituiert sind, oder
46. den unter c) 13. definierten Rest, der mit 1 oder 2 gleichen oder verschiedenen Resten aus der Reihe Methoxycarbonyl und ($C_1$-$C_4$)-Alkyl substituiert ist,
bedeutet;

d) $R^3$

1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,
bedeuten;

e) $R^4$
1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl
bedeutet;
f) $R^5$
1. Wasserstoff,
2. ($C_1$-$C_6$)-Alkyl,
3. ($C_3$-$C_8$)-Cycloalkyl,
4. Phenyl oder
5. Benzyl
bedeutet;
g) $R^6$
1. Wasserstoff,
2. ($C_1$-$C_6$)-Alkyl,
3. ($C_3$-$C_8$)-Cycloalkyl,
4. ($C_6$-$C_{12}$)-Aryl,
5. Benzyl,
6. ($C_1$-$C_9$)-Heteroaryl,der teilweise oder vollständig hydriert sein kann, und der
sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können.
7. ($C_1$-$C_4$)-Alkanoyl,
8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, $NR^{11}R^{12}$ oder

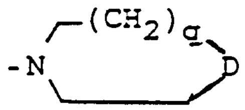

bedeutet;
9. ($C_1$-$C_9$)-Heteroaryl-($C_1$-$C_3$)-Alkyl, wobei der Heteroarylteil wie unter g) 6. definiert ist.
h) $R^7$
1. Wasserstoff,
2. ($C_1$-$C_6$)-Alkyl,
3. ($C_3$-$C_8$)-Cycloalkyl,
4. ($C_6$-$C_{12}$)-Aryl-($C_1$-$C_6$)-alkyl,
5. Phenyl oder
6. ($C_1$-$C_9$)-Heteroaryl der
sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
bedeutet;
i) $R^8$
1. Wasserstoff,
2. ($C_1$-$C_6$)-Alkyl,
3. ($C_3$-$C_8$)-Cycloalkyl,
4. Phenyl-$(CH_2)_q$-,
5. $OR^5$,
6. $NR^{11}R^{12}$ oder

7.

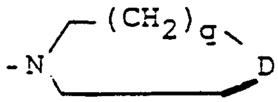

bedeutet;

j) $R^9$

1. $(C_1-C_6)$-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,

bedeutet;

k) $R^{10}$ Cyano, Nitro oder $CO_2R^7$ bedeutet;

l) $R^{11}$ und $R^{12}$ gleich oder verschieden sind und

1. Wasserstoff,
2. $(C_1-C_4)$-Alkyl,
3. Phenyl,
4. Benzyl oder
5. α-Methylbenzyl

bedeuten;

m) D $NR^{13}$, O oder $CH_2$ bedeutet;

n) $R^{13}$ Wasserstoff, $(C_1-C_4)$Alkyl oder Phenyl bedeutet;

o) A den Rest eines anellierten Heterobicyclus mit 8 bis 10 Ringatomen, wovon bis zu 9 Ringatome C-Atome sind, worin zwei nebeneinanderliegende Atome gemeinsame Bestandteile beider Ringe sind, wobei einer oder beide dieser Ringe sich formal vom Benzol ableiten, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind , bedeutet;

der mit bis zu 6 gleichen oder verschiedenen Resten $R^{14}$ oder $-(CH_2)_{n-1}-(CHR^6-CH_2)_{o-1}-R^{15}$ substituiert sein kann,

p) $R^{14}$

1. Halogen,
2. Oxo,
3. Nitroso,
4. Nitro,
5. Amino,
6. Cyano,
7. Hydroxy,
8. $(C_1-C_6)$-Alkyl,
9. $(C_1-C_4)$-Alkanoyl,
10. $(C_1-C_4)$-Alkanoyloxy,
11. $CO_2R^3$,
12. Methansulfonylamino,
13. Trifluormethansulfonylamino,
14. -CO-NH-$OR^9$,
15. -$SO_2$-$NR^6R^7$,
16. -$CH_2OR^7$,
17. $(C_1-C_9)$-Heteroaryl-$(CH_2)_q$-, wobei der Heteroarylteil wie unter g) 6 definiert ist;
18. $(C_7-C_{13})$-Aroyl,

19.

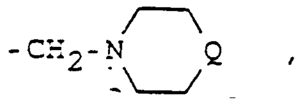

20.

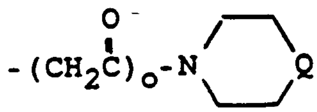

oder

21. ($C_6$-$C_{12}$)-Aryl bedeutet;

q) $R^{15}$

1. Wasserstoff,
2. ($C_1$-$C_6$)-Alkyl,
3. ($C_3$-$C_8$)-Cycloalkyl,
4. ($C_6$-$C_{12}$)-Aryl,
5. ($C_7$-$C_{13}$)-Aroyl,
6. ($C_1$-$C_4$)-Alkoxy,
7. ($C_1$-$C_4$)-Alkanoyloxy,
8. ($C_1$-$C_9$)-Heteroaryl, der wie unter h) 6. definiert ist,
9. $CO_2R^3$,
10. Halogen,
11. Cyano
12. Nitro,
13. $NR^6R^7$,
14. Hydroxy,
15. -CO-NH-CHR$^5$-$CO_2R^3$,
16. Sulfo,
17. -$SO_3R^3$,
18. -$SO_2$-$NR^7$-CO-$NR^6R^9$,
19. -$NR^7$-CO-$NR^6$-$SO_2$-$CH_2$-$R^5$,
20. -$C(CF_3)_2OH$,
21. Phosphonooxy,
22. -$PO_3H_2$,
23. -NH-$PO(OH)_2$,
24. -$S(O)_rR^6$,
25. -CO-$R^8$,
26. -CO-$NR^6R^9$,
27. -$CR^{20}(OH)$-$PO(OH)_2$,
28. den unter p) 20. definierten Rest,
29.

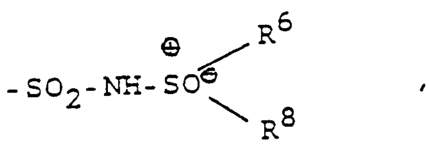

30.

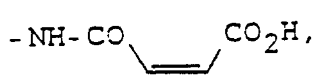

31.

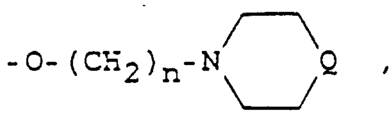

32. 5-Tetrazolyl-NH-CO-,
33. -CO-NH-NH-$SO_2CF_3$,
34.

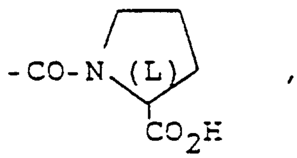

35.

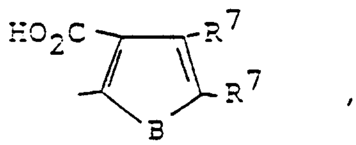

36.

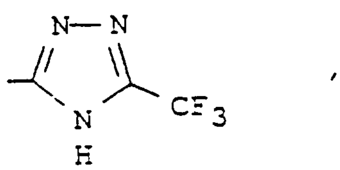

37.

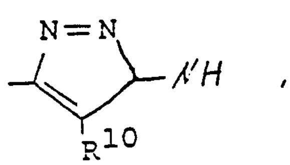

38.

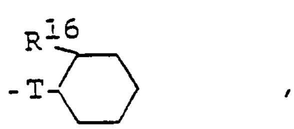

39.

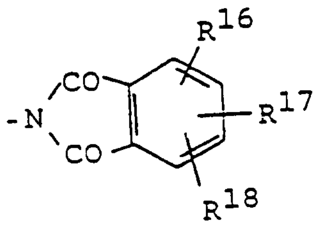

,

40. $-CO-NH-SO_2-R^{19}$

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r) B O, $NR^7$ oder S bedeutet;

s) W O oder S bedeutet;

t) L $(C_1-C_3)$-Alkandiyl bedeutet;

u) $R^{16}$ $CO_2R^3$ oder $CH_2CO_2R^3$ bedeutet;

v) $R^{17}$ Wasserstoff, Halogen, $(C_1-C_4)$-Alkyl oder $(C_1-C_4)$-Alkoxy bedeutet;

w) $R^{18}$ Wasserstoff, $(C_1-C_4)$-Alkyl oder Phenyl bedeutet;

x) $R^{19}$

1. $(C_1-C_8)$-Alkyl
2. $(C_3-C_8)$-Cycloalkyl
3. Phenyl
4. Benzyl oder
5. den unter x) 1. definierten Rest, worin 1 bis alle H-Atome durch Fluor oder Chlor substituiert sind,

bedeutet;

y) T

1. eine Einfachbindung,
2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. -CH = CH-
18. -CF = CF-,
19. -CH = CF-,
20. -CF = CH-,
21. $-CH_2-CH_2-$,
22. $-CF_2-CF_2-$,
23. $-CH(OR^3)-$
24. $-CH(OCOR^5)-$
25.

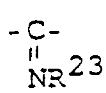

oder
26.

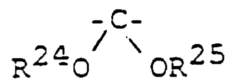

bedeutet;

z) $R^{20}$ und $R^{22}$ gleich oder verschieden sind und Wasserstoff, $(C_1-C_5)$-Alkyl, Phenyl, Allyl oder Benzyl bedeutet;

a') $R^{21}$ Wasserstoff, $(C_1-C_6)$Alkyl, Benzyl oder Allyl bedeutet;

b') $R^{23}$

1. $NR^{20}R^{21}$
2. Ureido,
3. Thioureido,
4. Toluol-4-sulfonyl oder
5. Benzolsulfonylamino

c') $R^{24}$ und $R^{25}$ gleich oder verschieden sind und $(C_1-C_4)$-Alkyl bedeuten oder gemeinsam für $-(CH_2)_q$-stehen;

d') Q $CH_2$, NH, O oder S bedeutet;

e') m eine ganze Zahl von 0 bis 5 ist,

f') n eine ganze Zahl von 1 bis 5 ist;

g') o eine ganze Zahl von 1 bis 10 ist;

h') q 0 oder 1 ist;

i') r 0, 1 oder 2 ist, oder

j') v eine ganze Zahl von 1 bis 6 ist;

dadurch gekennzeichnet, daß man eine Verbindung der Formel II

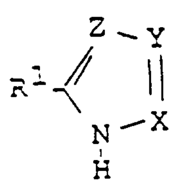

(II)

worin $R^1$, X, Y und Z wie oben definiert sind, unsetzt mit einer Verbindung der Formel III

$U-L-(O)_q-A$ (III)

worin L, A und q wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

**2.** Verfahren gemaß Anspruch 1, wobei in Formel I bedeuten:

a) $R^1$

1. $(C_3-C_{10})$-Alkyl,
2. $(C_3-C_{10})$-Alkenyl,
3. $(C_3-C_{10})$-Alkinyl,
4. $(C_3-C_8)$-Cycloalkyl,
5. Benzyl oder
6. Benzyl, das wie im Anspruch 1 definiert substituiert ist,

bedeutet;

b) $R^2$

1. Wasserstoff,
2. Halogen,
3. Nitro,
4. $C_v F_{2v+1}$

5. Pentafluorphenyl
6. Cyano
7. Phenyl,
8. Phenyl-($C_1$-$C_3$)-alkyl,
9. ($C_1$-$C_{10}$)-Alkyl,
10. ($C_3$-$C_{10}$)-Alkenyl,
11. Phenyl-($C_2$-$C_6$)-alkenyl,
12. 1-Imidazolyl-$(CH_2)_m$-
13. 1,2,3-Triazolyl-$(CH_2)_O$-
14. Tetrazolyl-$(CH_2)_m$-
15. -$(CH_2)_{o-1}$-$CHR^7$-$OR^5$
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ oder
27. einem wie unter b) 7., 8., 9., 10. oder 13. definierten Rest, der wie in Anspruch 1 unter c) 46., 45. oder 44. jeweils wie für einen solchen Rest beschrieben substituiert ist,

bedeutet;

c) $R^8$ Wasserstoff; ($C_1$-$C_5$)-Alkyl, $OR^5$ oder $NR^{11}R^{12}$ oder Morpholino bedeutet;

d) T
1. eine Einfachbindung,
2. -CO-
3. -$CONR^{21}$-
4. -$CH_2$-$CH_2$-
5. -$NR^{21}$-CO-
6. -O-$CH_2$-
7. -$CH_2$-O-
8. -S-$CH_2$-
9. -$CH_2$-S-
10. -NH-$CH_2$-
11. -$CH_2$-NH- oder
12. -CH = CH-

bedeutet und die übrigen Reste und Variablen wie im Anspruch 1 definiert sind, sowie deren physiologisch verträglichen Salze.

**3.** Verfahren gemäß Anspruch 1 oder 2, wobei die Symbole in Formel (I) bedeuten:

a) $R^1$ ($C_3$-$C_7$)-Alkyl, ($C_3$-$C_7$)-Alkenyl oder ($C_3$-$C_7$)-Alkinyl bedeutet;

b) $R^2$
1. Chlor,
2. Brom,
3. $C_v F_{2v+1}$ mit v = 1,2 oder 3,
4. Pentafluorphenyl,
5. -$S(O)_rR^6$,
6. $(CH_2)_{o-1}$-$CHR^7$-$OR^5$,
7. $(CH_2)_o$-O-CO-$R^3$,
8. -$COR^8$,
9. -$(CH_2)_o$-CO-$R^8$,
10. -$CH_2$-NH-CO-$R^8$,
11. -$(CH_2)_o$-NH-$SO_2$-$R^9$,
12. -CH = CH-$CHR^3$-$OR^6$,
13. Tetrazolyl-$(CH_2)_m$-

14. $-(CH_2)_n SO_2-NH-CO-NR^6R^9$,
15. $-(CH_2)_o-SO_3R^9$ oder
gegebenenfalls durch Hydroxy substituiertes $(C_1-C_6)$-Alkyl bedeutet;

c) $R^3$ Wasserstoff oder $(C_1-C_4)$-Alkyl bedeutet;

d) $R^6$ Wasserstoff, $(C_1-C_4)$-Alkyl, $(C_1-C_4)$-Alkanoyl, einen wie oben im Anspruch 1 unter g) 4., 6. oder 9. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, oder $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, bedeutet;

e) $R^7$ Wasserstoff, $(C_1-C_4)$-Alkyl, $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, oder $(C_6-C_{12})$-Aryl-$(C_1-C_4)$-Alkyl bedeutet;

f) $R^8$ Wasserstoff, $(C_1-C_4)$-Alkyl, $OR^5$ oder Morpholino bedeutet,

g) $R^9$ $CF_3$, $(C_1-C_6)$-Alkyl oder Phenyl bedeutet;

h) $R^{14}$
1. $(C_1-C_4)$-Alkyl,
2. $(C_1-C_4)$-Alkoxy,
3. Cyano,
4. Amino,
5. Nitroso,
6. Nitro,
7. Fluor,
8. Chlor,
9. Brom,
10. Hydroxy,
11. $CH_2OR^7$,
12. $(C_1-C_9)$-Heteroaryl-$CH_2$-, wobei der Heteroarylteil sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
13. $(C_1-C_4)$-Alkanoyloxy,
14. $(C_1-C_4)$Alkanoyl,
15. Benzoyl,
16.

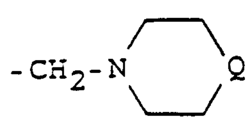
,

17. $-NH-CO-R^7$ oder
18. Tetrazolyl
bedeutet;

i) $R^{15}$
1. $(C_1-C_4)$-Alkyl,
2. $(C_6-C_{12})$-Aryl,
3. $(C_1-C_3)$-Alkanoyloxy,
4. $(C_1-C_4)$-Alkoxy,
5. $(C_1-C_9)$-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im

Indolizinyl) ein N-Atom sein können,
6. Cyano,
7. Nitro,
8. Hydroxy,
9. $-S(O)_rR^6$
10. $-SO_3R^3$
11. Chlor,
12. Brom,
13. Benzoyl,
14. $-CO_2R^3$,
15. $-CO-NH-R^6$,
16. $-NR^6R^7$,
17. $-CO-R^8$,
18. $-SO_2-NR^6R^7$,
19.

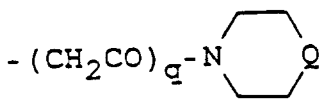

,

20.

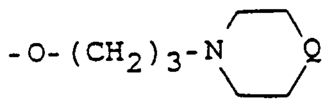

,

21. $-SO_2-NH-CO-NR^6R^9$,
22. $-PO_3H$
23. $-CO-CHR^5-CO_2H$,
24. $-NH-CO-NH-SO_2-CH_2-R^5$,
25. 5-Tetrazolyl-NH-CO-,
26.

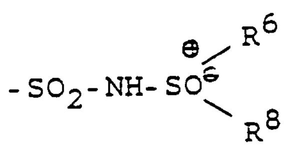

,

27.

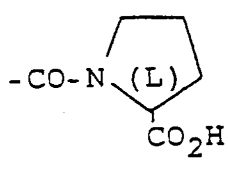

,

28.

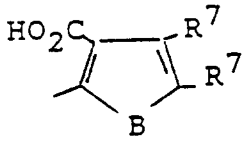

,

29.

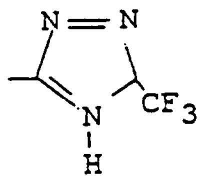

,

30.

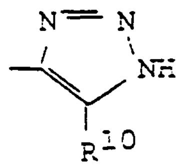

,

31.

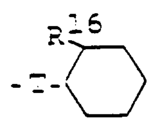

,

32.

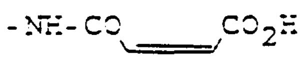

,

33.

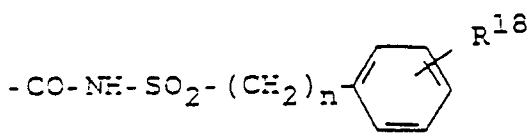

oder

34. den unter i) 2 definierten Rest, substituiert wie in Anspruch 1 definiert, bedeutet;

j) Q $CH_2$, NH oder O bedeutet;

k) $R^{18}$ Wasserstoff, Methyl oder Ethyl bedeutet;

l) T eine Einfachbindung, -O-, -CO-, -NHCO- oder $-OCH_2$-bedeutet

und die übrigen Reste und Variablen wie im Anspruch 1 definiert sind.

**4.** Verfahren gemäß einem der Ansprüche 1 bis 3, wobei die Symbole in Formel (I) bedeuten:

a) X N, Y $CR^2$ und Z $CR^2$ bedeuten;

b) X $CR^2$, Y N und Z $CR^2$ bedeuten;

c) X $CR^2$, Y $CR^2$ und Z N bedeuten oder

d) X, Y und Z jeweils N bedeuten.

**5.** Verfahren gemäß einem oder mehreren der Ansprüche 1 bis 4, worin

a) $R^2$ Chlor, Brom, $-S(O)_rR^6$, $-COR^8$ oder $-(CH_2)_nSO_2$-NH-CO-$NR^6R^9$ bedeutet;

b) $R^9$ ($C_1$-$C_6$)-Alkyl bedeutet;

c) $R^{14}$ Tetrazolyl bedeutet;
d) $R^{15}$ $-CO_2-R^3$, $-SO_2-NR^6R^7$, $-SO_2-NH-CO-NR^6R^9$ oder $-NH-CO-NH-SO_2-CH_2-R^5$ bedeutet;
e) Z gleich N ist;
f) X und Y beide $CR_2$ bedeuten;
g) q Null ist;
h) L $CH_2$ bedeutet;
i) A ein Rest von Benzothiophen, Benzofuran, Indol, Isoindol, Indazol, Benzimidazol, Chinolin, Isochinolin, Phthalazin, Chinoxalin, Chinazolin, Cinnolin, Benzthiazol, Benzothiazol-1,1-dioxid, Cumarin, Chroman, Benzoxazol, Benzisothiazol, Benzodiazine, Benztriazol, Benztriazin, Benzoxazin, Imidazo-pyridin, Imidazo-pyrimidin, Imidazo-pyrazin, Imidazo-pyridazin, Imidazo-thiazol, Pyrazolopyridin, Thienopyridin und Pyrrolopyrimidin bedeutet
der mit bis zu 6 gleichen oder verschiedenen Resten $R^{14}$ oder
$-(CH_2)_{n-1}-(CHR^6-CH_2)_{o-1}-R^{15}$ substituiert sein kann,
und die übrigen Reste und Variablen wie im Anspruch 3 definiert sind.

**6.** Verfahren zur Herstellung einer pharmazeutischen Zubereitung dadurch gekennzeichnet, daß man eine Verbindung hergestellt gemäß einem der Ansprüche 1 - 5 zusammen mit einem physiologisch unbedenklichen Träger und gegebenenfalls weiteren Zusatz- oder Hilfsstoffen in eine geeignete Darreichungsform bringt.

**7.** Verfahren zur Herstellung einer Verbindung der Formel (II')

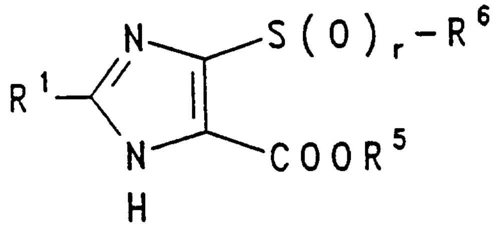

(II')

worin
a) $R^1$ n-Butyl bedeutet;
b) $R^6$ Methyl bedeutet;
c) $R^5$ Wasserstoff oder Ethyl bedeutet und
d) r 0, 1 oder 2 ist;
dadurch gekennzeichnet, daß ausgehend von einer Verbindung der Formel (III)

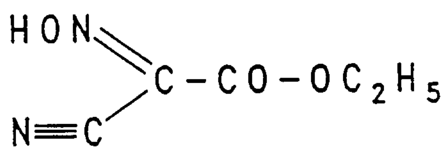

(III)

diese durch anschließende
- Reduktion zum Aminonitril
- Einführung einer Alkylgruppe mittels Acylierung,
- Addition einer Alkylmercaptangruppe an die Cyanogruppe und Ringschluß und gegebenenfalls zusätzlicher Oxidierung der Thioalkylgruppe und/oder Hydrolyse der Estergruppe in eine Verbindung der Formel (II') übergeführt wird.

**8.** Verfahren zur Herstellung einer Verbindung der Formel (II') gemäß Anspruch 7, wobei r gleich 0 ist und $R^5$ Ethyl bedeutet.

**Patentansprüche für folgenden Vertragsstaat : GR**

1. Verfahren zur Herstellung einer Verbindung der Formel I

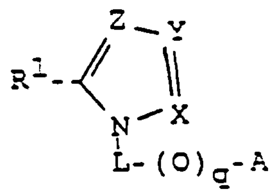

(I)

in welcher

a) X, Y und Z gleich oder verschieden sind und N oder $CR^2$ bedeuten,

b) $R^1$

1. $(C_2-C_{10})$-Alkyl,
2. $(C_3-C_{10})$-Alkenyl,
3. $(C_3-C_{10})$-Alkinyl,
4. $OR^3$,
5. $(C_3-C_8)$-Cycloalkyl,
6. $(C_4-C_{10})$-Cycloalkylalkyl,
7. $(C_5-C_{10})$-Cycloalkylalkenyl,
8. $(C_5-C_{10})$-Cycloalkylalkinyl,
9. $-(CH_2)_m-B-(CH_2)_n-R^4$,
10. Benzyl,
11. einem wie unter b) 1., 2., 3. oder 9. definierten Rest, der monosubstituiert ist mit $CO_2R^3$,
12. einem wie unter b) 1., 2., 3. oder 9. definierten Rest, worin 1 bis alle H-Atome durch Fluor substituiert sind, oder
13. den unter b) 10. definierten Rest, der am Phenyl mit 1 oder 2 gleichen oder verschiedenen Resten aus der Reihe Halogen, $(C_1-C_4)$-Alkoxy und Nitro substituiert ist,

bedeutet;

c) $R^2$

1. Wasserstoff,
2. Halogen,
3. Nitro,
4. $C_vF_{2v+1}$,
5. Pentafluorphenyl,
6. Cyano,
7. Phenyl,
8. Phenyl-$(C_1-C_3)$-alkyl,
9. $(C_1-C_{10})$-Alkyl,
10. $(C_3-C_{10})$-Alkenyl,
11. Phenyl-$(C_2-C_6)$-Alkenyl,
12. 1-Imidazolyl-$(CH_2)_m$-,
13. 1,2,3-Triazolyl-$(CH_2)_n$-,
14. Tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,

25.

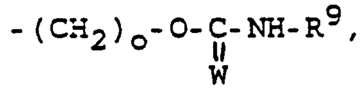

26.

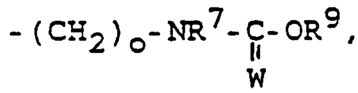

27. $-(CH_2)_o-NR^7-CO-NHR^9$,
28. $-(CH_2)_o-NR^7-SO_2R^9$,
29.

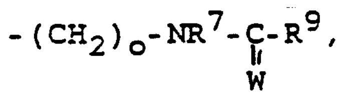

30. $-(CH_2)_nF$,
31. $-(CH_2)_n-O-NO_2$,
33. $-CH_2-N_3$,
34. $-(CH_2)_n-NO_2$,
35. $-CH=N-NR^5R^7$,
36. Phthalimido-$(CH_2)_n$-,
37.

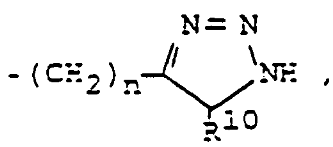

38.

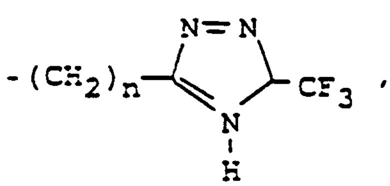

39.

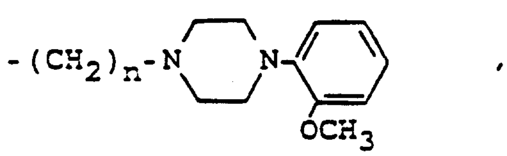

40.

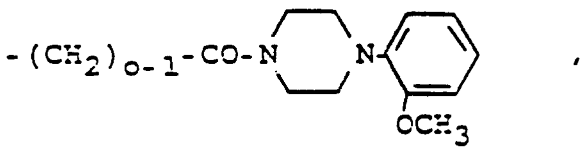

41. Phenyl-$SO_2$-NH-N = CH-,
42.

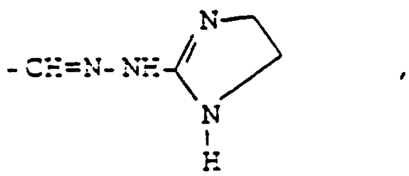

43. -$(CH_2)_n$-$SO_2$-$NR^7$-CO-$NR^6R^9$,
44. -$(CH_2)_o$-$SO_2R^9$,
44. einem wie unter c) 7. oder 8. definierten Rest, der am Phenyl mit 1 oder 2 gleichen oder verschiedenen Resten aus der Reihe Halogen, Hydroxy, Methoxy, Trifluormethyl, $CO_2R^3$ und Phenyl substituiert ist,
45. einem wie unter c) 9., 10., oder 18. definierten Rest, worin 1 bis alle H-Atome durch Fluor substituiert sind, oder
46. den unter c) 13. definierten Rest, der mit 1 oder 2 gleichen oder verschiedenen Resten aus der Reihe Methoxycarbonyl und ($C_1$-$C_4$)-Alkyl substituiert ist,
bedeutet;

d) $R^3$
1. Wasserstoff,
2. ($C_1$-$C_8$)-Alkyl,
3. ($C_3$-$C_8$)-Cycloalkyl,
4. Phenyl,
5. Benzyl oder
6. den unter d) 2. definierten Rest, worin 1 bis alle H-Atome durch Fluor substituiert sind,
bedeuten;

e) $R^4$
1. Wasserstoff,
2. ($C_1$-$C_6$)-Alkyl,
3. ($C_3$-$C_8$)-Cycloalkyl,
4. ($C_2$-$C_4$)-Alkenyl oder
5. ($C_2$-$C_4$)-Alkinyl
bedeutet;

f) $R^5$
1. Wasserstoff,
2. ($C_1$-$C_6$)-Alkyl,
3. ($C_3$-$C_8$)-Cycloalkyl,
4. Phenyl oder
5. Benzyl
bedeutet;

g) $R^6$
1. Wasserstoff,
2. ($C_1$-$C_6$)-Alkyl,
3. ($C_3$-$C_8$)-Cycloalkyl,
4. ($C_6$-$C_{12}$)-Aryl,
5. Benzyl,

6. ($C_1$-$C_9$)-Heteroaryl, der teilweise oder vollständig hydriert sein kann, und der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können.
7. ($C_1$-$C_4$)-Alkanoyl,
8. einen wie unter g) 4. oder 6. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, $NR^{11}R^{12}$ oder

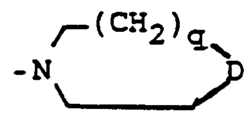

bedeutet;
9. ($C_1$-$C_9$)-Heteroaryl-($C_1$-$C_3$)-Alkyl, wobei der Heteroarylteil wie unter g) 6. definiert ist.

h) $R^7$
1. Wasserstoff,
2. ($C_1$-$C_6$)-Alkyl,
3. ($C_3$-$C_8$)-Cycloalkyl,
4. ($C_6$-$C_{12}$)-Aryl-($C_1$-$C_6$)-alkyl,
5. Phenyl oder
6. ($C_1$-$C_9$)-Heteroaryl der sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
bedeutet;

i) $R^8$
1. Wasserstoff,
2. ($C_1$-$C_6$)-Alkyl,
3. ($C_3$-$C_8$)-Cycloalkyl,
4. Phenyl-$(CH_2)_q$-,
5. $OR^5$,
6. $NR^{11}R^{12}$ oder
7.

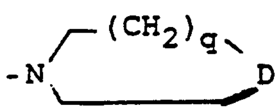

bedeutet;

j) $R^9$
1. ($C_1$-$C_6$)-Alkyl,
2. 1-Adamantyl,
3. 1-Naphthyl,
4. 1-Naphthylethyl,
5. Phenyl-$(CH_2)_q$- oder
6. den unter j) 1. definierten Rest, worin 1 bis alle H-Atome durch Fluor substituiert sind,
bedeutet;

k) $R^{10}$ Cyano, Nitro oder $CO_2R^7$ bedeutet;

l) $R^{11}$ und $R^{12}$ gleich oder verschieden sind und
1. Wasserstoff,
2. ($C_1$-$C_4$)-Alkyl,
3. Phenyl,

4. Benzyl oder
5. $\alpha$-Methylbenzyl
bedeuten;

m) D $NR^{13}$, O oder $CH_2$ bedeutet;

n) $R^{13}$ Wasserstoff, $(C_1\text{-}C_4)$Alkyl oder Phenyl bedeutet;

o) A den Rest eines anellierten Heterobicyclus mit 8 bis 10 Ringatomen, wovon bis zu 9 Ringatome C-Atome sind, worin zwei nebeneinanderliegende Atome gemeinsame Bestandteile beider Ringe sind, wobei einer oder beide dieser Ringe sich formal vom Benzol ableiten, in welchem eine oder mehrere CH-Gruppen durch N, $O^+$ und $S^+$ ersetzt sind und/oder in welchem zwei benachbarte CH-Gruppen (unter Bildung eines fünfgliedrigen aromatischen Rings) durch S, NH oder O ersetzt sind, bedeutet;
der mit bis zu 6 gleichen oder verschiedenen Resten $R^{14}$ oder $-(CH_2)_{n-1}-(CHR^6-CH_2)_{o-1}-R^{15}$ substituiert sein kann,

p) $R^{14}$
1. Halogen,
2. Oxo,
3. Nitroso,
4. Nitro,
5. Amino,
6. Cyano,
7. Hydroxy,
8. $(C_1\text{-}C_6)$-Alkyl,
9. $(C_1\text{-}C_4)$-Alkanoyl,
10. $(C_1\text{-}C_4)$-Alkanoyloxy,
11. $CO_2R^3$,
12. Methansulfonylamino,
13. Trifluormethansulfonylamino,
14. $-CO-NH-OR^9$,
15. $-SO_2-NR^6R^7$,
16. $-CH_2OR^7$,
17. $(C_1\text{-}C_9)$-Heteroaryl-$(CH_2)_q$-, wobei der Heteroarylteil wie unter g) 6 definiert ist;
18. $(C_7\text{-}C_{13})$-Aroyl,
19.

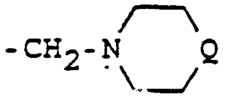

,

20.

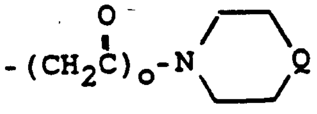

oder
21. $(C_6\text{-}C_{12})$-Aryl bedeutet;

q) $R^{15}$
1. Wasserstoff,
2. $(C_1\text{-}C_6)$-Alkyl,
3. $(C_3\text{-}C_8)$-Cycloalkyl,
4. $(C_6\text{-}C_{12})$-Aryl,
5. $(C_7\text{-}C_{13})$-Aroyl,
6. $(C_1\text{-}C_4)$-Alkoxy,
7. $(C_1\text{-}C_4)$-Alkanoyloxy,
8. $(C_1\text{-}C_9)$-Heteroaryl, der wie unter h) 6. definiert ist,

9. $CO_2R^3$,
10. Halogen,
11. Cyano
12. Nitro,
13. $NR^6R^7$,
14. Hydroxy,
15. $-CO-NH-CHR^5-CO_2R^3$,
16. Sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. Phosphonooxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. den unter p) 20. definierten Rest,
29.

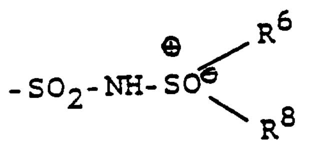

,

30.

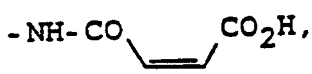

31.

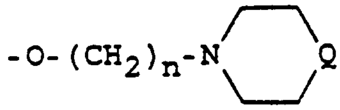

,

32. 5-Tetrazolyl-NH-CO-,
33. $-CO-NH-NH-SO_2CF_3$,
34.

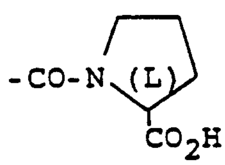

,

35.

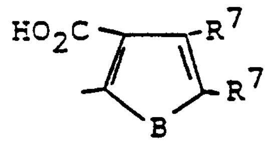

,

36.

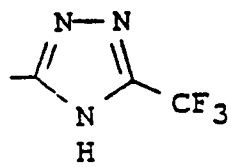

,

37.

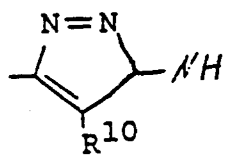

,

38.

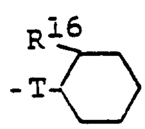

,

39.

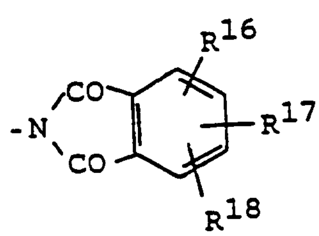

,

40. $-CO-NH-SO_2-R^{19}$

41. den unter q) 4. definierten Rest, substituiert mit 1 oder 2 gleichen oder verschiedenen Resten aus der Reihe Halogen, Cyano, Nitro, $NR^6R^7$ und Hydroxy bedeutet;

r) B O, $NR^7$ oder S bedeutet;

s) W O oder S bedeutet;

t) L $(C_1-C_3)$-Alkandiyl bedeutet;

u) $R^{16}$ $CO_2R^3$ oder $CH_2CO_2R^3$ bedeutet;

v) $R^{17}$ Wasserstoff, Halogen, $(C_1-C_4)$-Alkyl oder $(C_1-C_4)$-Alkoxy bedeutet;

w) $R^{18}$ Wasserstoff, $(C_1-C_4)$-Alkyl oder Phenyl bedeutet;

x) $R^{19}$

1. $(C_1-C_8)$-Alkyl
2. $(C_3-C_8)$-Cycloalkyl
3. Phenyl

4. Benzyl oder
5. den unter x) 1. definierten Rest, worin 1 bis alle H-Atome durch Fluor oder Chlor substituiert sind,
bedeutet;

y) T
1. eine Einfachbindung,
2. -CO-
3. $-CH_2-$
4. -O-
5. -S-
6. $-NR^{21}-$
7. $-CO-NR^{21}-$
8. $-NR^{21}-CO-$
9. $-O-CH_2-$
10. $-CH_2-O-$
11. $-S-CH_2-$
12. $-CH_2-S-$
13. $-NH-CR^{20}R^{22}-$
14. $-NR^{21}-SO_2-$
15. $SO_2-NR^{21}-$
16. $-CR^{20}R^{22}-NH-$
17. -CH = CH-
18. -CF = CF-,
19. -CH = CF-,
20. -CF = CH-,
21. $-CH_2-CH_2-$,
22. $-CF_2-CF_2-$,
23. $-CH(OR^3)-$
24. $-CH(OCOR^5)-$
25.

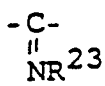

oder
26.

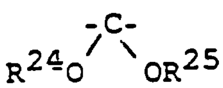

bedeutet;

z) $R^{20}$ und $R^{22}$ gleich oder verschieden sind und Wasserstoff, $(C_1-C_5)$-Alkyl, Phenyl, Allyl oder Benzyl bedeutet;

a') $R^{21}$ Wasserstoff, $(C_1-C_6)$Alkyl, Benzyl oder Allyl bedeutet;

b') $R^{23}$
1. $NR^{20}R^{21}$
2. Ureido,
3. Thioureido,
4. Toluol-4-sulfonyl oder
5. Benzolsulfonylamino

c') $R^{24}$ und $R^{25}$ gleich oder verschieden sind und $(C_1-C_4)$-Alkyl bedeuten oder gemeinsam für $-(CH_2)_q$-stehen;

d') Q $CH_2$, NH, O oder S bedeutet;

e') m eine ganze Zahl von 0 bis 5 ist,

f') n eine ganze Zahl von 1 bis 5 ist;

g') o eine ganze Zahl von 1 bis 10 ist;
h') q 0 oder 1 ist;
i') r 0, 1 oder 2 ist, oder
j') v eine ganze Zahl von 1 bis 6 ist;
dadurch gekennzeichnet, daß man eine Verbindung der Formel II

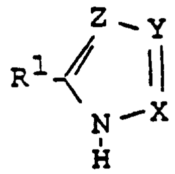

(II)

worin $R^1$, X, Y und Z wie oben definiert sind, umsetzt mit einer Verbindung der Formel III

U-L-$(O)_q$-A (III)

worin L, A und q wie oben definiert sind und U für eine Fluchtgruppe steht, gegebenenfalls temporär eingeführte Schutzgruppen wieder abspaltet und die erhaltenen Verbindungen der Formel I gegebenenfalls in ihre physiologisch verträglichen Salze überführt.

**2.** Verfahren gemäß Anspruch 1, wobei in Formel I bedeuten:
a) $R^1$
1. $(C_3\text{-}C_{10})$-Alkyl,
2. $(C_3\text{-}C_{10})$-Alkenyl,
3. $(C_3\text{-}C_{10})$-Alkinyl,
4. $(C_3\text{-}C_8)$-Cycloalkyl,
5. Benzyl oder
6. Benzyl, das wie im Anspruch 1 definiert substituiert ist,
bedeutet;
b) $R^2$
1. Wasserstoff,
2. Halogen,
3. Nitro,
4. $C_v F_{2v+1}$
5. Pentafluorphenyl
6. Cyano
7. Phenyl,
8. Phenyl-$(C_1\text{-}C_3)$-alkyl,
9. $(C_1\text{-}C_{10})$-Alkyl,
10. $(C_3\text{-}C_{10})$-Alkenyl,
11. Phenyl-$(C_2\text{-}C_6)$-alkenyl,
12. 1-Imidazolyl-$(CH_2)_m$-
13. 1,2,3-Triazolyl-$(CH_2)_O$-
14. Tetrazolyl-$(CH_2)_m$-
15. $-(CH_2)_{o-1}$-$CHR^7$-$OR^5$
16. $-(CH_2)_o$-O-$COR^3$,
17. -$COR^8$,
18. $-(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. $-(CH_2)_o$-NH-CO-$OR^9$,
23. $-(CH_2)_o$-NH-$SO_2$-$R^9$,
24. $-(CH_2)_nF$,
25. $-(CH_2)_o$-$SO_3R^9$,
26. $-(CH_2)_n$-$SO_2$-NH-CO-$NR^6R^9$ oder

27. einem wie unter b) 7., 8., 9., 10. oder 13. definierten Rest, der wie in Anspruch 1 unter c) 46., 45. oder 44. jeweils wie für einen solchen Rest beschrieben substituiert ist,
bedeutet;

c) $R^8$ Wasserstoff; ($C_1$-$C_5$)-Alkyl, $OR^5$ oder $NR^{11}R^{12}$ oder Morpholino bedeutet;

d) T

1. eine Einfachbindung,
2. -CO-
3. $-CONR^{21}-$
4. $-CH_2-CH_2-$
5. $-NR^{21}-CO-$
6. $-O-CH_2-$
7. $-CH_2-O-$
8. $-S-CH_2-$
9. $-CH_2-S-$
10. $-NH-CH_2-$
11. $-CH_2-NH-$ oder
12. -CH=CH-bedeutet und die übrigen Reste und Variablen wie im Anspruch 1 definiert sind, sowie deren physiologisch verträglichen Salze.

**3.** Verfahren gemäß Anspruch 1 oder 2, wobei die Symbole in Formel (I) bedeuten:

a) $R^1$ ($C_3$-$C_7$)-Alkyl, ($C_3$-$C_7$)-Alkenyl oder ($C_3$-$C_7$)-Alkinyl bedeutet;

b) $R^2$

1. Chlor,
2. Brom,
3. $C_v F_{2v+1}$ mit v = 1,2 oder 3,
4. Pentafluorphenyl,
5. $-S(O)_rR^6$,
6. $(CH_2)_{o-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. Tetrazolyl-$(CH_2)_m$-
14. $-(CH_2)_n\ SO_2-NH-CO-NR^6R^9$,
15. $-(CH_2)_o-SO_3R^9$ oder gegebenenfalls durch Hydroxy substituiertes ($C_1$-$C_6$)-Alkyl bedeutet;

c) $R^3$ Wasserstoff oder ($C_1$-$C_4$)-Alkyl bedeutet;

d) $R^6$ Wasserstoff, ($C_1$-$C_4$)-Alkyl, ($C_1$-$C_4$)-Alkanoyl, einen wie oben im Anspruch 1 unter g) 4., 6. oder 9. definierten Rest, substituiert mit 1 oder 2 gleichen oder verschiedenen Resten aus der Reihe Halogen, Hydroxy, Methoxy, Nitro, Cyano, $CO_2R^3$ und Trifluormethyl, oder ($C_1$-$C_9$)-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, bedeutet;

e) $R^7$ Wasserstoff, ($C_1$-$C_4$)-Alkyl, ($C_1$-$C_9$)-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können, oder ($C_6$-$C_{12}$)-Aryl-($C_1$-$C_4$)-Alkyl bedeutet;

f) $R^8$ Wasserstoff, ($C_1$-$C_4$)-Alkyl, $OR^5$ oder Morpholino bedeutet;

g) $R^9$ $CF_3$, ($C_1$-$C_6$)-Alkyl oder Phenyl bedeutet;

h) $R^{14}$

1. ($C_1$-$C_4$)-Alkyl,
2. ($C_1$-$C_4$)-Alkoxy,
3. Cyano,
4. Amino,
5. Nitroso,

6. Nitro,
7. Fluor,
8. Chlor,
9. Brom,
10. Hydroxy,
11. $CH_2OR^7$,
12. ($C_1$-$C_9$)-Heteroaryl-$CH_2$-, wobei der Heteroarylteil sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
13. ($C_1$-$C_4$)-Alkanoyloxy,
14. ($C_1$-$C_4$)Alkanoyl,
15. Benzoyl,
16.

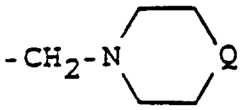

,

17. -NH-CO-$R^7$ oder
18. Tetrazolyl bedeutet;

i) $R^{15}$

1. ($C_1$-$C_4$)-Alkyl,
2. ($C_6$-$C_{12}$)-Aryl,
3. ($C_1$-$C_3$)-Alkanoyloxy,
4. ($C_1$-$C_4$)-Alkoxy,
5. ($C_1$-$C_9$)-Heteroaryl, das sich von Phenyl oder Naphthyl ableitet, in welchem eine oder mehrere CH-Gruppen durch N ersetzt sind und/oder in welchem mindestens zwei benachbarte CH-Gruppen (unter Bildung eines fünfgliedrigen aromatischen Rings) durch S, NH oder O ersetzt sind, wobei auch 1 oder beide Atome der Kondensationsstelle bicyclischer Reste (wie im Indolizinyl) ein N-Atom sein können,
6. Cyano,
7. Nitro,
8. Hydroxy,
9. -$S(O)_rR^6$
10. -$SO_3R^3$
11. Chlor,
12. Brom,
13. Benzoyl,
14. -$CO_2R^3$,
15. -CO-NH-$R^6$,
16. -$NR^6R^7$,
17. -CO-$R^8$,
18. -$SO_2$-$NR^6R^7$,
19.

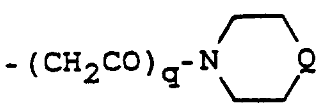

,

20.

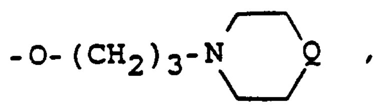

21. $-SO_2-NH-CO-NR^6R^9$,
22. $-PO_3H$
23. $-CO-CHR^5-CO_2H$,
24. $-NH-CO-NH-SO_2-CH_2-R^5$,
25. 5-Tetrazolyl-NH-CO-,
26.

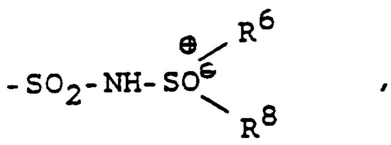

27.

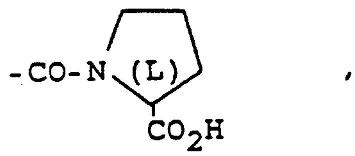

28.

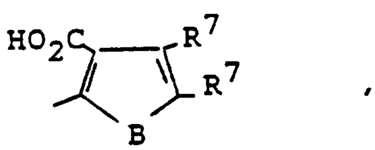

29.

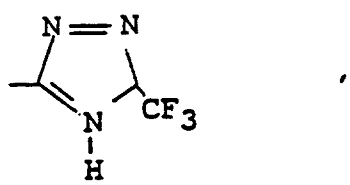

30.

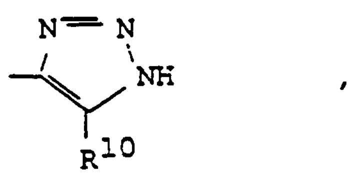

31.

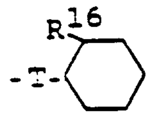

32.

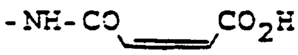

33.

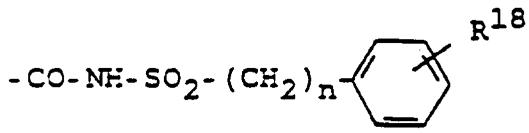

oder

34. den unter i) 2 definierten Rest, substituiert wie in Anspruch 1 definiert, bedeutet;

j) Q $CH_2$, NH oder O bedeutet;
k) $R^{18}$ Wasserstoff, Methyl oder Ethyl bedeutet;
l) T eine Einfachbindung, -O-, -CO-, -NHCO- oder $-OCH_2$-bedeutet

und die übrigen Reste und Variablen wie im Anspruch 1 definiert sind.

**4.** Verfahren gemäß einem der Ansprüche 1 bis 3, wobei die Symbole in Formel (I) bedeuten:
a) X N, Y $CR^2$ und Z $CR^2$ bedeuten;
b) X $CR^2$, Y N und Z $CR^2$ bedeuten;
c) X $CR^2$, Y $CR^2$ und Z N bedeuten
oder
d) X, Y und Z jeweils N bedeuten.

**5.** Verfahren gemäß einem oder mehreren der Ansprüche 1 bis 4, worin
a) $R^2$ Chlor, Brom, $-S(O)_rR^6$, $-COR^8$ oder $-(CH_2)_nSO_2-NH-CO-NR^6R^9$ bedeutet;
b) $R^9$ $(C_1-C_6)$-Alkyl bedeutet;
c) $R^{14}$ Tetrazolyl bedeutet;
d) $R^{15}$ $-CO_2-R^3$, $-SO_2-NR^6R^7$, $-SO_2-NH-CO-NR^6R^9$ oder $-NH-CO-NH-SO_2-CH_2-R^5$ bedeutet;
e) Z gleich N ist;
f) X und Y beide $CR_2$ bedeuten;
g) q Null ist;
h) L $CH_2$ bedeutet;
i) A ein Rest von Benzothiophen, Benzofuran, Indol, Isoindol, Indazol, Benzimidazol, Chinolin, Isochinolin, Phthalazin, Chinoxalin, Chinazolin, Cinnolin, Benzthiazol, Benzothiazol-1,1-dioxid, Cumarin, Chroman, Benzoxazol, Benzisothiazol, Benzodiazine, Benztriazol, Benztriazin, Benzoxazin, Imidazo-pyridin, Imidazo-pyrimidin, Imidazo-pyrazin, Imidazo-pyridazin, Imidazo-thiazol, Pyrazolopyridin, Thienopyridin und Pyrrolopyrimidin bedeutet
der mit bis zu 6 gleichen oder verschiedenen Resten $R^{14}$ oder $-(CH_2)_{n-1}-(CHR^6-CH_2)_{o-1}-R^{15}$ substituiert sein kann,

und die übrigen Reste und Variablen wie im Anspruch 3 definiert sind.

**6.** Verfahren zur Herstellung einer pharmazeutischen Zubereitung dadurch gekennzeichnet; daß man eine Verbindung hergestellt gemäß einem der Ansprüche 1 - 5 zusammen mit einem physiologisch unbedenklichen Träger und gegebenenfalls weiteren Zusatz- oder Hilfsstoffen in eine geeignete

Darreichungsform bringt.

**7.** Verfahren zur Herstellung einer Verbindung der Formel (II')

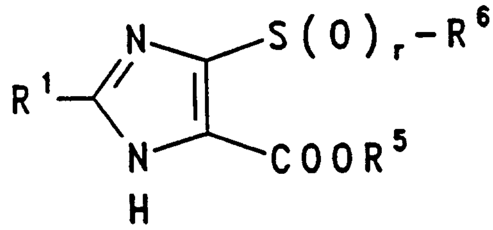

(II')

worin

a) $R^1$ n-Butyl bedeutet;
b) $R^6$ Methyl bedeutet;
c) $R^5$ Wasserstoff oder Ethyl bedeutet und
d) r 0, 1 oder 2 ist;

dadurch gekennzeichnet, daß ausgehend von einer Verbindung der Formel (III)

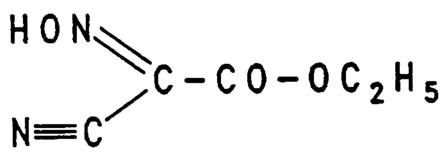

(III)

diese durch anschließende

- Reduktion zum Aminonitril
- Einführung einer Alkylgruppe mittels Acylierung,
- Addition einer Alkylmercaptangruppe an die Cyanogruppe und Ringschluß und gegebenenfalls zusätzlicher Oxidierung der Thioalkylgruppe und/oder Hydrolyse der Estergruppe in eine Verbindung der Formel (II') übergeführt wird.

**8.** Verfahren zur Herstellung einer Verbindung der Formel (II') gemäß Anspruch 7, wobei r gleich 0 ist und $R^5$ Ethyl bedeutet.

**9.** Verbindung der Formel (II')

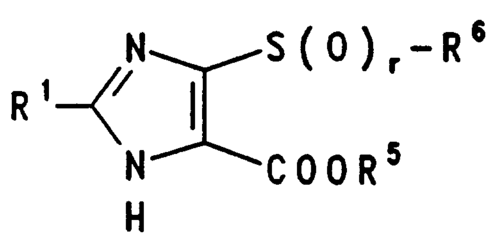

(II')

worin

a) $R^1$ n-Butyl bedeutet;
b) $R^6$ Methyl bedeutet;
c) $R^5$ Wasserstoff oder Ethyl bedeutet und
d) r 0, 1 oder 2 ist;

**10.** Verbindung der Formel (II') gemäß Anspruch 9, wobei r gleich 0 ist und $R^5$ Ethyl bedeutet.

## Claims

**Claims for the following Contracting States : AT, BE, CH, DE, DK, FR, GB, IT, LI, LU, NL, SE**

**1.** A compound of the formula I

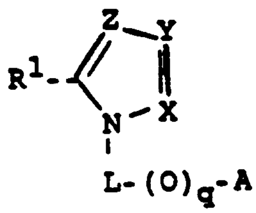

(I)

in which

a) X, Y and Z are identical or different and are N or $CR^2$,

b) $R^1$ is

1. $(C_2\text{-}C_{10})$-alkyl,
2. $(C_3\text{-}C_{10})$-alkenyl,
3. $(C_3\text{-}C_{10})$-alkynyl,
4. $OR^3$,
5. $(C_3\text{-}C_8)$-cycloalkyl,
6. $(C_4\text{-}C_{10})$-cycloalkylalkyl,
7. $(C_5\text{-}C_{10})$-cycloalkylalkenyl,
8. $(C_5\text{-}C_{10})$-cycloalkylalkynyl,
9. $-(CH_2)_m\text{-}B\text{-}(CH_2)_n\text{-}R^4$,
10. benzyl,
11. a radical which is defined as under b) 1., 2., 3. or 9. and is monosubstituted by $CO_2R^3$,
12. a radical which is defined as under b) 1., 2., 3. or 9. and in which 1 to all hydrogen atoms are replaced by fluorine, or
13. the radical which is defined under b) 10. and is substituted on the phenyl by 1 or 2 identical or different radicals from the series comprising halogen, $(C_1\text{-}C_4)$-alkoxy and nitro;

c) $R^2$ is

1. hydrogen,
2. halogen,
3. nitro,
4. $C_vF_{2v+1}$,
5. pentafluorophenyl,
6. cyano,
7. phenyl,
8. phenyl-$(C_1\text{-}C_3)$-alkyl,
9. $(C_1\text{-}C_{10})$-alkyl,
10. $(C_3\text{-}C_{10})$-alkenyl,
11. phenyl-$(C_2\text{-}C_6)$-alkenyl,
12. 1-imidazolyl-$(CH_2)_m$-,
13. 1,2,3-triazolyl-$(CH_2)_n$-,
14. tetrazolyl-$(CH_2)_m$-,
15. $-(CH_2)_{o-1}\text{-}CHR^7\text{-}OR^5$,
16. $-(CH_2)_o\text{-}O\text{-}CO\text{-}R^3$,
17. $-(CH_2)_o\text{-}S\text{-}R^6$,
18. $-S(O)_r\text{-}R^6$,
19. $-CH=CH\text{-}(CH_2)_m\text{-}CHR^3\text{-}OR^6$,
20. $-CH_2=CH\text{-}(CH_2)_m\text{-}CO\text{-}R^8$,
21. $-CO\text{-}R^8$,
22. $-CH=CH\text{-}(CH_2)_m\text{-}O\text{-}CO\text{-}R^7$,
23. $-(CH_2)_m\text{-}CH(CH_3)\text{-}CO\text{-}R^8$,
24. $-(CH_2)_o\text{-}CO\text{-}R^8$,

25.

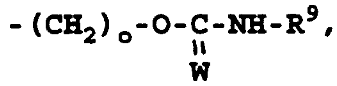

26.

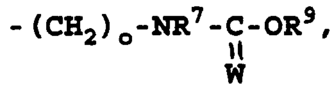

27. $-(CH_2)_o-NR^7-CO-NHR^9$,
28. $-(CH_2)_o-NR^7-SO_2R^9$,
29.

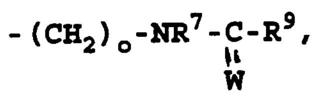

30. $-(CH_2)_nF$,
31. $-(CH_2)_n-O-NO_2$,
32. $-CH_2-N_3$,
33. $-(CH_2)_n-NO_2$,
34. $-CH=N-NR^5R^7$,
35. phthalimido-$(CH_2)_n$-,
36.

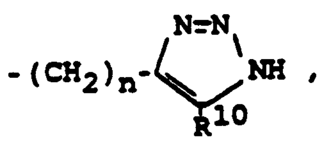

37.

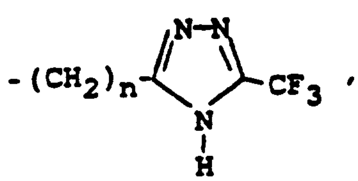

38.

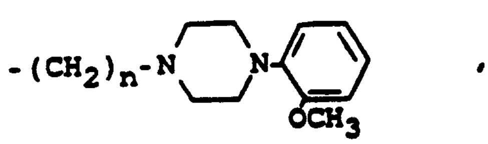

39.

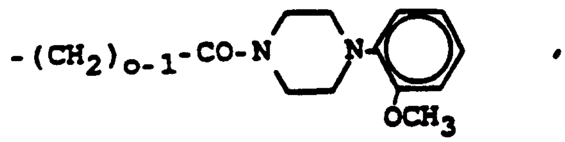

40. phenyl-$SO_2$-NH-N = CH-,

41.

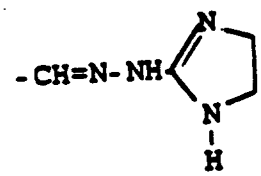

42. -$(CH_2)_n$-$SO_2$-$NR^7$-CO-$NR^6R^9$,

43. -$(CH_2)_o$-$SO_2R^9$,

44. a radical which is defined as under c) 7. or 8. and is substituted on the phenyl by 1 or 2 identical or different radicals from the series comprising halogen, hydroxyl, methoxy, trifluoromethyl, $CO_2R^3$ and phenyl,

45. a radical which is defined as under c) 9., 10. or 18. and in which 1 to all hydrogen atoms are replaced by fluorine, or

46. the radical which is defined under c) 13. and is substituted by 1 or 2 identical or different radicals from the series comprising methoxycarbonyl and $(C_1-C_4)$-alkyl;

d) $R^3$ is

1. hydrogen,
2. $(C_1-C_8)$-alkyl,
3. $(C_3-C_8)$-cycloalkyl,
4. phenyl,
5. benzyl or
6. the radical which is defined under d) 2. and in which 1 to all hydrogen atoms are replaced by fluorine;

e) $R^4$ is

1. hydrogen,
2. $(C_1-C_6)$-alkyl,
3. $(C_3-C_8)$-cycloalkyl,
4. $(C_2-C_4)$-alkenyl or
5. $(C_2-C_4)$-alkynyl;

f) $R^5$ is

1. hydrogen,
2. $(C_1-C_6)$-alkyl,
3. $(C_3-C_8)$-cycloalkyl,
4. phenyl or
5. benzyl;

g) $R^6$ is

1. hydrogen,
2. $(C_1-C_6)$-alkyl,
3. $(C_3-C_8)$-cycloalkyl,
4. $(C_6-C_{12})$-aryl,
5. benzyl,
6. $(C_1-C_9)$-heteroaryl, which can be partially or completely hydrogenated and which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,

7. $(C_1-C_4)$-alkanoyl,

8. a radical which is defined as under g) 4. or 6. and is substituted by 1 or 2 identical or different radicals from the series comprising halogen, hydroxyl, methoxy, nitro, cyano, $CO_2R^3$ and trifluoromethyl, $NR^{11}R^{12}$ or

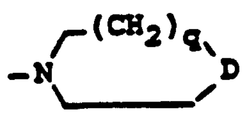

9. $(C_1-C_9)$-heteroaryl-$(C_1-C_3)$-alkyl, where the heteroaryl moiety is defined as under g) 6.;

h) $R^7$ is

1. hydrogen,
2. $(C_1-C_6)$-alkyl,
3. $(C_3-C_8)$-cycloalkyl,
4. $(C_6-C_{12})$-aryl-$(C_1-C_6)$-alkyl,
5. phenyl or
6. $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom;

i) $R^8$ is

1. hydrogen,
2. $(C_1-C_6)$-alkyl,
3. $(C_3-C_8)$-cycloalkyl,
4. phenyl-$(CH_2)_q$-,
5. $OR^5$,
6. $NR^{11}R^{12}$ or
7.

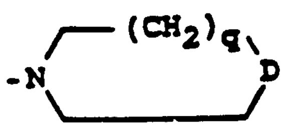

;

j) $R^9$ is

1. $(C_1-C_6)$-alkyl,
2. 1-adamantyl,
3. 1-naphthyl,
4. 1-naphthylethyl,
5. phenyl-$(CH_2)_q$- or
6. the radical which is defined under j) 1. and in which 1 to all hydrogen atoms are replaced by fluorine;

k) $R^{10}$ is cyano, nitro or $CO_2R^7$;

l) $R^{11}$ and $R^{12}$ are identical or different and are

1. hydrogen,
2. $(C_1-C_4)$-alkyl,
3. phenyl,
4. benzyl or
5. $\alpha$-methylbenzyl;

m) D is $NR^{13}$, O or $CH_2$;

n) $R^{13}$ is hydrogen, $(C_1-C_4)$-alkyl or phenyl;

o) A is a fused heterobicyclic radical which has 8 to 10 ring atoms, of which up to 9 ring atoms are carbon atoms, in which two atoms next to one another are common constituents of both rings, where one or both of these rings are formally derived from benzene, in which one or more CH groups are replaced by N, $O^+$ and $S^+$, and/or in which two adjacent CH groups are replaced (to form a five-membered aromatic ring) by S, NH or O, and which can be substituted by up to 6 identical or

different radicals $R^{14}$ or $-(CH_2)_{n-1}-(CHR^6-CH_2)_{o-1}-R^{15}$;

p) $R^{14}$ is

1. halogen,
2. oxo,
3. nitroso,
4. nitro,
5. amino,
6. cyano,
7. hydroxyl,
8. $(C_1-C_6)$-alkyl,
9. $(C_1-C_4)$-alkanoyl,
10. $(C_1-C_4)$-alkanoyloxy,
11. $CO_2R^3$,
12. methanesulfonylamino,
13. trifluoromethanesulfonylamino,
14. $-CO-NH-OR^9$,
15. $-SO_2-NR^6R^7$,
16. $-CH_2OR^7$,
17. $(C_1-C_9)$-heteroaryl-$(CH_2)_q$-, where the heteroaryl moiety is defined as under h) 6.,
18. $(C_7-C_{13})$-aroyl,
19.

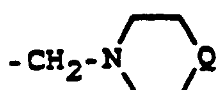

20.

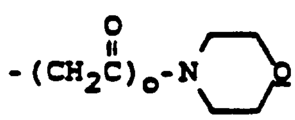

or

21. $(C_6-C_{12})$-aryl;

q) $R^{15}$ is

1. hydrogen,
2. $(C_1-C_6)$-alkyl,
3. $(C_3-C_8)$-cycloalkyl,
4. $(C_6-C_{12})$-aryl,
5. $(C_7-C_{13})$-aroyl,
6. $(C_1-C_4)$-alkoxy,
7. $(C_1-C_4)$-alkanoyloxy,
8. $(C_1-C_9)$-heteroaryl which is defined as under h) 6.,
9. $CO_2R^3$,
10. halogen,
11. cyano,
12. nitro,
13. $NR^6R^7$,
14. hydroxyl,
15. $-CO-NH-CHR^5-CO_2R^3$,
16. sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. phosphonooxy,

22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. the radical defined under p) 20.,
29.

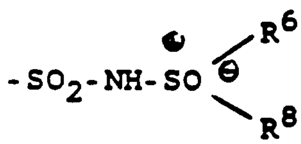
,

30.

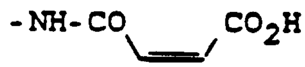
,

31.

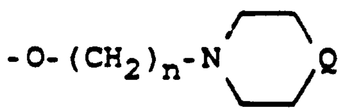
,

32. 5-tetrazolyl-NH-CO-,
33. $-CO-NH-NH-SO_2CF_3$,
34.

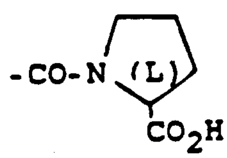
,

35.

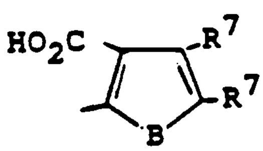
,

36.

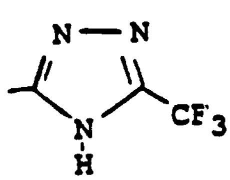
,

37.

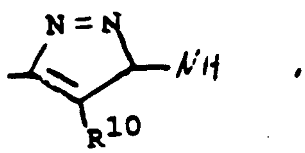

38.

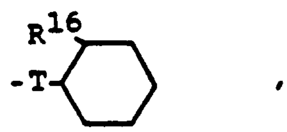

39.

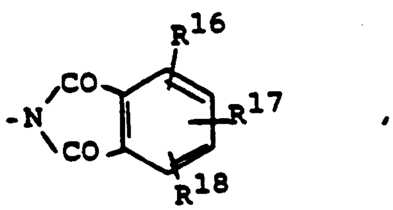

40. $-CO-NH-SO_2-R^{19}$,

41. the radical which is defined under q) 4. and is substituted by 1 or 2 identical or different radicals from the series comprising halogen, cyano, nitro, $NR^6R^7$ and hydroxyl;

r) B is O, $NR^7$ or S;

s) W is O or S;

t) L is $(C_1-C_3)$-alkanediyl;

u) $R^{16}$ is $CO_2R^3$ or $CH_2CO_2R^3$;

v) $R^{17}$ is hydrogen, halogen, $(C_1-C_4)$-alkyl or $(C_1-C_4)$-alkoxy;

w) $R^{18}$ is hydrogen, $(C_1-C_4)$-alkyl or phenyl;

x) $R^{19}$ is

1. $(C_1-C_8)$-alkyl,
2. $(C_3-C_8)$-cycloalkyl,
3. phenyl,
4. benzyl or
5. the radical which is defined under x) 1. and in which 1 to all hydrogen atoms are replaced by fluorine or chlorine;

y) T is

1. a single bond,
2. -CO-,
3. $-CH_2-$,
4. -O-,
5. -S-,
6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,

15. $-SO_2-NR^{21}-$,
16. $-CR^{20}R^{22}-NH-$,
17. -CH=CH-,
18. -CF=CF-,
19. -CH=CF-,
20. -CF=CH-,
21. $-CH_2-CH_2-$,
22. $-CF_2-CF_2-$,
23. $-CH(OR^3)-$,
24. $-CH(OCOR^5)-$,
25.

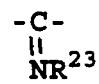

or
26.

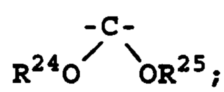

z) $R^{20}$ and $R^{22}$ are identical or different and are hydrogen, $(C_1-C_5)$-alkyl, phenyl, allyl or benzyl;
a') $R^{21}$ is hydrogen, $(C_1-C_6)$-alkyl, benzyl or allyl;
b') $R^{23}$ is
1. $NR^{20}R^{21}$,
2. ureido,
3. thioureido,
4. toluene-4-sulfonyl or
5. benzenesulfonylamino
c') $R^{24}$ and $R^{25}$ are identical or different and are $(C_1-C_4)$-alkyl or together are $-(CH_2)_q-$;
d') Q is $CH_2$, NH, O or S;
e') m is an integer from 0 to 5;
f') n is an integer from 1 to 5;
g') o is an integer from 1 to 10;
h') q is 0 or 1;
i') r is 0, 1 or 2, or
j') v is an integer from 1 to 6;
and the physiologically tolerated salts thereof.

**2.** A compound of the formula I as claimed in claim 1, in which
a) $R^1$ is
1. $(C_3-C_{10})$-alkyl,
2. $(C_3-C_{10})$-alkenyl,
3. $(C_3-C_{10})$-alkynyl,
4. $(C_3-C_8)$-cycloalkyl,
5. benzyl or
6. benzyl which is substituted as defined in claim 1;
b) $R^2$ is
1. hydrogen,
2. halogen,
3. nitro,
4. $C_vF_{2v+1}$,
5. pentafluorophenyl,
6. cyano,
7. phenyl,

8. phenyl-($C_1$-$C_3$)-alkyl,
9. ($C_1$-$C_{10}$)-alkyl,
10. ($C_3$-$C_{10}$)-alkenyl,
11. phenyl-($C_2$-$C_6$)-alkenyl,
12. 1-imidazolyl-$(CH_2)_m$-,
13. 1,2,3-triazolyl-$(CH_2)_o$-,
14. tetrazolyl-$(CH_2)_m$-,
15. -$(CH_2)_{o-1}$-$CHR^7$-$OR^5$,
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ or
27. a radical which is defined as under b) 7., 8., 9., 10. or 13. and which is substituted as defined in claim 1 under c) 46., 45. or 44. in each case as described for a radical of this type;

c) $R^8$ is hydrogen; ($C_1$-$C_5$)-alkyl, $OR^5$ or $NR^{11}R^{12}$ or morpholino;

d) T is
1. a single bond,
2. -CO-,
3. -$CONR^{21}$-,
4. -$CH_2$-$CH_2$-,
5. -$NR^{21}$-CO-,
6. -O-$CH_2$-,
7. -$CH_2$-O-,
8. -S-$CH_2$-,
9. -$CH_2$-S-,
10. -NH-$CH_2$-,
11. -$CH_2$-NH- or
12. -CH = CH-and the other radicals and variables are defined as in claim 1, and the physiologically tolerated salts thereof.

**3.** A compound of the formula I as claimed in claim 1 or 2, in which

a) $R^1$ is ($C_3$-$C_7$)-alkyl, ($C_3$-$C_7$)-alkenyl or ($C_3$-$C_7$)-alkynyl;

b) $R^2$ is
1. chlorine,
2. bromine,
3. $C_vF_{2v+1}$ with v = 1, 2 or 3,
4. pentafluorophenyl,
5. -$S(O)_rR^6$,
6. $(CH_2)_{o-1}$-$CHR^7$-$OR^5$,
7. $(CH_2)_o$-O-CO-$R^3$,
8. -$COR^8$,
9. -$(CH_2)_o$-CO-$R^8$,
10. -$CH_2$-NH-CO-$R^8$,
11. -$(CH_2)_o$-NH-$SO_2$-$R^9$,
12. -CH = CH-$CHR^3$-$OR^6$,
13. tetrazolyl-$(CH_2)_m$-,
14. -$(CH_2)_nSO_2$-NH-CO-$NR^6R^9$,
15. -$(CH_2)_o$-$SO_3R^9$ or optionally hydroxyl-substituted ($C_1$-$C_6$)-alkyl;

c) $R^3$ is hydrogen or ($C_1$-$C_4$)-alkyl;

d) $R^6$ is hydrogen, ($C_1$-$C_4$)-alkyl, ($C_1$-$C_4$)-alkanoyl, a radical which is defined as above in claim 1 under g) 4., 6. or 9. and is substituted by 1 or 2 identical or different radicals from the series

comprising halogen, hydroxyl, methoxy, nitro, cyano, $CO_2R^3$ and trifluoromethyl, or ($C_1$-$C_9$)-heteroaryl which is derived from phenyl or naphthyl, in which one or more CH groups are replaced by N and/or in which at least two adjacent CH groups are replaced (to form a five-membered aromatic ring) by S, NH or O where 1 or both atoms at the fusion point of bicyclic radicals (such as in indolizinyl) can also be an N atom;

e) $R^7$ is hydrogen, ($C_1$-$C_4$)-alkyl, ($C_1$-$C_9$)-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom, or ($C_6$-$C_{12}$)-aryl-($C_1$-$C_4$)-alkyl;

f) $R^8$ is hydrogen, ($C_1$-$C_4$)-alkyl, $OR^5$ or morpholino;

g) $R^9$ is $CF_3$, ($C_1$-$C_6$)-alkyl or phenyl;

h) $R^{14}$ is

1. ($C_1$-$C_4$)-alkyl,
2. ($C_1$-$C_4$) -alkoxy,
3. cyano,
4. amino,
5. nitroso,
6. nitro,
7. fluorine,
8. chlorine,
9. bromine,
10. hydroxyl,
11. $CH_2OR^7$,
12. ($C_1$-$C_9$)-heteroaryl-$CH_2$-, where the heteroaryl moiety is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
13. ($C_1$-$C_4$)-alkanoyloxy,
14. ($C_1$-$C_4$)-alkanoyl,
15. benzoyl,
16.

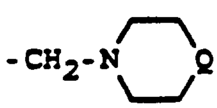

17. -NH-CO-$R^7$ or
18. tetrazolyl;

i) $R^{15}$ is

1. ($C_1$-$C_4$)-alkyl,
2. ($C_6$-$C_{12}$)-aryl,
3. ($C_1$-$C_3$)-alkanoyloxy,
4. ($C_1$-$C_4$)-alkoxy,
5. ($C_1$-$C_9$)-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
6. cyano,
7. nitro,
8. hydroxyl,
9. -$S(O)_rR^6$,
10. -$SO_3R^3$,
11. chlorine,
12. bromine,
13. benzoyl,
14. -$CO_2R^3$,

15. $-CO-NH-R^6$,
16. $-NR^6R^7$,
17. $-CO-R^8$,
18. $-SO_2-NR^6R^7$,
19.

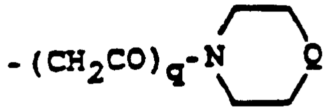

,

20.

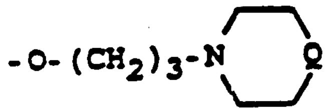

,

21. $-SO_2-NH-CO-NR^6R^9$,
22. $-PO_3H$,
23. $-CO-CHR^5-CO_2H$,
24. $-NH-CO-NH-SO_2-CH_2-R^5$,
25. 5-tetrazolyl-NH-CO-,
26.

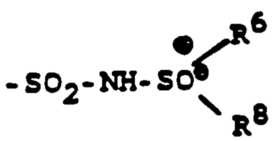

27.

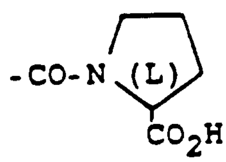

,

28.

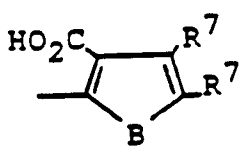

,

29.

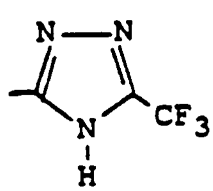

,

30.

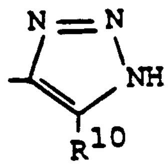,

31.

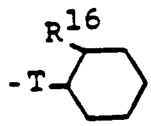,

32.

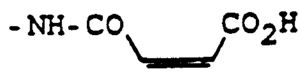,

33.

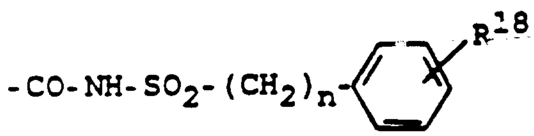

or

34. the radical which is defined under i) 2. and is substituted as defined in claim 1;

j) Q is $CH_2$, NH or O;

k) $R^{18}$ is hydrogen, methyl or ethyl;

l) T is a single bond, -O-, -CO-, -NHCO- or $-OCH_2-$

and the other radicals and variables are defined as in claim 1, and the physiologically tolerated salts thereof.

**4.** A compound as claimed in any of claims 1 to 3, in which

a) X is N, Y is $CR^2$ and Z is $CR^2$;

b) X is $CR^2$, Y is N and Z is $CR^2$;

c) X is $CR^2$, Y is $CR^2$ and Z is N

or

d) X, Y and Z are each N, and the physiologically tolerated salts thereof.

**5.** A compound as claimed in one or more of claims 1 to 4, in which

a) $R^2$ is chlorine, bromine, $-S(O)_rR^6$, $-COR^8$ or $-(CH_2)_nSO_2-NH-CO-NR^6R^9$;

b) $R^9$ is $(C_1-C_6)$-alkyl;

c) $R^{14}$ is tetrazolyl;

d) $R^{15}$ is $-CO_2-R^3$, $-SO_2-NR^6R^7$, $-SO_2-NH-CO-NR^6R^9$ or $-NH-CO-NH-SO_2-CH_2-R^5$;

e) Z is N;

f) X and Y are both $CR_2$;

g) q is zero;

h) L is $CH_2$;

i) A is a radical of benzothiophene, benzofuran, indole, isoindole, indazole, benzimidazole, quinoline, isoquinoline, phthalazine, quinoxaline, quinazoline, cinnoline, benzothiazole, benzothiazole 1,1-dioxide, coumarin, chroman, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thienopyridine and pyrrolopyrimidine, which can be substituted by up to 6 identical or different radicals $R^{14}$ or - $(CH_2)_{n-1}$-$(CHR^6$-$CH_2)_{0-1}$-$R^{15}$, and the other radicals and variables are defined as in claim 3, and the physiologically tolerated salts thereof.

**6.** A process for preparing a compound of the formula I as claimed in any of claims 1 to 4, which comprises reacting a compound of the formula II

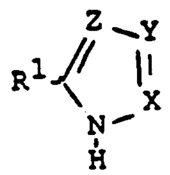

(II)

in which $R^1$, X, Y and Z are defined as in claim 1, with a compound of the formula III

U-L-$(O)_q$-A (III)

in which L, A and q are defined as in claim 1, and U is a leaving group, where appropriate eliminating again protective groups which have been temporarily introduced, and converting the resulting compounds of the formula I, where appropriate, into the physiologically tolerated salts thereof.

**7.** A compound as claimed in any of claims 1-5 for use as medicine.

**8.** A compound as claimed in any of claims 1-5 for use as medicine for the treatment of high blood pressure.

**9.** A pharmaceutical preparation containing a compound as claimed in any of claims 1-5.

**10.** A process for preparing a preparation as claimed in claim 9, which comprises converting a compound as claimed in any of claims 1-5 together with a physiologically acceptable vehicle and, where appropriate, other additives or auxiliaries into a suitable dosage form.

**11.** A compound of the formula

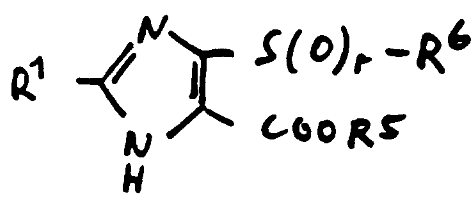

in which
a) $R^1$ is n-butyl;
b) $R^6$ is methyl;
c) $R^5$ is hydrogen or ethyl, and
d) r is 0, 1 or 2.

**12.** A compound of the formula as claimed in claim 11 where r is 0, and $R^5$ is ethyl.

**Claims for the following Contracting State : ES**

**1.** A process for preparing a compound of the formula I

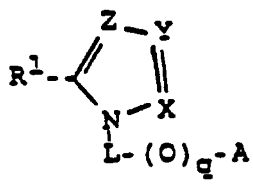

(I)

in which

a) X, Y and Z are identical or different and are N or $CR^2$,

b) $R^1$ is

1. $(C_2-C_{10})$-alkyl,
2. $(C_3-C_{10})$-alkenyl,
3. $(C_3-C_{10})$-alkynyl,
4. $OR^3$,
5. $(C_3-C_8)$-cycloalkyl,
6. $(C_4-C_{10})$-cycloalkylalkyl,
7. $(C_5-C_{10})$-cycloalkylalkenyl,
8. $(C_5-C_{10})$-cycloalkylalkynyl,
9. $-(CH_2)_m-B-(CH_2)_n-R^4$,
10. benzyl,
11. a radical which is defined as under b) 1., 2., 3. or 9. and is monosubstituted by $CO_2R^3$,
12. a radical which is defined as under b) 1., 2., 3. or 9. and in which 1 to all hydrogen atoms are replaced by fluorine, or
13. the radical which is defined under b) 10. and is substituted on the phenyl by 1 or 2 identical or different radicals from the series comprising halogen, $(C_1-C_4)$-alkoxy and nitro;

c) $R^2$ is

1. hydrogen,
2. halogen,
3. nitro,
4. $C_vF_{2v+1}$,
5. pentafluorophenyl,
6. cyano,
7. phenyl,
8. phenyl-$(C_1-C_3)$-alkyl,
9. $(C_1-C_{10})$-alkyl,
10. $(C_3-C_{10})$-alkenyl,
11. phenyl-$(C_2-C_6)$-alkenyl,
12. 1-imidazolyl-$(CH_2)_m$-,
13. 1,2,3-triazolyl-$(CH_2)_n$-,
14. tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,

25.

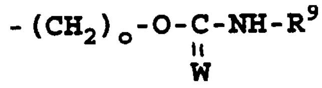

26.

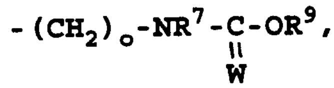

27. $-(CH_2)_o-NR^7-CO-NHR^9$,

28. $-(CH_2)_o-NR^7-SO_2R^9$,

29.

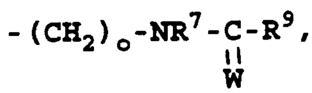

30. $-(CH_2)_nF$,

31. $-(CH_2)_n-O-NO_2$,

32. $-CH_2-N_3$,

33. $-(CH_2)_n-NO_2$,

34. $-CH=N-NR^5R^7$,

35. phthalimido-$(CH_2)_n$-,

36.

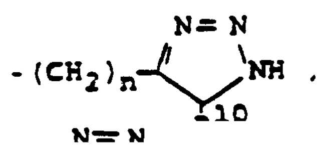

37.

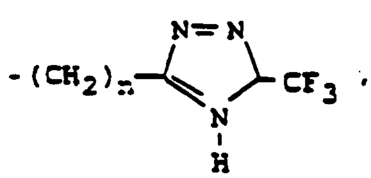

38.

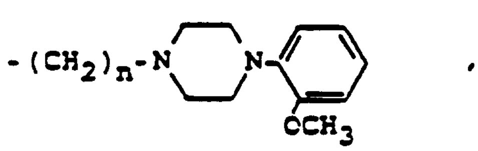

39.

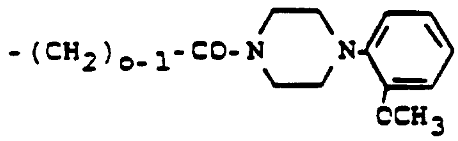,

40. phenyl-$SO_2$-NH-N = CH-,
41.

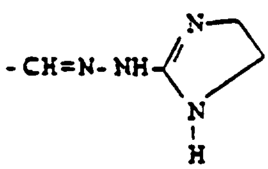,

42. -$(CH_2)_n$-$SO_2$-$NR^7$-CO-$NR^6R^9$,
43. -$(CH_2)_o$-$SO_2R^9$,
44. a radical which is defined as under c) 7. or 8. and is substituted on the phenyl by 1 or 2 identical or different radicals from the series comprising halogen, hydroxyl, methoxy, trifluoromethyl, $CO_2R^3$ and phenyl,
45. a radical which is defined as under c) 9., 10. or 18. and in which 1 to all hydrogen atoms are replaced by fluorine, or
46. the radical which is defined under c) 13. and is substituted by 1 or 2 identical or different radicals from the series comprising methoxycarbonyl and ($C_1$-$C_4$)-alkyl;

d) $R^3$ is
1. hydrogen,
2. ($C_1$-$C_8$)-alkyl,
3. ($C_3$-$C_8$)-cycloalkyl,
4. phenyl,
5. benzyl or
6. the radical which is defined under d) 2. and in which 1 to all hydrogen atoms are replaced by fluorine;

e) $R^4$ is
1. hydrogen,
2. ($C_1$-$C_6$)-alkyl,
3. ($C_3$-$C_8$)-cycloalkyl,
4. ($C_2$-$C_4$)-alkenyl or
5. ($C_2$-$C_4$)-alkynyl;

f) $R^5$ is
1. hydrogen,
2. ($C_1$-$C_6$)-alkyl,
3. ($C_3$-$C_8$)-cycloalkyl,
4. phenyl or
5. benzyl;

g) $R^6$ is
1. hydrogen,
2. ($C_1$-$C_6$)-alkyl,
3. ($C_3$-$C_8$)-cycloalkyl,
4. ($C_6$-$C_{12}$)-aryl,
5. benzyl,
6. ($C_1$-$C_9$)-heteroaryl which can be partially or completely hydrogenated and which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also

be an N atom,
7. $(C_1-C_4)$-alkanoyl,
8. a radical which is defined as under g) 4. or 6. and is substituted by 1 or 2 identical or different radicals from the series comprising halogen, hydroxyl, methoxy, nitro, cyano, $CO_2R^3$ and trifluoromethyl, $NR^{11}R^{12}$ or

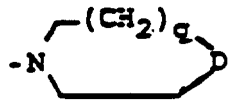

9. $(C_1-C_9)$-heteroaryl-$(C_1-C_3)$-alkyl, where the heteroaryl moiety is defined as under g) 6.;

h) $R^7$ is
1. hydrogen,
2. $(C_1-C_6)$-alkyl,
3. $(C_3-C_8)$-cycloalkyl,
4. $(C_6-C_{12})$-aryl-$(C_1-C_6)$-alkyl,
5. phenyl or
6. $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom;

i) $R^8$ is
1. hydrogen,
2. $(C_1-C_6)$-alkyl,
3. $(C_3-C_8)$-cycloalkyl,
4. phenyl-$(CH_2)_q$-,
5. $OR^5$,
6. $NR^{11}R^{12}$ or
7.

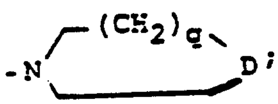

;

j) $R^9$ is
1. $(C_1-C_6)$-alkyl,
2. 1-adamantyl,
3. 1-naphthyl,
4. 1-naphthylethyl,
5. phenyl-$(CH_2)_q$- or
6. the radical which is defined under j) 1. and in which 1 to all hydrogen atoms are replaced by fluorine;

k) $R^{10}$ is cyano, nitro or $CO_2R^7$;

l) $R^{11}$ and $R^{12}$ are identical or different and are
1. hydrogen,
2. $(C_1-C_4)$-alkyl,
3. phenyl,
4. benzyl or
5. $\alpha$-methylbenzyl;

m) D is $NR^{13}$, O or $CH_2$;

n) $R^{13}$ is hydrogen, $(C_1-C_4)$-alkyl or phenyl;

o) A is a fused heterobicyclic radical which has 8 to 10 ring atoms, of which up to 9 ring atoms are carbon atoms, in which two atoms next to one another are common constituents of both rings, where one or both of these rings are formally derived from benzene, in which one or more CH groups are replaced by N, $O^+$ and $S^+$, and/or in which two adjacent CH groups are replaced (to form a five-membered aromatic ring) by S, NH or O, and which can be substituted by up to 6 identical or

different radicals $R^{14}$ or $-(CH_2)_{n-1}-(CHR^6-CH_2)_{o-1}-R^{15}$;

p) $R^{14}$ is

1. halogen,
2. oxo,
3. nitroso,
4. nitro,
5. amino,
6. cyano,
7. hydroxyl,
8. $(C_1-C_6)$-alkyl,
9. $(C_1-C_4)$-alkanoyl,
10. $(C_1-C_4)$-alkanoyloxy,
11. $CO_2R^3$,
12. methanesulfonylamino,
13. trifluoromethanesulfonylamino,
14. $-CO-NH-OR^9$,
15. $-SO_2-NR^6R^7$,
16. $-CH_2OR^7$,
17. $(C_1-C_9)$-heteroaryl-$(CH_2)_q$-, where the heteroaryl moiety is defined as under g) 6.,
18. $(C_7-C_{13})$-aroyl,
19.

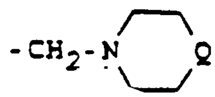

,

20.

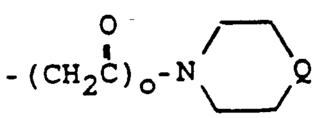

or

21. $(C_6-C_{12})$-aryl;

q) $R^{15}$ is

1. hydrogen,
2. $(C_1-C_6)$-alkyl,
3. $(C_3-C_8)$-cycloalkyl,
4. $(C_6-C_{12})$-aryl,
5. $(C_7-C_{13})$-aroyl,
6. $(C_1-C_4)$-alkoxy,
7. $(C_1-C_4)$-alkanoyloxy,
8. $(C_1-C_9)$-heteroaryl which is defined as under h) 6.,
9. $CO_2R^3$,
10. halogen,
11. cyano,
12. nitro,
13. $NR^6R^7$,
14. hydroxyl,
15. $-CO-NH-CHR^5-CO_2R^3$,
16. sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. phosphonooxy,

22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. the radical defined under p) 20.,
29.

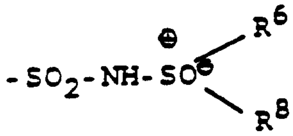
.

30.

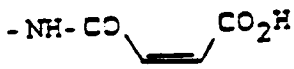
,

31.

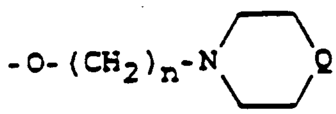
,

32. 5-tetrazolyl-NH-CO-,
33. $-CO-NH-NH-SO_2CF_3$,
34.

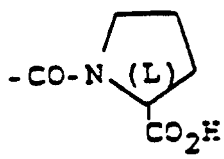
,

35.

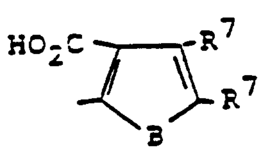
,

36.

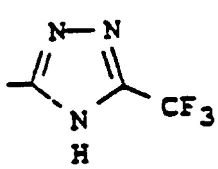
,

37.

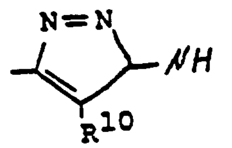

,

38.

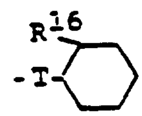

,

39.

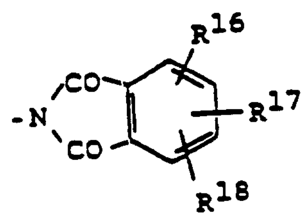

,

40. $-CO-NH-SO_2-R^{19}$,

41. the radical which is defined under q) 4. and is substituted by 1 or 2 identical or different radicals from the series comprising halogen, cyano, nitro, $NR^6R^7$ and hydroxyl;

r) B is O, $NR^7$ or S;

s) W is O or S;

t) L is $(C_1-C_3)$-alkanediyl;

u) $R^{16}$ is $CO_2R^3$ or $CH_2CO_2R^3$;

v) $R^{17}$ is hydrogen, halogen, $(C_1-C_4)$-alkyl or $(C_1-C_4)$-alkoxy;

w) $R^{18}$ is hydrogen, $(C_1-C_4)$-alkyl or phenyl;

x) $R^{19}$ is

1. $(C_1-C_8)$-alkyl,
2. $(C_3-C_8)$-cycloalkyl,
3. phenyl,
4. benzyl or
5. the radical which is defined under x) 1. and in which 1 to all hydrogen atoms are replaced by fluorine or chlorine;

y) T is

1. a single bond,
2. -CO-,
3. $-CH_2-$,
4. -O-,
5. -S-,
6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,

15. $-SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

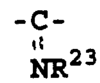

or
26.

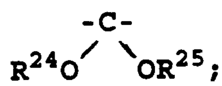

z) $R^{20}$ and $R^{22}$ are identical or different and are hydrogen, $(C_1-C_5)$-alkyl, phenyl, allyl or benzyl;
a') $R^{21}$ is hydrogen, $(C_1-C_6)$-alkyl, benzyl or allyl;
b') $R^{23}$ is
1. $NR^{20}R^{21}$,
2. ureido,
3. thioureido,
4. toluene-4-sulfonyl or
5. benzenesulfonylamino
c') $R^{24}$ and $R^{25}$ are identical or different and are $(C_1-C_4)$-alkyl or together are $-(CH_2)_q-$;
d') Q is $CH_2$, NH, O or S;
e') m is an integer from 0 to 5;
f') n is an integer from 1 to 5;
g') o is an integer from 1 to 10;
h') q is 0 or 1;
i') r is 0, 1 or 2, or
j') v is an integer from 1 to 6;

which comprises reacting a compound of the formula II

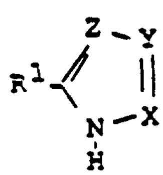

(II)

in which $R^1$, X, Y and Z are defined as above, with a compound of the formula III

$U-L-(O)_q-A$ (III)

in which L, A and q are defined as above, and U is a leaving group, where appropriate eliminating again protective groups which have been temporarily introduced, and converting the resulting compounds of the formula I, where appropriate, into the physiologically tolerated salts thereof.

**2.** The process as claimed in claim 1, in which, in formula I:

a) $R^1$ is
1. $(C_3-C_{10})$-alkyl,
2. $(C_3-C_{10})$-alkenyl,
3. $(C_3-C_{10})$-alkynyl,
4. $(C_3-C_8)$-cycloalkyl,
5. benzyl or
6. benzyl which is substituted as defined in claim 1;

b) $R^2$ is
1. hydrogen,
2. halogen,
3. nitro,
4. $C_vF_{2v+1}$,
5. pentafluorophenyl,
6. cyano,
7. phenyl,
8. phenyl-$(C_1-C_3)$-alkyl,
9. $(C_1-C_{10})$-alkyl,
10. $(C_3-C_{10})$-alkenyl,
11. phenyl-$(C_2-C_6)$-alkenyl,
12. 1-imidazolyl-$(CH_2)_m$-,
13. 1,2,3-triazolyl-$(CH_2)_o$-,
14. tetrazolyl-$(CH_2)_m$-,
15. $-(CH_2)_{o-1}-CHR^7-OR^5$,
16. $-(CH_2)_o-O-COR^3$,
17. $-COR^8$,
18. $-(CH_2)_o-CO-R^8$,
19. $-S(O)_rR^6$,
20. $-CH=CH-(CH_2)_m-CHR^3-OR^6$.
21. $-CH_2=CH-(CH_2)_m-CO-R^8$,
22. $-(CH_2)_o-NH-CO-OR^9$,
23. $-(CH_2)_o-NH-SO_2-R^9$,
24. $-(CH_2)_nF$,
25. $-(CH_2)_o-SO_3R^9$,
26. $-(CH_2)_n-SO_2-NH-CO-NR^6R^9$ or
27. a radical which is defined as under b) 7., 8., 9., 10. or 13. and which is substituted as defined in claim 1 under c) 46., 45. or 44. in each case as described for a radical of this type;

c) $R^8$ is hydrogen; $(C_1-C_5)$-alkyl, $OR^5$ or $NR^{11}R^{12}$ or morpholino;

d) T is
1. a single bond,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$ or
12. -CH=CH-and the other radicals and variables are defined as in claim 1, and the physiologically tolerated salts thereof.

**3.** The process as claimed in claim 1 or 2, in which, in formula (I), the symbols are:

a) $R^1$ is $(C_3-C_7)$-alkyl, $(C_3-C_7)$-alkenyl or $(C_3-C_7)$-alkynyl;

b) $R^2$ is
1. chlorine,
2. bromine,
3. $C_vF_{2v+1}$ with v = 1, 2 or 3,

4. pentafluorophenyl,
5. $-S(O)_rR^6$,
6. $(CH_2)_{o-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. tetrazolyl-$(CH_2)_m$-,
14. $-(CH_2)_nSO_2-NH-CO-NR^6R^9$,
15. $-(CH_2)_o-SO_3R^9$ or optionally hydroxyl-substituted $(C_1-C_6)$-alkyl;

c) $R^3$ is hydrogen or $(C_1-C_4)$-alkyl;
d) $R^6$ is hydrogen, $(C_1-C_4)$-alkyl, $(C_1-C_4)$-alkanoyl, a radical which is defined as above in claim 1 under g) 4., 6. or 9. and is substituted by 1 or 2 identical or different radicals from the series comprising halogen, hydroxyl, methoxy, nitro, cyano, $CO_2R^3$ and trifluoromethyl, or $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom;
e) $R^7$ is hydrogen, $(C_1-C_4)$-alkyl, $(C_1-C_9)$ - heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom, or $(C_6-C_{12})$-aryl-$(C_1-C_4)$-alkyl;
f) $R^8$ is hydrogen, $(C_1-C_4)$-alkyl, $OR^5$ or morpholino;
g) $R^9$ is $CF_3$, $(C_1-C_6)$-alkyl or phenyl;
h) $R^{14}$ is

1. $(C_1-C_4)$-alkyl,
2. $(C_1-C_4)$-alkoxy,
3. cyano,
4. amino,
5. nitroso,
6. nitro,
7. fluorine,
8. chlorine,
9. bromine,
10. hydroxyl,
11. $CH_2OR^7$,
12. $(C_1-C_9)$-heteroaryl-$CH_2$-, where the heteroaryl moiety is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
13. $(C_1-C_4)$-alkanoyloxy,
14. $(C_1-C_4)$-alkanoyl,
15. benzoyl,
16.

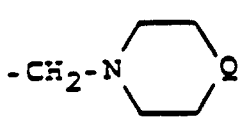

,

17. $-NH-CO-R^7$ or
18. tetrazolyl;

i) $R^{15}$ is

1. $(C_1-C_4)$-alkyl,

2. $(C_6-C_{12})$-aryl,
3. $(C_1-C_3)$-alkanoyloxy,
4. $(C_1-C_4)$-alkoxy,
5. $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
6. cyano,
7. nitro,
8. hydroxyl,
9. $-S(O)_rR^6$,
10. $-SO_3R^3$,
11. chlorine,
12. bromine,
13. benzoyl,
14. $-CO_2R^3$,
15. $-CO-NH-R^6$,
16. $-NR^6R^7$,
17. $-CO-R^8$,
18. $-SO_2-NR^6R^7$,
19.

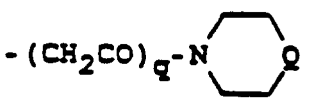

,

20.

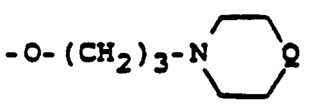

,

21. $-SO_2-NH-CO-NR^6R^9$,
22. $-PO_3H$,
23. $-CO-CHR^5-CO_2H$,
24. $-NH-CO-NH-SO_2-CH_2-R^5$,
25. 5-tetrazolyl-NH-CO-,
26.

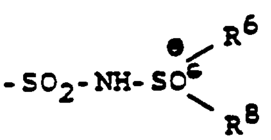

,

27.

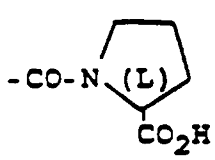

,

28.

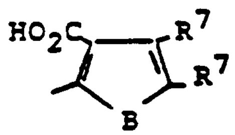 ,

29.

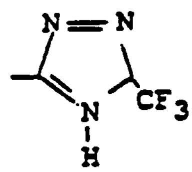 ,

30.

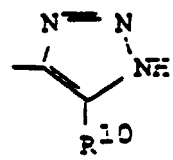 ,

31.

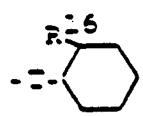 ,

32.

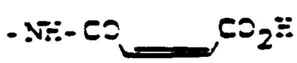 ,

33.

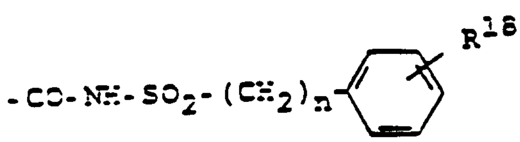

or

34. the radical which is defined under i) 2. and is substituted as defined in claim 1;

j) Q is $CH_2$, NH or O;

k) $R^{18}$ is hydrogen, methyl or ethyl;

l) T is a single bond, -O-, -CO-, -NHCO- or $-OCH_2-$

and the other radicals and variables are defined as in claim 1.

**4.** The process as claimed in any of claims 1 to 3, in which, in formula (I), the symbols are:

a) X is N, Y is $CR^2$ and Z is $CR^2$;

b) X is $CR^2$, Y is N and Z is $CR^2$;
c) X is $CR^2$, Y is $CR^2$ and Z is N or
d) X, Y and Z are each N.

**5.** The process as claimed in one or more of claims 1 to 4 in which
a) $R^2$ is chlorine, bromine, $-S(O)_rR^6$, $-COR^8$ or $-(CH_2)_nSO_2-NH-CO-NR^6R^9$;
b) $R^9$ is $(C_1-C_6)$-alkyl;
c) $R^{14}$ is tetrazolyl;
d) $R^{15}$ is $-CO_2-R^3$, $-SO_2-NR^6R^7$, $-SO_2-NH-CO-NR^6R^9$ or $-NH-CO-NH-SO_2-CH_2-R^5$;
e) Z is N;
f) X and Y are both $CR_2$;
g) q is zero;
h) L is $CH_2$;
i) A is a radical of benzothiophene, benzofuran, indole, isoindole, indazole, benzimidazole, quinoline, isoquinoline, phthalazine, quinoxaline, quinazoline, cinnoline, benzothiazole, benzothiazole 1,1-dioxide, coumarin, chroman, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thienopyridine and pyrrolopyrimidine, which can be substituted by up to 6 identical or different radicals $R^{14}$ or $-(CH_2)_{n-1}-(CHR^6-CH_2)_{o-1}-R^{15}$, and the other radicals and variables are defined as in claim 3.

**6.** A process for preparing a pharmaceutical preparation, which comprises converting a compound prepared as claimed in any of claims 1-5 together with a physiologically acceptable vehicle and, where appropriate, other additives or auxiliaries into a suitable dosage form.

**7.** A process for preparing a compound of the formula (II')

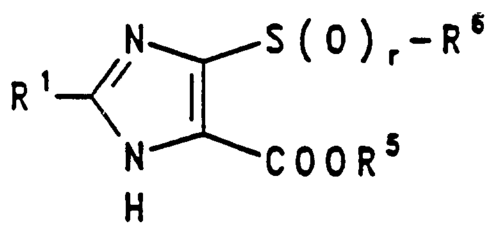

(II')

in which
a) $R^1$ is n-butyl;
b) $R^6$ is methyl;
c) $R^5$ is hydrogen or ethyl, and
d) r is 0, 1 or 2,
which comprises starting from a compound of the formula (III)

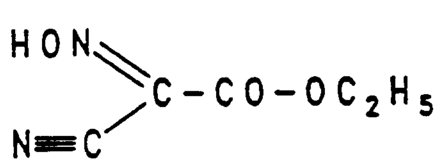

(III)

and converting the latter by subsequent
- reduction into the amino nitrile
- introduction of an alkyl group by acylation,
- addition of an alkyl mercaptan group onto the cyano group and ring closure and, where appropriate, additional oxidation of the thioalkyl group and/or hydrolysis of the ester group into a compound of the formula (II').

**8.** The process for preparing a compound of the formula (II') as claimed in claim 7, where r is 0, and $R^5$ is ethyl.

**Claims for the following Contracting State : GR**

**1.** A process for preparing a compound of the formula I

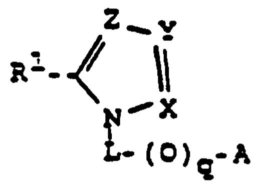

(I)

in which

a) X, Y and Z are identical or different and are N or $CR^2$,

b) $R^1$ is

1. $(C_2-C_{10})$-alkyl,
2. $(C_3-C_{10})$-alkenyl,
3. $(C_3-C_{10})$-alkynyl,
4. $OR^3$,
5. $(C_3-C_8)$-cycloalkyl,
6. $(C_4-C_{10})$-cycloalkylalkyl,
7. $(C_5-C_{10})$-cycloalkylalkenyl,
8. $(C_5-C_{10})$-cycloalkylalkynyl,
9. $-(CH_2)_m-B-(CH_2)_n-R^4$,
10. benzyl,
11. a radical which is defined as under b) 1., 2., 3. or 9. and is monosubstituted by $CO_2R^3$,
12. a radical which is defined as under b) 1., 2., 3. or 9. and in which 1 to all hydrogen atoms are replaced by fluorine, or
13. the radical which is defined under b) 10. and is substituted on the phenyl by 1 or 2 identical or different radicals from the series comprising halogen, $(C_1-C_4)$-alkoxy and nitro;

c) $R^2$ is

1. hydrogen,
2. halogen,
3. nitro,
4. $C_vF_{2v+1}$,
5. pentafluorophenyl,
6. cyano,
7. phenyl,
8. phenyl-$(C_1-C_3)$-alkyl,
9. $(C_1-C_{10})$-alkyl,
10. $(C_3-C_{10})$-alkenyl,
11. phenyl-$(C_2-C_6)$-alkenyl,
12. 1-imidazolyl-$(CH_2)_m$-,
13. 1,2,3-triazolyl-$(CH_2)_n$-,
14. tetrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,

25.

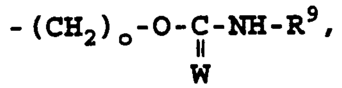

26.

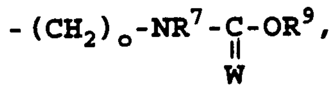

27. $-(CH_2)_o-NR^7-CO-NHR^9$,
28. $-(CH_2)_o-NR^7-SO_2R^9$,
29.

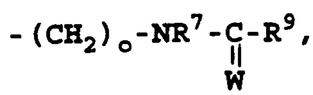

30. $-(CH_2)_nF$,
31. $-(CH_2)_n-O-NO_2$,
32. $-CH_2-N_3$,
33. $-(CH_2)_n-NO_2$,
34. $-CH=N-NR^5R^7$,
35. phthalimido-$(CH_2)_n$-,
36.

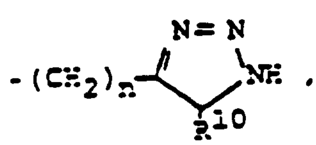

37.

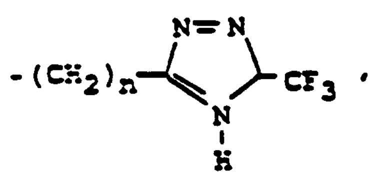

38.

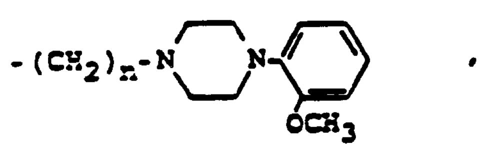

39.

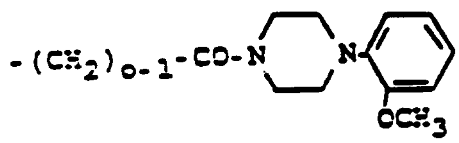,

40. phenyl-$SO_2$-NH-N = CH-,

41.

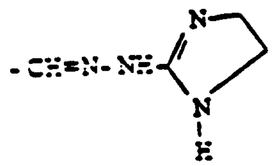,

42. -$(CH_2)_n$-$SO_2$-$NR^7$-CO-$NR^6R^9$,

43. -$(CH_2)_o$-$SO_2R^9$,

44. a radical which is defined as under c) 7. or 8. and is substituted on the phenyl by 1 or 2 identical or different radicals from the series comprising halogen, hydroxyl, methoxy, trifluoromethyl, $CO_2R^3$ and phenyl,

45. a radical which is defined as under c) 9., 10. or 18. and in which 1 to all hydrogen atoms are replaced by fluorine, or

46. the radical which is defined under c) 13. and is substituted by 1 or 2 identical or different radicals from the series comprising methoxycarbonyl and $(C_1-C_4)$-alkyl;

d) $R^3$ is

1. hydrogen,
2. $(C_1-C_8)$-alkyl,
3. $(C_3-C_8)$-cycloalkyl,
4. phenyl,
5. benzyl or
6. the radical which is defined under d) 2. and in which 1 to all hydrogen atoms are replaced by fluorine;

e) $R^4$ is

1. hydrogen,
2. $(C_1-C_6)$-alkyl,
3. $(C_3-C_8)$-cycloalkyl,
4. $(C_2-C_4)$-alkenyl or
5. $(C_2-C_4)$-alkynyl;

f) $R^5$ is

1. hydrogen,
2. $(C_1-C_6)$-alkyl,
3. $(C_3-C_8)$-cycloalkyl,
4. phenyl or
5. benzyl;

g) $R^6$ is

1. hydrogen,
2. $(C_1-C_6)$-alkyl,
3. $(C_3-C_8)$-cycloalkyl,
4. $(C_6-C_{12})$-aryl,
5. benzyl,
6. $(C_1-C_9)$-heteroaryl which can be partially or completely hydrogenated and which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,

7. $(C_1-C_4)$-alkanoyl,

8. a radical which is defined as under g) 4. or 6. and is substituted by 1 or 2 identical or different radicals from the series comprising halogen, hydroxyl, methoxy, nitro, cyano, $CO_2R^3$ and trifluoromethyl, $NR^{11}R^{12}$ or

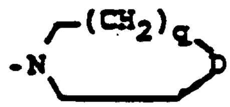

9. $(C_1-C_9)$-heteroaryl-$(C_1-C_3)$-alkyl, where the heteroaryl moiety is defined as under g) 6.;

h) $R^7$ is

1. hydrogen,
2. $(C_1-C_6)$-alkyl,
3. $(C_3-C_8)$-cycloalkyl,
4. $(C_6-C_{12})$-aryl-$(C_1-C_6)$-alkyl,
5. phenyl or
6. $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom;

i) $R^8$ is

1. hydrogen,
2. $(C_1-C_6)$-alkyl,
3. $(C_3-C_8)$-cycloalkyl,
4. phenyl-$(CH_2)_q$-,
5. $OR^5$,
6. $NR^{11}R^{12}$ or
7.

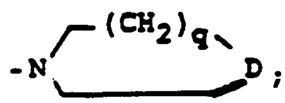

j) $R^9$ is

1. $(C_1-C_6)$-alkyl,
2. 1-adamantyl,
3. 1-naphthyl,
4. 1-naphthylethyl,
5. phenyl-$(CH_2)_q$- or
6. the radical which is defined under j) 1. and in which 1 to all hydrogen atoms are replaced by fluorine;

k) $R^{10}$ is cyano, nitro or $CO_2R^7$;

l) $R^{11}$ and $R^{12}$ are identical or different and are

1. hydrogen,
2. $(C_1-C_4)$-alkyl,
3. phenyl,
4. benzyl or
5. $\alpha$-methylbenzyl;

m) D is $NR^{13}$, O or $CH_2$;

n) $R^{13}$ is hydrogen, $(C_1-C_4)$-alkyl or phenyl;

o) A is a fused heterobicyclic radical which has 8 to 10 ring atoms, of which up to 9 ring atoms are carbon atoms, in which two atoms next to one another are common constituents of both rings, where one or both of these rings are formally derived from benzene, in which one or more CH groups are replaced by N, $O^+$ and $S^+$, and/or in which two adjacent CH groups are replaced (to form a five-membered aromatic ring) by S, NH or O, and which can be substituted by up to 6 identical or different radicals $R^{14}$ or $-(CH_2)_{n-1}-(CHR^6-CH_2)_{o-1}-R^{15}$;

p) $R^{14}$ is
1. halogen,
2. oxo,
3. nitroso,
4. nitro,
5. amino,
6. cyano,
7. hydroxyl,
8. $(C_1-C_6)$-alkyl,
9. $(C_1-C_4)$-alkanoyl,
10. $(C_1-C_4)$-alkanoyloxy,
11. $CO_2R^3$,
12. methanesulfonylamino,
13. trifluoromethanesulfonylamino,
14. $-CO-NH-OR^9$,
15. $-SO_2-NR^6R^7$,
16. $-CH_2OR^7$,
17. $(C_1-C_9)$-heteroaryl-$(CH_2)_q$-, where the heteroaryl moiety is defined as under g) 6.,
18. $(C_7-C_{13})$-aroyl,
19.

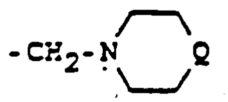

,

20.

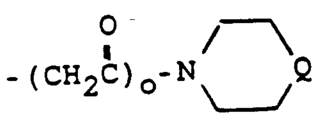

or
21. $(C_6-C_{12})$-aryl;

q) $R^{15}$ is
1. hydrogen,
2. $(C_1-C_6)$-alkyl,
3. $(C_3-C_8)$-cycloalkyl,
4. $(C_6-C_{12})$-aryl,
5. $(C_7-C_{13})$-aroyl,
6. $(C_1-C_4)$-alkoxy,
7. $(C_1-C_4)$-alkanoyloxy,
8. $(C_1-C_9)$-heteroaryl which is defined as under h) 6.,
9. $CO_2R^3$,
10. halogen,
11. cyano,
12. nitro,
13. $NR^6R^7$,
14. hydroxyl,
15. $-CO-NH-CHR^5-CO_2R^3$,
16. sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. phosphonooxy,
22. $-PO_3H_2$,

23. -NH-PO(OH)$_2$,
24. -S(O)$_r$R$^6$,
25. -CO-R$^8$,
26. -CO-NR$^6$R$^9$,
27. -CR$^{20}$(OH)-PO(OH)$_2$,
28. the radical defined under p) 20.,
29.

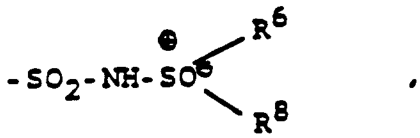

30.

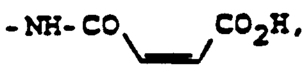

31.

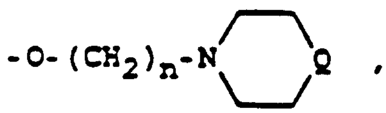

32. 5-tetrazolyl-NH-CO-,
33. -CO-NH-NH-SO$_2$CF$_3$,
34.

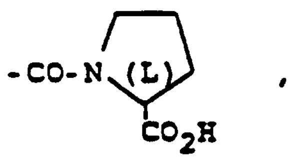

35.

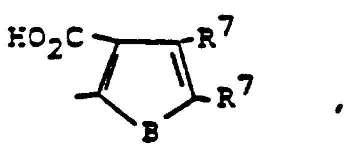

36.

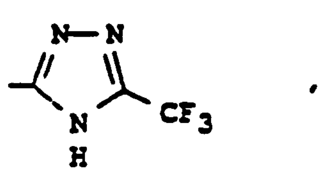

37.

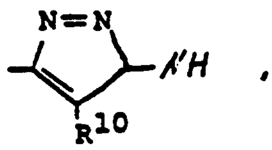

38.

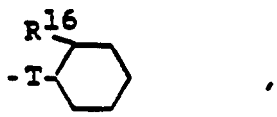

39.

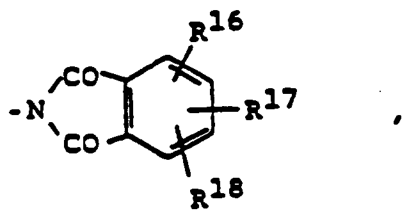

40. $-CO-NH-SO_2-R^{19}$,

41. the radical which is defined under q) 4. and is substituted by 1 or 2 identical or different radicals from the series comprising halogen, cyano, nitro, $NR^6R^7$ and hydroxyl;

r) B is O, $NR^7$ or S;

s) W is O or S;

t) L is $(C_1-C_3)$-alkanediyl;

u) $R^{16}$ is $CO_2R^3$ or $CH_2CO_2R^3$;

v) $R^{17}$ is hydrogen, halogen, $(C_1-C_4)$-alkyl or $(C_1-C_4)$-alkoxy;

w) $R^{18}$ is hydrogen, $(C_1-C_4)$-alkyl or phenyl;

x) $R^{19}$ is

1. $(C_1-C_8)$-alkyl,
2. $(C_3-C_8)$-cycloalkyl,
3. phenyl,
4. benzyl or
5. the radical which is defined under x) 1. and in which 1 to all hydrogen atoms are replaced by fluorine or chlorine;

y) T is

1. a single bond,
2. -CO-,
3. $-CH_2-$,
4. -O-,
5. -S-,
6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $-SO_2-NR^{21}-$,

16. $-CR^{20}R^{22}-NH-$,
17. $-CH=CH-$,
18. $-CF=CF-$,
19. $-CH=CF-$,
20. $-CF=CH-$,
21. $-CH_2-CH_2-$,
22. $-CF_2-CF_2-$,
23. $-CH(OR^3)-$,
24. $-CH(OCOR^5)-$,
25.

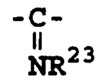

or
26.

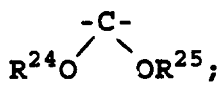

z) $R^{20}$ and $R^{22}$ are identical or different and are hydrogen, $(C_1-C_5)$-alkyl, phenyl, allyl or benzyl;
a') $R^{21}$ is hydrogen, $(C_1-C_6)$-alkyl, benzyl or allyl;
b') $R^{23}$ is
1. $NR^{20}R^{21}$,
2. ureido,
3. thioureido,
4. toluene-4-sulfonyl or
5. benzenesulfonylamino

c') $R^{24}$ and $R^{25}$ are identical or different and are $(C_1-C_4)$-alkyl or together are $-(CH_2)_q-$;
d') Q is $CH_2$, NH, O or S;
e') m is an integer from 0 to 5;
f') n is an integer from 1 to 5;
g') o is an integer from 1 to 10;
h') q is 0 or 1;
i') r is 0, 1 or 2, or
j') v is an integer from 1 to 6;

which comprises reacting a compound of the formula II

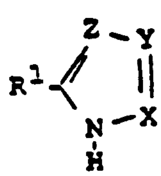

(II)

in which $R^1$, X, Y and Z are defined as above, with a compound of the formula III

$U-L-(O)_q-A$ (III)

in which L, A and q are defined as above, and U is a leaving group, where appropriate eliminating again protective groups which have been temporarily introduced, and converting the resulting compounds of the formula I, where appropriate, into the physiologically tolerated salts thereof.

**2.** The process as claimed in claim 1, in which, in formula I:
a) $R^1$ is

1. $(C_3-C_{10})$-alkyl,
2. $(C_3-C_{10})$-alkenyl,
3. $(C_3-C_{10})$-alkynyl,
4. $(C_3-C_8)$-cycloalkyl,
5. benzyl or
6. benzyl which is substituted as defined in claim 1;

b) $R^2$ is

1. hydrogen,
2. halogen,
3. nitro,
4. $C_vF_{2v+1}$,
5. pentafluorophenyl,
6. cyano,
7. phenyl,
8. phenyl-$(C_1-C_3)$-alkyl,
9. $(C_1-C_{10})$-alkyl,
10. $(C_3-C_{10})$-alkenyl,
11. phenyl-$(C_2-C_6)$-alkenyl,
12. 1-imidazolyl-$(CH_2)_m$-,
13. 1,2,3-triazolyl-$(CH_2)_o$-,
14. tetrazolyl-$(CH_2)_m$-,
15. $-(CH_2)_{o-1}-CHR^7-OR^5$,
16. $-(CH_2)_o-O-COR^3$,
17. $-COR^8$,
18. $-(CH_2)_o-CO-R^8$,
19. $-S(O)_rR^6$,
20. $-CH=CH-(CH_2)_m-CHR^3-OR^6$,
21. $-CH_2=CH-(CH_2)_m-CO-R^8$,
22. $-(CH_2)_o-NH-CO-OR^9$,
23. $-(CH_2)_o-NH-SO_2-R^9$,
24. $-(CH_2)_nF$,
25. $-(CH_2)_o-SO_3R^9$,
26. $-(CH_2)_n-SO_2-NH-CO-NR^6R^9$ or
27. a radical which is defined as under b) 7., 8., 9., 10. or 13. and which is substituted as defined in claim 1 under c) 46., 45. or 44. in each case as described for a radical of this type;

c) $R^8$ is hydrogen; $(C_1-C_5)$-alkyl, $OR^5$ or $NR^{11}R^{12}$ or morpholino;

d) T is

1. a single bond,
2. -CO-,
3. $-CONR^{21}-$,
4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$ or
12. $-CH=CH-$

and the other radicals and variables are as defined in claim 1, and the physiologically tolerated salts thereof.

**3.** The process as claimed in claim 1 or 2, in which, in formula (I), the symbols are:

a) $R^1$ is $(C_3-C_7)$-alkyl, $(C_3-C_7)$-alkenyl or $(C_3-C_7)$-alkynyl;

b) $R^2$ is

1. chlorine,
2. bromine,
3. $C_vF_{2v+1}$ with v = 1, 2 or 3,

4. pentafluorophenyl,
5. $-S(O)_rR^6$,
6. $(CH_2)_{o-1}$-$CHR^7$-$OR^5$,
7. $(CH_2)_o$-O-CO-$R^3$,
8. $-COR^8$,
9. $-(CH_2)_o$-CO-$R^8$,
10. $-CH_2$-NH-CO-$R^8$,
11. $-(CH_2)_o$-NH-$SO_2$-$R^9$,
12. -CH = CH-$CHR^3$-$OR^6$,
13. tetrazolyl-$(CH_2)_m$-,
14. $-(CH_2)_nSO_2$-NH-CO-$NR^6R^9$,
15. $-(CH_2)_o$-$SO_3R^9$ or
optionally hydroxyl-substituted ($C_1$-$C_6$)-alkyl;

c) $R^3$ is hydrogen or ($C_1$-$C_4$)-alkyl;

d) $R^6$ is hydrogen, ($C_1$-$C_4$)-alkyl, ($C_1$-$C_4$)-alkanoyl, a radical which is defined as above in claim 1 under g) 4., 6. or 9. and is substituted by 1 or 2 identical or different radicals from the series comprising halogen, hydroxyl, methoxy, nitro, cyano, $CO_2R^3$ and trifluoromethyl, or ($C_1$-$C_9$)-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom;

e) $R^7$ is hydrogen, ($C_1$-$C_4$)-alkyl, ($C_1$-$C_9$)-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom, or ($C_6$-$C_{12}$)-aryl-($C_1$-$C_4$)-alkyl;

f) $R^8$ is hydrogen, ($C_1$-$C_4$)-alkyl, $OR^5$ or morpholino;

g) $R^9$ is $CF_3$, ($C_1$-$C_6$)-alkyl or phenyl;

h) $R^{14}$ is
1. ($C_1$-$C_4$)-alkyl,
2. ($C_1$-$C_4$)-alkoxy,
3. cyano,
4. amino,
5. nitroso,
6. nitro,
7. fluorine,
8. chlorine,
9. bromine,
10. hydroxyl,
11. $CH_2OR^7$,
12. ($C_1$-$C_9$)-heteroaryl-$CH_2$-, where the heteroaryl moiety is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
13. ($C_1$-$C_4$)-alkanoyloxy,
14. ($C_1$-$C_4$)-alkanoyl,
15. benzoyl,
16.

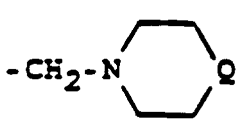

,

17. -NH-CO-$R^7$ or
18. tetrazolyl;

i) $R^{15}$ is
1. ($C_1$-$C_4$)-alkyl,

2. $(C_6-C_{12})$-aryl,
3. $(C_1-C_3)$-alkanoyloxy,
4. $(C_1-C_4)$-alkoxy,
5. $(C_1-C_9)$-heteroaryl which is derived from phenyl or naphthyl, in which one or more CH groups are replaced by N and/or in which at least two adjacent CH groups are replaced (to form a five-membered aromatic ring) by S, NH or O, where 1 or both atoms at the fusion point of bicyclic radicals (such as in indolizinyl) can also be an N atom,
6. cyano,
7. nitro,
8. hydroxyl,
9. $-S(O)_rR^6$,
10. $-SO_3R^3$,
11. chlorine,
12. bromine,
13. benzoyl,
14. $-CO_2R^3$,
15. $-CO-NH-R^6$,
16. $-NR^6R^7$,
17. $-CO-R^8$,
18. $-SO_2-NR^6R^7$,
19.

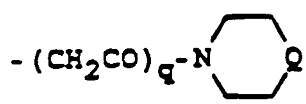
,

20.

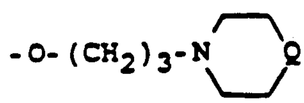
,

21. $-SO_2-NH-CO-NR^6R^9$,
22. $-PO_3H$,
23. $-CO-CHR^5-CO_2H$,
24. $-NH-CO-NH-SO_2-CH_2-R^5$,
25. 5-tetrazolyl-NH-CO-,
26.

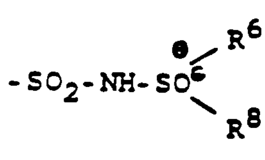
,

27.

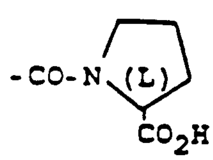
,

28.

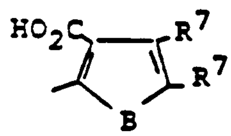 ,

29.

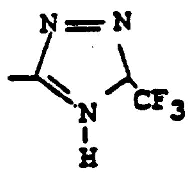 ,

30.

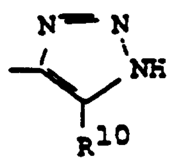 ,

31.

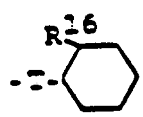 ,

32.

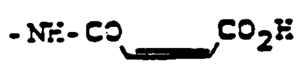 ,

33.

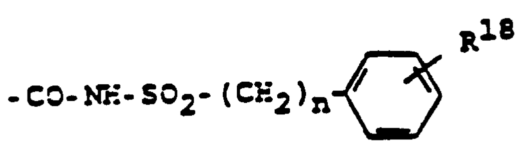

or

34. the radical which is defined under i) 2. and is substituted as defined in claim 1;

j) Q is $CH_2$, NH or O;

k) $R^{18}$ is hydrogen, methyl or ethyl;

l) T is a single bond, -O-, -CO-, -NHCO- or $-OCH_2-$

and the other radicals and variables are defined as in claim 1.

**4.** The process as claimed in any of claims 1 to 3, in which, in formula (I), the symbols are:

a) X is N, Y is $CR^2$ and Z is $CR^2$;

b) X is $CR^2$, Y is N and Z is $CR^2$;

c) X is $CR^2$, Y is $CR^2$ and Z is N or
d) X, Y and Z are each N.

5. The process as claimed in one or more of claims 1 to 4, in which
a) $R^2$ is chlorine, bromine, $-S(O)_rR^6$, $-COR^8$ or $-(CH_2)_nSO_2-NH-CO-NR^6R^9$;
b) $R^9$ is $(C_1-C_6)$-alkyl;
c) $R^{14}$ is tetrazolyl;
d) $R^{15}$ is $-CO_2-R^3$, $-SO_2-NR^6R^7$, $-SO_2-NH-CO-NR^6R^9$ or $-NH-CO-NH-SO_2-CH_2-R^5$;
e) Z is N;
f) X and Y are both $CR_2$;
g) q is zero;
h) L is $CH_2$;
i) A is a radical of benzothiophene, benzofuran, indole, isoindole, indazole, benzimidazole, quinoline, isoquinoline, phthalazine, quinoxaline, quinazoline, cinnoline, benzothiazole, benzothiazole-1,1-dioxide, coumarin, chromane, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thienopyridine and pyrrolopyrimidine, which can be substituted by up to 6 identical or different radicals $R^{14}$ or $-(CH_2)_{n-1}-(CHR^6-CH_2)_{o-1}-R^{15}$, and the other radicals and variables are defined as in claim 3.

6. A process for preparing a pharmaceutical preparation, which comprises converting a compound prepared as claimed in any of claims 1-5 together with a physiologically acceptable vehicle and, where appropriate, other additives or auxiliaries into a suitable dosage form.

7. A process for preparing a compound of the formula (II')

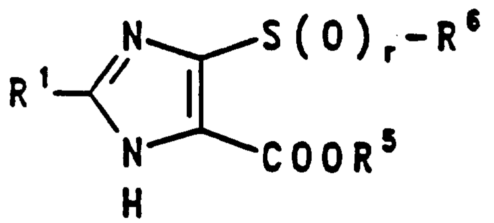

(II')

in which
a) $R^1$ is n-butyl;
b) $R^6$ is methyl;
c) $R^5$ is hydrogen or ethyl, and
d) r is 0, 1 or 2,
which comprises starting from a compound of the formula (III)

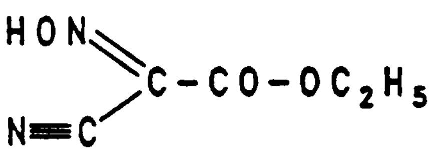

(III)

and converting the latter by subsequent
- reduction into the amino nitrile
- introduction of an alkyl group by acylation,
- addition of an alkyl mercaptan group onto the cyano group and ring closure and, where appropriate, additional oxidation of the thioalkyl group and/or hydrolysis of the ester group into a compound of the formula (II').

8. The process for preparing a compound of the formula (II') as claimed in claim 7, where r is 0, and $R^5$ is ethyl.

**9.** A compound of the formula (II')

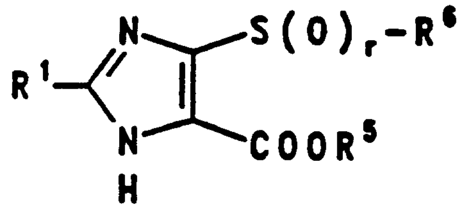

(II')

in which

a) $R^1$ is n-butyl;
b) $R^6$ is methyl;
c) $R^5$ is hydrogen or ethyl, and
d) r is 0, 1 or 2.

**10.** A compound of the formula (II') as claimed in claim 9, where r is 0, and $R^5$ is ethyl.

**Revendications**

**Revendications pour les Etats contractants suivants : AT, BE, CH, DE, DK, FR, GB, IT, LI, LU, NL, SE**

**1.** Composé de formule I

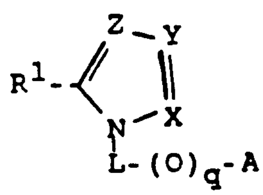

(I)

dans laquelle

a) X, Y et Z sont identiques ou différents et représentent N ou $CR^2$,
b) $R^1$ représente
1. un groupe alkyle en $C_2$-$C_{10}$,
2. un groupe alcényle en $C_3$-$C_{10}$,
3. un groupe alcynyle en $C_3$-$C_{10}$,
4. $OR^3$,
5. un groupe cycloalkyle en $C_3$-$C_8$,
6. un groupe cycloalkylalkyle en $C_4$-$C_{10}$,
7. un groupe cycloalkylalcényle en $C_5$-$C_{10}$,
8. un groupe cycloalkylalcynyle en $C_5$-$C_{10}$,
9. $-(CH_2)_m-B-(CH_2)_n-R^4$,
10. le groupe benzyle,
11. un radical tel que défini en b) 1., 2., 3. ou 9., qui est monosubstitué par $CO_2R^3$,
12. un radical tel que défini en b) 1., 2., 3. ou 9., dans lequel de 1 à l'ensemble des atomes d'hydrogène sont remplacés par des atomes de fluor, ou
13. le radical défini en b) 10. qui est substitué sur le fragment phényle par 1 ou 2 atomes ou radicaux identiques ou différents choisis parmi des atomes d'halogène, des groupes alcoxy en $C_1$-$C_4$ et nitro;
c) $R^2$ représente
1. un atome d'hydrogène,
2. un atome d'halogène,
3. le groupe nitro,
4. $C_vF_{2v+1}$,
5. le groupe pentafluorophényle,
6. le groupe cyano,

7. le groupe phényle,
8. un groupe phényl-alkyle($C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_n$-,
14. un groupe tétrazolyl-$(CH_2)_m$-,
15. -$(CH_2)_{o-1}$-$CHR^7$-$OR^5$,
16. -$(CH_2)_o$-O-CO-$R^3$,
17. -$(CH_2)_o$-S-$R^6$,
18. -$S(O)_r$-$R^6$,
19. -CH = CH-$(CH_2)_m$-$CHR^3$-$OR^6$,
20. -$CH_2$ = CH-$(CH_2)_m$-CO-$R^8$,
21. -CO-$R^8$,
22. -CH = CH-$(CH_2)_m$-O-CO-$R^7$,
23. -$(CH_2)_m$-$CH(CH_3)$-CO-$R^8$,
24. -$(CH_2)_o$-CO-$R^8$,
25.

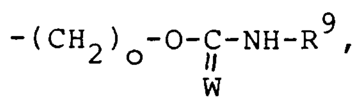

26.

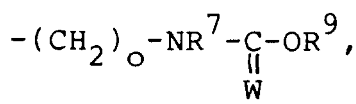

27. -$(CH_2)_o$-$NR^7$-CO-$NHR^9$,
28. -$(CH_2)_o$-$NR^7$-$SO_2R^9$,
29

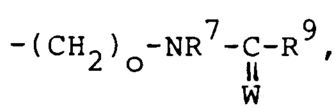

.
30. -$(CH_2)_nF$,
31. -$(CH_2)_n$-O-$NO_2$,
32. -$CH_2$-$N_3$,
33. -$(CH_2)_n$-$NO_2$,
34. -CH = N-$NR^5R^7$,
35. un groupe phtalimido-$(CH_2)_n$-,
36.

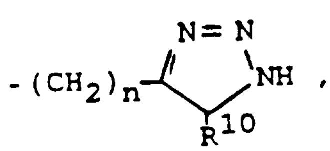

37.

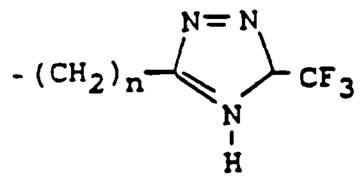 ,

38.

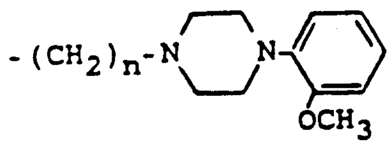 ,

39.

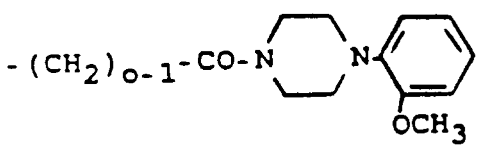 ,

40. un groupe phényl-$SO_2$-NH-N = CH-,

41.

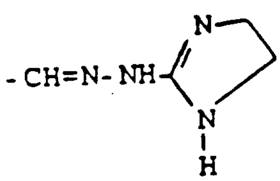 ,

42. -$(CH_2)_n$-$SO_2$-$NR^7$-CO-$NR^6R^9$,

43. -$(CH_2)_o$-$SO_2R^9$,

44. un radical tel que défini en c) 7. ou 8., qui est substitué sur le fragment phényle par 1 ou 2 atomes ou radicaux identiques ou différents, choisis parmi des atomes d'halogène et des groupes hydroxy, méthoxy, trifluorométhyle, $CO_2R^3$ et phényle,

45. un radical tel que défini en c) 9., 10. ou 18., dans lequel de 1 à l'ensemble des atomes d'hydrogène sont remplacés par des atomes de fluor, ou

46. le radical défini en c) 13., qui est substitué par un ou deux radicaux identiques ou différents choisis parmi des groupes méthoxycarbonyle et alkyle en $C_1$-$C_4$;

d) $R^3$ représente

1. un atome d'hydrogène,
2. un groupe alkyle en $C_1$-$C_8$,
3. un groupe cycloalkyle en $C_3$-$C_8$,
4. le groupe phényle,
5. le groupe benzyle ou
6. le radical défini en d) 2., dans lequel de 1 à l'ensemble des atomes d'hydrogène sont remplacés par des atomes de fluor;

e) $R^4$ représente

1. un atome d'hydrogène,
2. un groupe alkyle en $C_1$-$C_6$,
3. un groupe cycloalkyle en $C_3$-$C_8$,
4. un groupe alcényle en $C_2$-$C_4$ ou

5. un groupe alcynyle en $C_2$-$C_4$;

f) $R^5$ représente

1. un atome d'hydrogène,
2. un groupe alkyle en $C_1$-$C_6$,
3. un groupe cycloalkyle en $C_3$-$C_8$,
4. le groupe phényle ou
5. le groupe benzyle;

g) $R^6$ représente

1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. le groupe benzyle,
6. un groupe hétéroaryle en $C_1$-$C_9$, qui peut être partiellement ou totalement hydrogéné et qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux de la position (ou point) de condensation de radicaux bicycliques (comme dans le radical indolizinyle) pouvant également être un (des) atome(s) d'azote,
7. un groupe alcanoyle en $C_1$-$C_4$,
8. un radical tel que défini en g) 4. ou 6., substitué par 1 ou 2 atomes ou radicaux choisis parmi des atomes d'halogène et des groupes hydroxy, méthoxy, nitro, cyano, $CO_2R^3$ et trifluorométhyle, $NR^{11}R^{12}$ ou

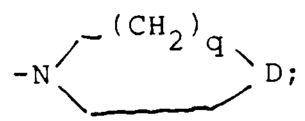

9. un groupe hétéroaryl($C_1$-$C_9$)-alkyle($C_1$-$C_3$), le fragment hétéroaryle étant tel que défini en g) 6.;

h) $R^7$ représente

1. un atome d'hydrogène,
2. un groupe alkyle en $C_1$-$C_6$,
3. un groupe cycloalkyle en $C_3$-$C_8$,
4. un groupe aryl($C_6$-$C_{12}$)-alkyle($C_1$-$C_6$),
5. le groupe phényle ou
6. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote;

i) $R^8$ représente

1. un atome d'hydrogène,
2. un groupe alkyle en $C_1$-$C_6$,
3. un groupe cycloalkyle en $C_3$-$C_8$,
4. un groupe phényl-$(CH_2)_q$-,
5. $OR^5$,
6. $NR^{11}R^{12}$ ou
7.

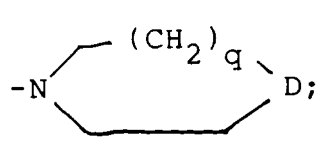

j) $R^9$ représente

1. un groupe alkyle en $C_1$-$C_6$,

2. le groupe 1-adamantyle,
3. le groupe 1-naphtyle,
4. le groupe 1-naphtyléthyle,
5. un groupe phényl-$(CH_2)_q$- ou
6. le radical défini en j) 1., dans lequel de 1 à l'ensemble des atomes d'hydrogène sont remplacés par des atomes de fluor;

k) $R^{10}$ représente un groupe cyano, nitro ou $CO_2R^7$;

l) $R^{11}$ et $R^{12}$ sont identiques ou différents et représentent
1. un atome d'hydrogène,
2. un groupe alkyle en $C_1$-$C_4$,
3. le groupe phényle,
4. le groupe benzyle ou
5. le groupe $\alpha$-méthylbenzyle;

m) D représente $NR^{13}$, O ou $CH_2$;

n) $R^{13}$ représente un atome d'hydrogène ou un groupe alkyle en $C_1$-$C_4$ ou phényle;

o) A représente le reste d'un hétérocycle bicyclique condensé ayant de 8 à 10 atomes formant les cycles, dont jusqu'à 9 atomes formant les cycles sont des atomes de carbone, dans lequel deux atomes adjacents sont des composants communs des deux cycles, un de ces cycles ou les deux dérivant, de par la formule, du benzène, dans lequel un ou plusieurs groupes CH sont remplacés par N, $O^+$ et $S^+$ et/ou dans lequel deux groupes CH contigus (avec formation d'un noyau aromatique à cinq chaînons) sont remplacés par S, NH ou O; qui peut être substitué par jusqu'à six radicaux $R^{14}$ ou $-(CH_2)_{n-1}-(CHR^6-CH_2)_{o-1}-R^{15}$, identiques ou différents;

p) $R^{14}$ représente
1. un atome d'halogène,
2. le groupe oxo,
3. le groupe nitroso,
4. le groupe nitro,
5. le groupe amino,
6. le groupe cyano,
7. le groupe hydroxy,
8. un groupe alkyle en $C_1$-$C_6$,
9. un groupe alcanoyle en $C_1$-$C_4$,
10. un groupe alcanoyloxy en $C_1$-$C_4$,
11. $CO_2R^3$,
12. le groupe méthanesulfonylamino,
13. le groupe trifluorométhanesulfonylamino,
14. $-CO-NH-OR^9$,
15. $-SO_2-NR^6R^7$,
16. $-CH_2-OR^7$,
17. un groupe hétéroaryl$(C_1$-$C_9)$-$(CH_2)_q$-, le fragment hétéroaryle étant tel que défini en h) 6.;
18. un groupe aroyle en $C_7$-$C_{13}$,
19.

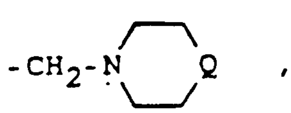

20.

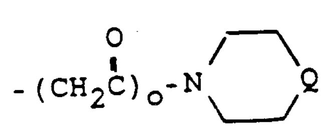

ou

21. un groupe aryle en $C_6$-$C_{12}$;

q) $R^{15}$ représente

1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. un groupe aroyle en $C_7$-$C_{13}$,
6. un groupe alcoxy en $C_1$-$C_4$,
7. un groupe alcanoyloxy en $C_1$-$C_4$,
8. un groupe hétéroaryle en $C_1$-$C_9$ qui est tel que défini en h) 6.,
9. $CO_2R^3$,
10. un atome d'halogène,
11. le groupe cyano,
12. le groupe nitro,
13. $NR^6R^7$,
14. le groupe hydroxy,
15. $-CO-NH-CHR^5-CO_2R^3$,
16. le groupe sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. le groupe phosphono-oxy.
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. le radical défini en p) 20.,
29.

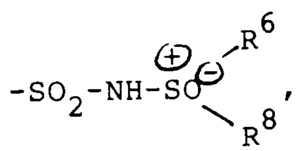

30.

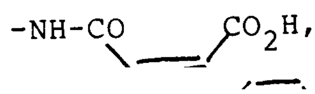

31.

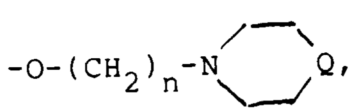

32. le groupe 5-tétrazolyl-NH-CO,
33. $-CO-NH-NH-SO_2CF_3$,

34.

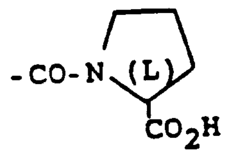

,

35.

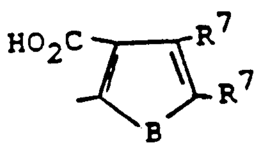

,

36.

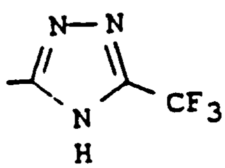

,

37.

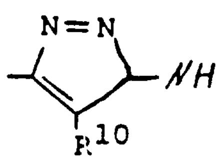

,

38.

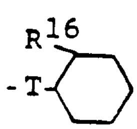

,

39.

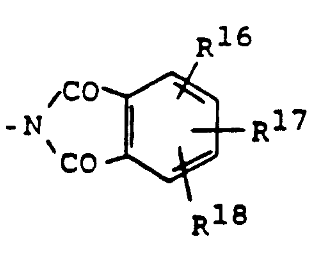

,

40. $-CO-NH-SO_2-R^{19}$

41. le radical défini en q) 4., substitué par 1 ou 2 atomes ou radicaux identiques ou différents choisis parmi des atomes d'halogène et des groupes cyano, nitro, $NR^6R^7$ et hydroxy;

r) B représente O, $NR^7$ ou S;

s) W représente O ou S;

t) L représente un groupe alcanediyle en $C_1$-$C_3$;
u) $R^{16}$ représente $CO_2R^3$ ou $CH_2CO_2R^3$;
v) $R^{17}$ représente un atome d'hydrogène ou d'halogène ou un groupe alkyle en $C_1$-$C_4$ ou alcoxy en $C_1$-$C_4$;
w) $R^{18}$ représente un atome d'hydrogène ou un groupe alkyle en $C_1$-$C_4$ ou phényle;
x) $R^{19}$ représente
1. un groupe alkyle en $C_1$-$C_8$,
2. un groupe cycloalkyle en $C_3$-$C_8$,
3. le groupe phényle,
4. le groupe benzyle ou
5. le radical défini en x) 1., dans lequel de 1 à l'ensemble des atomes d'hydrogène sont remplacés par des atomes de fluor ou de chlore;
y) T représente
1. une simple liaison,
2. -CO-,
3. $-CH_2-$,
4. -O-,
5. -S-,
6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $-SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

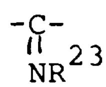

ou
26.

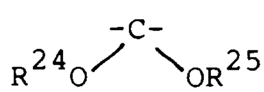
;

z) $R^{20}$ et $R^{22}$ sont identiques ou différents et représentent un atome d'hydrogène ou un groupe alkyle en $C_1$-$C_5$, phényle, allyle ou benzyle;
a') $R^{21}$ représente un atome d'hydrogène ou un groupe alkyle en $C_1$-$C_6$, benzyle ou allyle,
b') $R^{23}$ représente
1. $NR^{20}R^{21}$,
3. le groupe uréido,
3. le groupe thiouréido,
4. le groupe toluène-4-sulfonyle ou

5. le groupe benzènesulfonylamino;
c') $R^{24}$ et $R^{25}$ sont identiques ou différents et représentent un groupe alkyle en $C_1$-$C_4$, ou forment ensemble un groupe $-(CH_2)_q-$;
d') Q représente $CH_2$, NH, O ou S;
e') m est un nombre entier allant de 0 à 5;
f') n est un nombre entier allant de 1 à 5;
g') o est un nombre entier allant de 1 à 10;
h') q est 0 ou 1;
i') r est 0, 1 ou 2; ou
j') v est un nombre entier allant de 1 à 6; et sels physiologiquement acceptables de ce composé.

**2.** Composé de formule I selon la revendication 1, dans lequel
a) $R^1$ représente
1. un groupe alkyle en $C_3$-$C_{10}$,
2. un groupe alcényle en $C_3$-$C_{10}$,
3. un groupe alcynyle en $C_3$-$C_{10}$,
4. un groupe cycloalkyle en $C_3$-$C_8$,
5. le groupe benzyle ou
6. un groupe benzyle qui est substitué comme défini dans la revendication 1;
b) $R^2$ représente
1. un atome d'hydrogène,
2. un atome d'halogène,
3. le groupe nitro,
4. $C_vF_{2v+1}$,
5. le groupe pentafluorophényle,
6. le groupe cyano,
7. le groupe phényle,
8. un groupe phényl-alkyle($C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_o$-,
14. un groupe tétrazolyl-$(CH_2)_m$-,
15. $-(CH_2)_{o-1}-CHR^7-OR^5$,
16. $-(CH_2)_o-O-COR^3$,
17. $-COR^8$,
18. $-(CH_2)_o-CO-R^8$,
19. $-S(O)_rR^6$,
20. $-CH=CH-(CH_2)_m-CHR^3-OR^6$,
21. $-CH_2=CH-(CH_2)_m-CO-R^8$,
22. $-(CH_2)_o-NH-CO-OR^9$,
23. $-(CH_2)_o-NH-SO_2-R^9$,
24. $-(CH_2)_nF$,
25. $-(CH_2)_o-SO_3R^9$,
26. $-(CH_2)_n-SO_2-NH-CO-NR^6R^9$ ou
27. un radical défini comme en b) 7., 8., 9., 10. ou 13., qui est substitué comme décrit pour un tel radical dans la revendication 1 en c) 46., 45. ou 44.;
c) $R^8$ représente un atome d'hydrogène ou un groupe alkyle en $C_1$-$C_5$, $OR^5$, $NR^{11}R^{12}$ ou morpholino;
d) T représente
1. une simple liaison,
2. -CO-,
3. $-CONR^{21}$,
4. $-CH_2-CH_2-$,
5. $-NR^{21}-CO-$,
6. $-O-CH_2-$,
7. $-CH_2-O-$,
8. $-S-CH_2-$,

9. $-CH_2-S-$,
10. $-NH-CH_2-$,
11. $-CH_2-NH-$ ou
12. $-CH=CH-$

et les autres radicaux et variables sont tels que définis dans la revendication 1, et sels physiologiquement acceptables de ce composé.

3. Composé de formule I selon la revendication 1 ou 2, dans lequel
a) $R^1$ représente un groupe alkyle en $C_3$-$C_7$, alcényle en $C_3$-$C_7$ ou alcynyle en $C_3$-$C_7$;
b) $R^2$ représente
1. un atome de chlore,
2. un atome de brome,
3. $C_vF_{2v+1}$, avec v = 1, 2 ou 3,
4. le groupe pentafluorophényle,
5. $-S(O)_rR^6$,
6. $(CH_2)_{o-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. un groupe tétrazolyl-$(CH_2)_m$-,
14. $-(CH_2)_nSO_2-NH-CO-NR^6R^9$,
15. $-(CH_2)_o-SO_3R^9$ ou un radical alkyle en $C_1$-$C_6$ éventuellement substitué par le groupe hydroxy;
c) $R^3$ représente un atome d'hydrogène ou un groupe alkyle en $C_1$-$C_4$;
d) $R^6$ représente un atome d'hydrogène ou un groupe alkyle en $C_1$-$C_4$, alcanoyle en $C_1$-$C_4$, un radical tel que défini plus haut dans la revendication 1 en g) 4., 6. ou 9., substitué par 1 ou 2 atomes ou radicaux identiques ou différents, choisis parmi des atomes d'halogène et des groupes hydroxy, méthoxy, nitro, cyano, $CO_2R^3$ et trifluorométhyle, ou un radical hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,
e) $R^7$ représente un atome d'hydrogène ou un groupe alkyle en $C_1$-$C_4$, hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote, ou aryl($C_6$-$C_{12}$)-alkyle($C_1$-$C_4$);
f) $R^8$ représente un atome d'hydrogène ou un groupe alkyle en $C_1$-$C_4$, $OR^5$ ou morpholino;
g) $R^9$ représente $CF_3$, un groupe alkyle en $C_1$-$C_6$ ou phényle;
h) $R^{14}$ représente
1. un groupe alkyle en $C_1$-$C_4$,
2. un groupe alcoxy en $C_1$-$C_4$,
3. le groupe cyano,
4. le groupe amino,
5. le groupe nitroso,
6. le groupe nitro,
7. un atome de fluor,
8. un atome de chlore,
9. un atome de brome,
10. le groupe hydroxy,
11. $CH_2OR^7$,
12. un groupe hétéroaryl($C_1$-$C_9$)-$CH_2$-, le fragment hétéroaryle dérivant du groupe phényle ou naphtyle, dans lequel un ou plusieurs lequel un ou plusieurs groupes CH sont remplacés par N et/ou dans lequel au moins deux groupes CH contigus (avec formation d'un noyau aromatique à

cinq chaînons) sont rémplacés par S, NH ou O, un atome ou les deux du point de condensation de radicaux bicycliques (comme dans le radical indolizinyle) pouvant également être un(des) atome(s) d'azote,

13. un groupe alcanoyloxy en $C_1$-$C_4$,
14. un groupe alcanoyle en $C_1$-$C_4$,
15. le groupe benzoyle,
16.

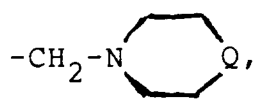

17. -NH-CO-$R^7$ ou
18. le groupe tétrazolyle;

i) $R^{15}$ représente

1. un groupe alkyle en $C_1$-$C_4$,
2. un groupe aryle en $C_6$-$C_{12}$,
3. un groupe alcanoyloxy en $C_1$-$C_3$,
4. un groupe alcoxy en $C_1$-$C_4$,
5. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,
6. le groupe cyano,
7. le groupe nitro,
8. le groupe hydroxy,
9. -$S(O)_rR^6$,
10. -$SO_3R^3$,
11. un atome de chlore,
12. un atome de brome,
13. le groupe benzoyle,
14. -$CO_2R^3$,
15. -CO-NH-$R^6$,
16. -$NR^6R^7$,
17. -CO-$R^8$,
18. -$SO_2$-$NR^6R^7$,
19.

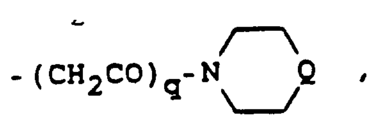

20.

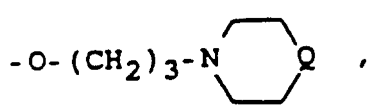

21. -$SO_2$-NH-CO-$NR^6R^9$,
22. -$PO_3H$,
23. -CO-$CHR^5$-$CO_2H$,
24. -NH-CO-$SO_2$-$CH_2$-$R^5$,
25. le groupe 5-tétrazolyl-NH-CO-,

26.

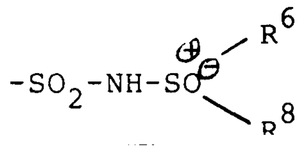

27.

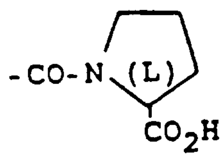

,

28.

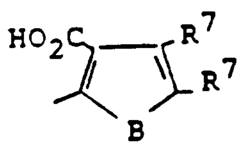

,

29.

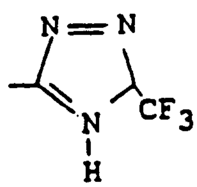

,

30.

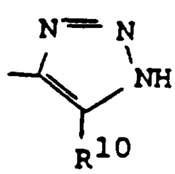

,

31.

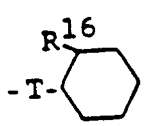

,

32.

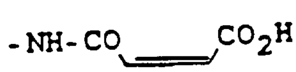

,

33.

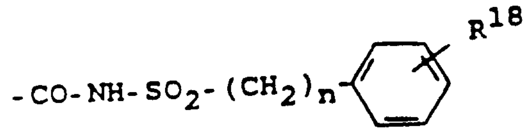

ou

34. le radical défini en i) 2., substitué comme défini dans la revendication 1;

j) Q représente $CH_2$, NH ou O;

k) $R^{18}$ représente un atome d'hydrogène ou le groupe méthyle ou éthyle;

l) T représente une simple liaison, -O-, -CO-, -NHCO- ou $-OCH_2-$

et les autres radicaux et variables sont tels que définis dans la revendication 1, et sels physiologiquement acceptables de ce composé.

**4.** Composé selon l'une des revendications 1 à 3, dans lequel

a) X représente N, Y représente $CR^2$ et Z représente $CR^2$;

b) X représente $CR^2$, Y représente N et Z représente $CR^2$;

c) X représente $CR^2$, Y représente $CR^2$ et Z représente N; ou

d) X, Y et Z représentent chacun N,

et sels physiologiquement acceptables de ce composé.

**5.** Composé selon une ou plusieurs des revendications 1 à 4, dans lequel

a) $R^2$ représente un atome de chlore ou de brome, ou un groupe $-S(O)_rR^6$, $-COR^8$ ou $-(CH_2)_nSO_2-NH-CO-NR^6R^9$;

b) $R^9$ représente un groupe alkyle en $C_1-C_6$;

c) $R^{14}$ représente le groupe tétrazolyle;

d) $R^{15}$ représente un groupe $-CO_2-R^3$, $-SO_2-NR^6R^7$, $-SO_2-NH-CO-NR^6R^9$ ou $-NH-CO-NH-SO_2-CH_2-R^5$;

e) Z est N ;

f) X et Y représentent l'un et l'autre $CR_2$;

g) q est zéro;

h) L représente $CH_2$;

i) A représente un reste de benzothiophène, benzofuranne, indole, isoindole, indazole, benzimidazole, quinoléine, isoquinoléine, phtalazine, quinoxaline, quinazoline, cinnoline, benzothiazole, benzothiazol-1,1-dioxyde, coumarine, chromane, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thiénopyridine ou pyrrolopyrimidine, qui peut être substitué par jusqu'à 6 radicaux $R^{14}$ ou $-(CH_2)_{n-1}-(CHR^6-CH_2)_{o-1}-R^{15}$, identiques ou différents,

et les autres radicaux et variables sont tels que définis dans la revendication 3,

et sels physiologiquement acceptables de ce composé.

**6.** Procédé pour la préparation d'un composé de formule I selon l'une des revendications 1 à 4, caractérisé en ce que l'on fait réagir un composé de formule II

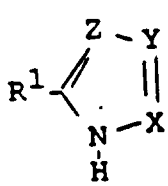

(II)

dans laquelle $R^1$, X, Y et Z sont tels que définis dans la revendication 1, avec un composé de formule III

U-L-$(O)_q$-A (III)

dans laquelle L, A et q sont tels que définis dans la revendication 1, et U représente un groupe partant, éventuellement on élimine des groupes protecteurs temporairement introduits , et éventuellement on convertit les composés de formule I obtenus en leurs sels physiologiquement acceptables.

**7.** Composé selon l'une des revendications 1 à 5, pour utilisation en tant que médicament.

**8.** Composé selon l'une des revendications 1 à 5, pour utilisation dans le traitement de l'hypertension artérielle.

**9.** Composition pharmaceutique contenant un composé selon l'une des revendications 1 à 5.

**10.** Procédé pour la fabrication d'une composition selon la revendication 9, caractérisé en ce que l'on met sous une présentation appropriée un composé préparé selon l'une des revendications 1 à 5, conjointement avec un véhicule physiologiquement acceptable et éventuellement d'autres additifs ou adjuvants.

**11.** Composé de formule

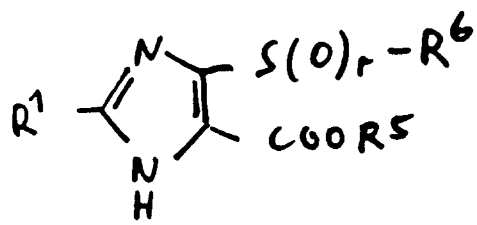

dans laquelle
a) $R^1$ représente le groupe n-butyle,
b) $R^6$ représente le groupe méthyle,
c) $R^5$ représente un atome d'hydrogène ou le groupe éthyle et
d) r est 0, 1 ou 2.

**12.** Composé de formule selon la revendication 11, dans lequel r est égal à 0 et $R^5$ représente le groupe éthyle.

**Revendications pour l'Etat contractant suivant : ES**

**1.** Procédé pour la préparation d'un composé de formule I dans laquelle

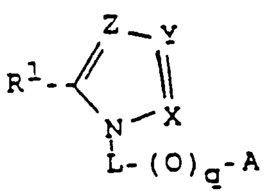

(I)

a) X, Y et Z sont identiques ou différents et représentent N ou $CR^2$,
b) $R^1$ représente
1. un groupe alkyle en $C_2$-$C_{10}$,
2. un groupe alcényle en $C_3$-$C_{10}$,
3. un groupe alcynyle en $C_3$-$C_{10}$,
4. $OR^3$,
5. un groupe cycloalkyle en $C_3$-$C_8$,
6. un groupe cycloalkylalkyle en $C_4$-$C_{10}$,

7. un groupe cycloalkylalcényle en $C_5$-$C_{10}$,
8. un groupe cycloalkylalcynyle en $C_5$-$C_{10}$,
9. $-(CH_2)_m-B-(CH_2)_n-R^4$,
10. le groupe benzyle,
11. un radical tel que défini en b) 1., 2., 3. ou 9., qui est monosubstitué par $CO_2R^3$,
12. un radical tel que défini en b) 1., 2., 3. ou 9., dans lequel de 1 à l'ensemble des atomes d'hydrogène sont remplacés par des atomes de fluor, ou
13. le radical défini en b) 10. qui est substitué sur le fragment phényle par 1 ou 2 atomes ou radicaux identiques ou différents choisis parmi des atomes d'halogène, des groupes alcoxy en $C_1$-$C_4$ et nitro;

c) $R^2$ représente

1. un atome d'hydrogène,
2. un atome d'halogène,
3. le groupe nitro,
4. $C_vF_{2v+1}$,
5. le groupe pentafluorophényle,
6. le groupe cyano,
7. le groupe phényle,
8. un groupe phényl-alkyle($C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_n$-,
14. un groupe tétrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,
25.

$$-(CH_2)_o-O-\underset{\underset{W}{\|}}{C}-NH-R^9,$$

26.

$$-(CH_2)_o-NR^7-\underset{\underset{W}{\|}}{C}-OR^9,$$

27. $-(CH_2)_o-NR^7-CO-NHR^9$,
28. $-(CH_2)_o-NR^7-SO_2R^9$,
29.

$$-(CH_2)_o-NR^7-\underset{\underset{W}{\|}}{C}-R^9,$$

30. $-(CH_2)_nF$,

31. $-(CH_2)_n-O-NO_2$,
32. $-CH_2-N_3$,
33. $-(CH_2)_n-NO_2$,
34. $-CH=N-NR^5R^7$,
35. un groupe phtalimido-$(CH_2)_n$-,
36.

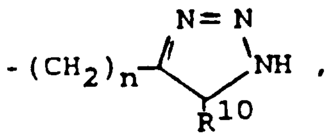

37.

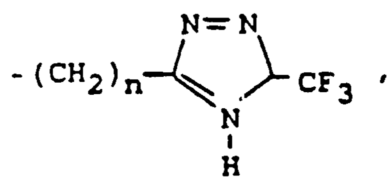

38.

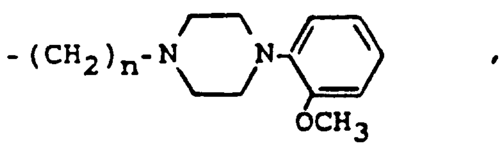

39.

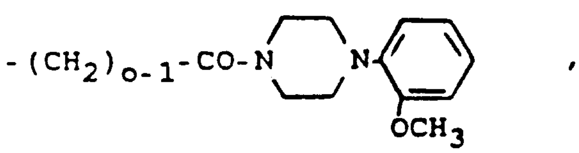

40. un groupe phényl-$SO_2$-NH-N=CH-,
41.

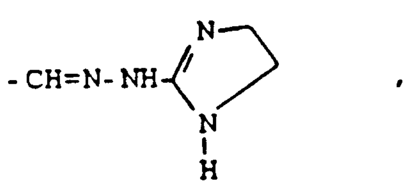

42. $-(CH_2)_n-SO_2-NR^7-CO-NR^6R^9$,
43. $-(CH_2)_o-SO_2R^9$,
44. un radical tel que défini en c) 7. ou 8., qui est substitué sur le fragment phényle par 1 ou 2 atomes ou radicaux identiques ou différents, choisis parmi des atomes d'halogène et des groupes hydroxy, méthoxy, trifluorométhyle, $CO_2R^3$ et phényle,
45. un radical tel que défini en c) 9., 10. ou 18., dans lequel de 1 à l'ensemble des atomes d'hydrogène sont remplacés par des atomes de fluor, ou
46. le radical défini en c) 13., qui est substitué par un ou deux radicaux identiques ou différents choisis parmi des groupes méthoxycarbonyle et alkyle en $C_1$-$C_4$;

d) $R^3$ représente
1. un atome d'hydrogène,
2. un groupe alkyle en $C_1$-$C_8$,
3. un groupe cycloalkyle en $C_3$-$C_8$,
4. le groupe phényle,
5. le groupe benzyle ou
6. le radical défini en d) 2., dans lequel de 1 à l'ensemble des atomes d'hydrogène sont remplacés par des atomes de fluor;

e) $R^4$ représente
1. un atome d'hydrogène,
2. un groupe alkyle en $C_1$-$C_6$,
3. un groupe cycloalkyle en $C_3$-$C_8$,
4. un groupe alcényle en $C_2$-$C_4$ ou
5. un groupe alcynyle en $C_2$-$C_4$;

f) $R^5$ représente
1. un atome d'hydrogène,
2. un groupe alkyle en $C_1$-$C_6$,
3. un groupe cycloalkyle en $C_3$-$C_8$,
4. le groupe phényle ou
5. le groupe benzyle;

g) $R^6$ représente
1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. le groupe benzyle,
6. un groupe hétéroaryle en $C_1$-$C_9$, qui peut être partiellement ou totalement hydrogéné et qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux de la position (ou point) de condensation de radicaux bicycliques (comme dans le radical indolizinyle) pouvant également être un(des) atome(s) d'azote,
7. un groupe alcanoyle en $C_1$-$C_4$,
8. un radical tel que défini en g) 4. ou 6., substitué par 1 ou 2 atomes ou radicaux choisis parmi des atomes d'halogène et des groupes hydroxy, méthoxy, nitro, cyano, $CO_2R^3$, trifluorométhyle, $NR^{11}R^{12}$ ou

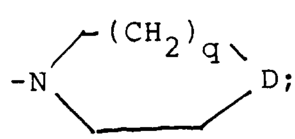

9. un groupe hétéroaryl($C_1$-$C_9$)-alkyle($C_1$-$C_3$), le fragment hétéroaryle étant tel que défini en g) 6.;

h) $R^7$ représente
1. un atome d'hydrogène,
2. un groupe alkyle en $C_1$-$C_6$,
3. un groupe cycloalkyle en $C_3$-$C_8$,
4. un groupe aryl($C_6$-$C_{12}$)-alkyle($C_1$-$C_6$),
5. le groupe phényle ou
6. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote;

i) $R^8$ représente
1. un atome d'hydrogène,
2. un groupe alkyle en $C_1$-$C_6$,

3. un groupe cycloalkyle en $C_3$-$C_8$,
4. un groupe phényl-$(CH_2)_q$-,
5. $OR^5$,
6. $NR^{11}R^{12}$ ou
7.

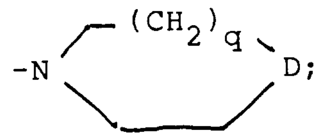

j) $R^9$ représente
1. un groupe alkyle en $C_1$-$C_6$,
2. le groupe 1-adamantyle,
3. le groupe 1-naphtyle,
4. le groupe 1-naphtyléthyle,
5. un groupe phényl-$(CH_2)_q$- ou
6. le radical défini en j) 1., dans lequel de 1 à l'ensemble des atomes d'hydrogène sont remplacés par des atomes de fluor;

k) $R^{10}$ représente un groupe cyano, nitro ou $CO_2R^7$;

l) $R^{11}$ et $R^{12}$ sont identiques ou différents et représentent
1. un atome d'hydrogène,
2. un groupe alkyle en $C_1$-$C_4$,
3. le groupe phényle,
4. le groupe benzyle ou
5. le groupe $\alpha$-méthylbenzyle;

m) D représente $NR^{13}$, O ou $CH_2$;

n) $R^{13}$ représente un atome d'hydrogène ou un groupe alkyle en $C_1$-$C_4$ ou phényle;

o) A représente le reste d'un hétérocycle bicyclique condensé ayant de 8 à 10 atomes formant les cycles, dont jusqu'à 9 atomes formant les cycles sont des atomes de carbone, dans lequel deux atomes adjacents sont des composants communs des deux cycles, un de ces cycles ou les deux dérivant, de par la formule, du benzène, dans lequel un ou plusieurs groupes CH sont remplacés par N, $O^+$ et $S^+$ et/ou dans lequel deux groupes CH contigus (avec formation d'un noyau aromatique à cinq chaînons) sont remplacés par S, NH ou O; qui peut être substitué par jusqu'à six radicaux $R^{14}$ ou $-(CH_2)_{n-1}-(CHR^6-CH_2)_{o-1}-R^{15}$, identiques ou différents;

p) $R^{14}$ représente
1. un atome d'halogène,
2. le groupe oxo,
3. le groupe nitroso,
4. le groupe nitro,
5. le groupe amino,
6. le groupe cyano,
7. le groupe hydroxy,
8. un groupe alkyle en $C_1$-$C_6$,
9. un groupe alcanoyle en $C_1$-$C_4$,
10. un groupe alcanoyloxy en $C_1$-$C_4$,
11. $CO_2R^3$,
12. le groupe méthanesulfonylamino,
13. le groupe trifluorométhanesulfonylamino,
14. $-CO-NH-OR^9$,
15. $-SO_2-NR^6R^7$,
16. $-CH_2-OR^7$,
17. un groupe hétéroaryl($C_1$-$C_9$)-$(CH_2)_q$-, le fragment hétéroaryle étant tel que défini en g) 6.;
18. un groupe aroyle en $C_7$-$C_{13}$,

19.

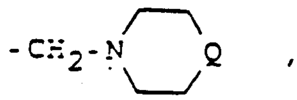

20.

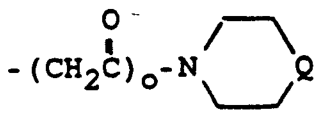

ou

21. un groupe aryle en $C_6$-$C_{12}$;

q) $R^{15}$ représente

1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. un groupe aroyle en $C_7$-$C_{13}$,
6. un groupe alcoxy en $C_1$-$C_4$,
7. un groupe alcanoyloxy en $C_1$-$C_4$,
8. un groupe hétéroaryle en $C_1$-$C_9$ qui est tel que défini en h) 6.,
9. $CO_2R^3$,
10. un atome d'halogène,
11. le groupe cyano,
12. le groupe nitro,
13. $NR^6R^7$,
14. le groupe hydroxy,
15. $-CO-NH-CHR^5-CO_2R^3$,
16. le groupe sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. le groupe phosphono-oxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. le radical défini en p) 20.,
29.

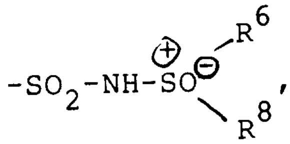

30.

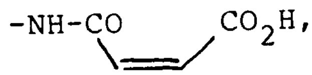

31.

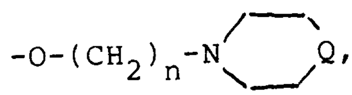

32. le groupe 5-tétrazolyl-NH-CO,

33. $-CO-NH-NH-SO_2CF_3$,

34.

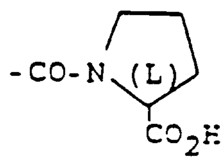

,

35.

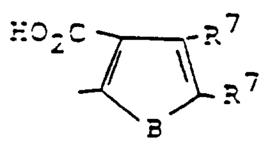

,

36.

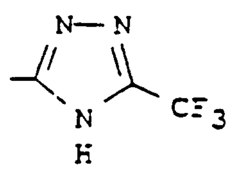

,

37.

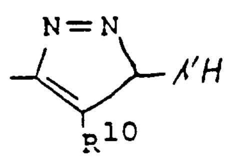

,

38.

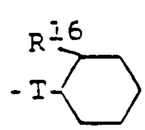

,

39.

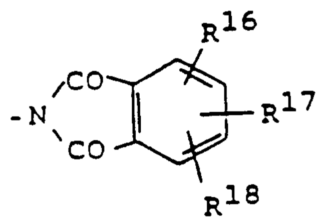

40. $-CO-NH-SO_2-R^{19}$

41. le radical défini en q) 4., substitué par 1 ou 2 atomes ou radicaux identiques ou différents choisis parmi des atomes d'halogène et des groupes cyano, nitro, $NR^6R^7$ et hydroxy;

r) B représente O, $NR^7$ ou S;

s) W représente O ou S;

t) L représente un groupe alcanediyle en $C_1$-$C_3$;

u) $R^{16}$ représente $CO_2R^3$ ou $CH_2CO_2R^3$;

v) $R^{17}$ représente un atome d'hydrogène ou d'halogène ou un groupe alkyle en $C_1$-$C_4$ ou alcoxy en $C_1$-$C_4$;

w) $R^{18}$ représente un atome d'hydrogène ou un groupe alkyle en $C_1$-$C_4$ ou phényle;

x) $R^{19}$ représente

1. un groupe alkyle en $C_1$-$C_8$,
2. un groupe cycloalkyle en $C_3$-$C_8$,
3. le groupe phényle,
4. le groupe benzyle ou
5. le radical défini en x) 1., dans lequel de 1 à l'ensemble des atomes d'hydrogène sont remplacés par des atomes de fluor ou de chlore;

y) T représente

1. une simple liaison,
2. -CO-,
3. $-CH_2-$,
4. -O-,
5. -S-,
6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $-SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,

25.

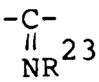

ou

26.

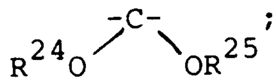

z) $R^{20}$ et $R^{22}$ sont identiques ou différents et représentent un atome d'hydrogène ou un groupe alkyle en $C_1$-$C_5$, phényle, allyle ou benzyle;

a') $R^{21}$ représente un atome d'hydrogène ou un groupe alkyle en $C_1$-$C_6$, benzyle ou allyle,

b') $R^{23}$ représente 1. $NR^{20}R^{21}$, 3. le groupe uréido, 3. le groupe thiouréido, 4. le groupe toluène-4-sulfonyle ou 5. le groupe benzènesulfonylamino;

c') $R^{24}$ et $R^{25}$ sont identiques ou différents et représentent un groupe alkyle en $C_1$-$C_4$, ou forment ensemble un groupe $-(CH_2)_q-$;

d') Q représente $CH_2$, NH, O ou S;

e') m est un nombre entier allant de 0 à 5;

f') n est un nombre entier allant de 1 à 5;

g') o est un nombre entier allant de 1 à 10;

h') q est 0 ou 1;

i') r est 0, 1 ou 2; ou

j') v est un nombre entier allant de 1 à 6;

caractérisé en ce que l'on fait réagir un composé de formule II

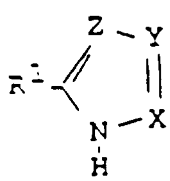

(II)

dans laquelle $R^1$, X, Y et Z sont tels que définis plus haut, avec un composé de formule III

$U-L-(O)_q-A$ (III)

dans laquelle L, A et q sont tels que définis plus haut, et U représente un groupe partant, éventuellement on élimine des groupes protecteurs temporairement introduits, et éventuellement on convertit les composés de formule I obtenus en leurs sels physiologiquement acceptables.

**2.** Procédé selon la revendication 1, dans lequel, dans la formule I,

a) $R^1$ représente

1. un groupe alkyle en $C_3$-$C_{10}$,
2. un groupe alcényle en $C_3$-$C_{10}$,
3. un groupe alcynyle en $C_3$-$C_{10}$,
4. un groupe cycloalkyle en $C_3$-$C_8$,
5. le groupe benzyle ou
6. un groupe benzyle qui est substitué comme défini dans la revendication 1;

b) $R^2$ représente

1. un atome d'hydrogène,
2. un atome d'halogène,
3. le groupe nitro,

4. $C_vF_{2v+1}$,
5. le groupe pentafluorophényle,
6. le groupe cyano,
7. le groupe phényle,
8. un groupe phényl-alkyle-$C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_o$-,
14. un groupe tétrazolyl-$(CH_2)_m$-,
15. -$(CH_2)_{o-1}$-$CHR^7$-$OR^5$,
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ ou
27. un radical défini comme en b) 7., 8., 9., 10. ou 13., qui est substitué comme décrit pour un tel radical dans la revendication 1 en c) 46., 45. ou 44.;

c) $R^8$ représente un atome d'hydrogène ou un groupe alkyle en $C_1$-$C_5$, $OR^5$, $NR^{11}R^{12}$ ou morpholino;

d) T représente
1. une simple liaison,
2. -CO-,
3. -$CONR^{21}$,
4. -$CH_2$-$CH_2$-,
5. -$NR^{21}$-CO-,
6. -O-$CH_2$-,
7. -$CH_2$-O-,
8. -S-$CH_2$-,
9. -$CH_2$-S-,
10. -NH-$CH_2$-,
11. -$CH_2$-NH- ou
12. -CH = CH-

et les autres radicaux et variables sont tels que définis dans la revendication 1, ainsi que pour la préparation des sels physiologiquement acceptables d'un tel composé.

**3.** Procédé selon la revendication 1 ou 2, caractérisé en ce que les symboles dans la formule I ont les significations suivantes:

a) $R^1$ représente un groupe alkyle en $C_3$-$C_7$, alcényle en $C_3$-$C_7$ ou alcynyle en $C_3$-$C_7$;

b) $R^2$ représente
1. un atome de chlore,
2. un atome de brome,
3. $C_vF_{2v+1}$, avec v = 1, 2 ou 3,
4. le groupe pentafluorophényle,
5. -$S(O)_rR^6$,
6. $(CH_2)_{o-1}$-$CHR^7$-$OR^5$,
7. $(CH_2)_o$-O-CO-$R^3$,
8. -$COR^8$,
9. -$(CH_2)_o$-CO-$R^8$,
10. -$CH_2$-NH-CO-$R^8$,
11. -$(CH_2)_o$-NH-$SO_2$-$R^9$,
12. -CH = CH-$CHR^3$-$OR^6$,

13. un groupe tétrazolyl-$(CH_2)_m$-,
14. -$(CH_2)_nSO_2$-NH-CO-$NR^6R^9$,
15. -$(CH_2)_o$-$SO_3R^9$ ou un radical alkyle en $C_1$-$C_6$ éventuellement substitué par le groupe hydroxy;

c) $R^3$ représente un atome d'hydrogène ou un groupe alkyle en $C_1$-$C_4$;

d) $R^6$ représente un atome d'hydrogène ou un groupe alkyle en $C_1$-$C_4$, alcanoyle en $C_1$-$C_4$, un radical tel que défini plus haut dans la revendication 1 en g) 4., 6. ou 9., substitué par 1 ou 2 atomes ou radicaux identiques ou différents, choisis parmi des atomes d'halogène et des groupes hydroxy, méthoxy, nitro, cyano, $CO_2R^3$ et trifluorométhyle, ou un radical hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,

e) $R^7$ représente un atome d'hydrogène ou un groupe alkyle en $C_1$-$C_4$, hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote, ou aryl($C_6$-$C_{12}$)-alkyle($C_1$-$C_4$);

f) $R^8$ représente un atome d'hydrogène ou un groupe alkyle en $C_1$-$C_4$, $OR^5$ ou morpholino;

g) $R^9$ représente $CF_3$, un groupe alkyle en $C_1$-$C_6$ ou phényle;

h) $R^{14}$ représente

1. un groupe alkyle en $C_1$-$C_4$,
2. un groupe alcoxy en $C_1$-$C_4$,
3. le groupe cyano,
4. le groupe amino,
5. le groupe nitroso,
6. le groupe nitro,
7. un atome de fluor,
8. un atome de chlore,
9. un atome de brome,
10. le groupe hydroxy,
11. $CH_2OR^7$,
12. un groupe hétéroaryl($C_1$-$C_9$)-$CH_2$-, le fragment hétéroaryle dérivant du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,
13. un groupe alcanoyloxy en $C_1$-$C_4$,
14. un groupe alcanoyle en $C_1$-$C_4$,
15. le groupe benzoyle,
16.

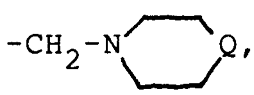

17. -NH-CO-$R^7$ ou
18. le groupe tétrazolyle;

i) $R^{15}$ représente

1. un groupe alkyle en $C_1$-$C_4$,
2. un groupe aryle en $C_6$-$C_{12}$,
3. un groupe alcanoyloxy en $C_1$-$C_3$,
4. un groupe alcoxy en $C_1$-$C_4$,
5. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou

O, un atome ou les deux du point de condensation de radicaux bicycliques (comme dans le radical indolizinyle) pouvant également être un(des) atome(s) d'azote,

6. le groupe cyano,
7. le groupe nitro,
8. le groupe hydroxy,
9. $-S(O)_rR^6$,
10. $-SO_3R^3$,
11. un atome de chlore,
12. un atome de brome,
13. le groupe benzoyle,
14. $-CO_2R^3$,
15. $-CO-NH-R^6$,
16. $-NR^6R^7$,
17. $-CO-R^8$,
18. $-SO_2-NR^6R^7$,
19.

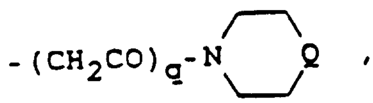

20.

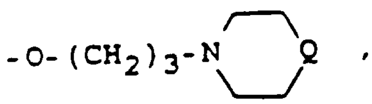

21. $-SO_2-NH-CO-NR^6R^9$,
22. $-PO_3H$,
23. $-CO-CHR^5-CO_2H$,
24. $-NH-CO-SO_2-CH_2-R^5$,
25. le groupe 5-tétrazolyl-NH-CO-,
26.

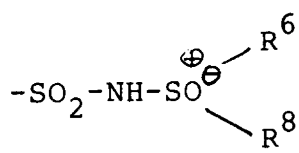

27.

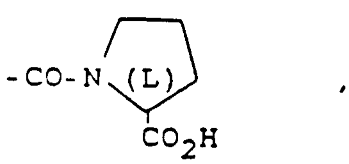

28.

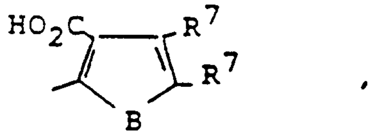

29.

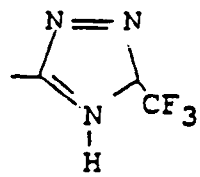 ,

30.

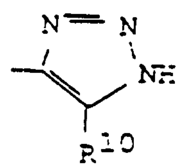 ,

31.

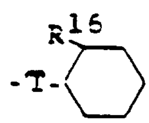 ,

32.

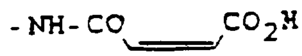 ,

33.

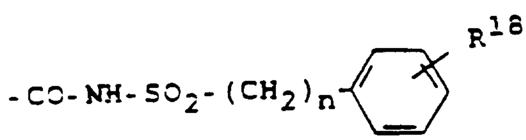

ou

34. le radical défini en i) 2., substitué comme défini dans la revendication 1;

j) Q représente $CH_2$, NH ou O;

k) $R^{18}$ représente un atome d'hydrogène ou le groupe méthyle ou éthyle;

l) T représente une simple liaison, -O-, -CO-, -NHCO- ou $-OCH_2-$

et les autres radicaux et variables sont tels que définis dans la revendication 1.

**4.** Procédé selon l'une des revendications 1 à 3, dans lequel, dans la formule (I), les symboles ont les significations suivantes:

a) X représente N, Y représente $CR^2$ et Z représente $CR^2$;

b) X représente $CR^2$, Y représente N et Z représente $CR^2$;

c) X représente $CR^2$, Y représente $CR^2$ et Z représente N; ou

d) X, Y et Z représentent chacun N.

**5.** Procédé selon l'une des revendications 1 à 4, dans lequel

a) $R^2$ représente un atome de chlore ou de brome, ou un groupe $-S(O)_rR^6$, $-COR^8$ ou $-(CH_2)_nSO_2-NH-CO-NR^6R^9$;
b) $R^9$ représente un groupe alkyle en $C_1-C_6$;
c) $R^{14}$ représente le groupe tétrazolyle;
d) $R^{15}$ représente un groupe $-CO_2-R^3$, $-SO_2-NR^6R^7$, $-SO_2-NH-CO-NR^6R^9$ ou $-NH-CO-NH-SO_2-CH_2-R^5$;
e) Z est N ;
f) X et Y représentent l'un et l'autre $CR_2$;
g) q est zéro;
h) L représente $CH_2$;
i) A représente un reste de benzothiophène, benzofuranne, indole, isoindole, indazole, benzimidazole, quinoléine, isoquinoléine, phtalazine, quinoxaline, quinazoline, cinnoline, benzothiazole, benzothiazol-1,1-dioxyde, coumarine, chromane, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thiénopyridine ou pyrrolopyrimidine, qui peut être substitué par jusqu'à 6 radicaux $R^{14}$ ou $-(CH_2)_{n-1}-(CHR^6-CH_2)_{o-1}-R^{15}$, identiques ou différents,

et les autres radicaux et variables sont tels que définis dans la revendication 3.

**6.** Procédé pour la fabrication d'une composition pharmaceutique, caractérisé en ce que l'on met sous une présentation appropriée un composé préparé selon l'une des revendications 1 à 5, conjointement avec un véhicule physiologiquement acceptable et éventuellement d'autres additifs ou adjuvants.

**7.** Procédé pour la préparation d'un composé de formule (II')

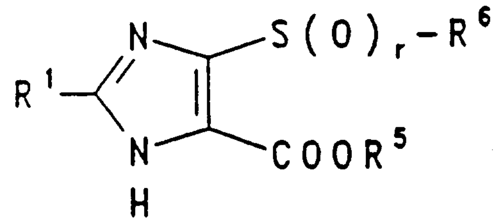

(II')

dans laquelle
a) $R^1$ représente le groupe n-butyle,
b) $R^6$ représente le groupe méthyle,
c) $R^5$ représente un atome d'hydrogène ou le groupe éthyle et
d) r est 0, 1 ou 2,

caractérisé en ce que l'on convertit en un composé de formule (II') un composé de formule (III)

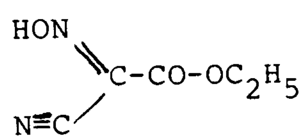

(III)

par, successivement,
- réduction en l'aminonitrile,
- introduction d'un groupe alkyle par acylation,
- addition d'un groupe alkylmercaptan sur le groupe cyano et cyclisation, et éventuellement oxydation supplémentaire du groupe thioalkyle et/ou hydrolyse du groupe ester.

**8.** Procédé pour la préparation d'un composé de formule (II') selon la revendication 7, dans lequel r est égal à 0 et $R^5$ représente le groupe éthyle.

**Revendications pour l'Etat contractant suivant : GR**

**1.** Procédé pour la préparation d'un composé de formule I

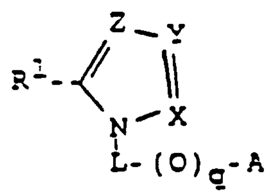

(I)

dans laquelle

a) X, Y et Z sont identiques ou différents et représentent N ou $CR^2$,

b) $R^1$ représente

1. un groupe alkyle en $C_2$-$C_{10}$,
2. un groupe alcényle en $C_3$-$C_{10}$,
3. un groupe alcynyle en $C_3$-$C_{10}$,
4. $OR^3$,
5. un groupe cycloalkyle en $C_3$-$C_8$,
6. un groupe cycloalkylalkyle en $C_4$-$C_{10}$,
7. un groupe cycloalkylalcényle en $C_5$-$C_{10}$,
8. un groupe cycloalkylalcynyle en $C_5$-$C_{10}$,
9. $-(CH_2)_m-B-(CH_2)_n-R^4$,
10. le groupe benzyle,
11. un radical tel que défini en b) 1., 2., 3. ou 9., qui est monosubstitué par $CO_2R^3$,
12. un radical tel que défini en b) 1., 2., 3. ou 9., dans lequel de 1 à l'ensemble des atomes d'hydrogène sont remplacés par des atomes de fluor, ou
13. le radical défini en b) 10. qui est substitué sur le fragment phényle par 1 ou 2 atomes ou radicaux identiques ou différents choisis parmi des atomes d'halogène, des groupes alcoxy en $C_1$-$C_4$ et nitro;

c) $R^2$ représente

1. un atome d'hydrogène,
2. un atome d'halogène,
3. le groupe nitro,
4. $C_vF_{2v+1}$,
5. le groupe pentafluorophényle,
6. le groupe cyano,
7. le groupe phényle,
8. un groupe phényl-alkyle($C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_n$-,
14. un groupe tétrazolyl-$(CH_2)_m$-,
15. $-(CH_2)_{o-1}-CHR^7-OR^5$,
16. $-(CH_2)_o-O-CO-R^3$,
17. $-(CH_2)_o-S-R^6$,
18. $-S(O)_r-R^6$,
19. $-CH=CH-(CH_2)_m-CHR^3-OR^6$,
20. $-CH_2=CH-(CH_2)_m-CO-R^8$,
21. $-CO-R^8$,
22. $-CH=CH-(CH_2)_m-O-CO-R^7$,
23. $-(CH_2)_m-CH(CH_3)-CO-R^8$,
24. $-(CH_2)_o-CO-R^8$,

25.

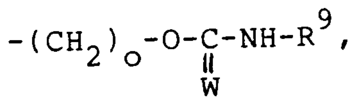

26.

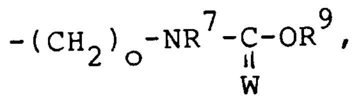

27. $-(CH_2)_o-NR^7-CO-NHR^9$,

28. $-(CH_2)_o-NR^7-SO_2R^9$,

29.

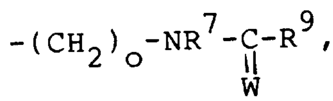

30. $-(CH_2)_nF$,

31. $-(CH_2)_n-O-NO_2$,

32. $-CH_2-N_3$,

33. $-(CH_2)_n-NO_2$,

34. $-CH=N-NR^5R^7$,

35. un groupe phtalimido-$(CH_2)_n$-,

36.

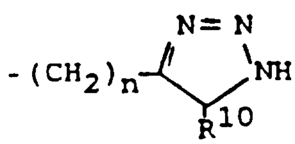
,

37.

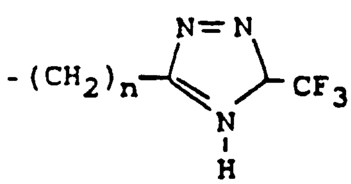
,

38.

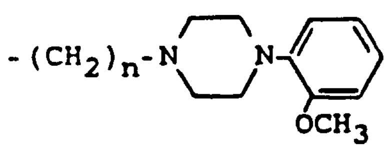
,

39.

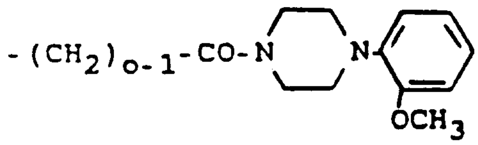

,

40. un groupe phényl-$SO_2$-NH-N = CH-,

41.

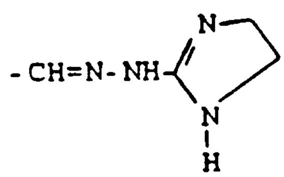

,

42. -$(CH_2)_n$-$SO_2$-$NR^7$-CO-$NR^6R^9$,

43. -$(CH_2)_o$-$SO_2R^9$,

44. un radical tel que défini en c) 7. ou 8., qui est substitué sur le fragment phényle par 1 ou 2 atomes ou radicaux identiques ou différents, choisis parmi des atomes d'halogène et des groupes hydroxy, méthoxy, trifluorométhyle, $CO_2R^3$ et phényle,

45. un radical tel que défini en c) 9., 10. ou 18., dans lequel de 1 à l'ensemble des atomes d'hydrogène sont remplacés par des atomes de fluor, ou

46. le radical défini en c) 13., qui est substitué par un ou deux radicaux identiques ou différents choisis parmi des groupes méthoxycarbonyle et alkyle en $C_1$-$C_4$;

d) $R^3$ représente

1. un atome d'hydrogène,
2. un groupe alkyle en $C_1$-$C_8$,
3. un groupe cycloalkyle en $C_3$-$C_8$,
4. le groupe phényle,
5. le groupe benzyle ou
6. le radical défini en d) 2., dans lequel de 1 à l'ensemble des atomes d'hydrogène sont remplacés par des atomes de fluor;

e) $R^4$ représente

1. un atome d'hydrogène,
2. un groupe alkyle en $C_1$-$C_6$,
3. un groupe cycloalkyle en $C_3$-$C_8$,
4. un groupe alcényle en $C_2$-$C_4$ ou
5. un groupe alcynyle en $C_2$-$C_4$;

f) $R^5$ représente

1. un atome d'hydrogène,
2. un groupe alkyle en $C_1$-$C_6$,
3. un groupe cycloalkyle en $C_3$-$C_8$,
4. le groupe phényle ou
5. le groupe benzyle;

g) $R^6$ représente

1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. le groupe benzyle,
6. un groupe hétéroaryle en $C_1$-$C_9$, qui peut être partiellement ou totalement hydrogéné et qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux de la position

(ou du point) de condensation de radicaux bicycliques (comme dans le radical indolizinyle) pouvant également être un(des) atome(s) d'azote,

7. un groupe alcanoyle en $C_1$-$C_4$,

8. un radical tel que défini en g) 4. ou 6., substitué par 1 ou 2 atomes ou radicaux choisis parmi des atomes d'halogène et des groupes hydroxy, méthoxy, nitro, cyano, $CO_2R^3$, trifluorométhyle, $NR^{11}R^{12}$ ou

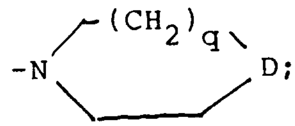

9. un groupe hétéroaryl($C_1$-$C_9$)-alkyle($C_1$-$C_3$), le fragment hétéroaryle étant tel que défini en g) 6.;

h) $R^7$ représente

1. un atome d'hydrogène,
2. un groupe alkyle en $C_1$-$C_6$,
3. un groupe cycloalkyle en $C_3$-$C_8$,
4. un groupe aryl($C_6$-$C_{12}$)-alkyle($C_1$-$C_6$),
5. le groupe phényle ou
6. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote;

i) $R^8$ représente

1. un atome d'hydrogène,
2. un groupe alkyle en $C_1$-$C_6$,
3. un groupe cycloalkyle en $C_3$-$C_8$,
4. un groupe phényl-$(CH_2)_q$-,
5. $OR^5$,
6. $NR^{11}R^{12}$ ou
7.

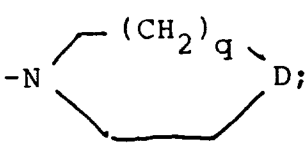

j) $R^9$ représente

1. un groupe alkyle en $C_1$-$C_6$,
2. le groupe 1-adamantyle,
3. le groupe 1-naphtyle,
4. le groupe 1-naphtyléthyle,
5. un groupe phényl-$(CH_2)_q$- ou
6. le radical défini en j) 1., dans lequel de 1 à l'ensemble des atomes d'hydrogène sont remplacés par des atomes de fluor;

k) $R^{10}$ représente un groupe cyano, nitro ou $CO_2R^7$;

l) $R^{11}$ et $R^{12}$ sont identiques ou différents et représentent

1. un atome d'hydrogène,
2. un groupe alkyle en $C_1$-$C_4$,
3. le groupe phényle,
4. le groupe benzyle ou
5. le groupe $\alpha$-méthylbenzyle;

m) D représente $NR^{13}$, O ou $CH_2$;

n) $R^{13}$ représente un atome d'hydrogène ou un groupe alkyle en $C_1$-$C_4$ ou phényle;

o) A représente le reste d'un hétérocycle bicyclique condensé ayant de 8 à 10 atomes formant les cycles, dont jusqu'à 9 atomes formant les cycles sont des atomes de carbone, dans lequel deux atomes adjacents sont des composants communs des deux cycles, un de ces cycles ou les deux dérivant, de par la formule, du benzène, dans lequel un ou plusieurs groupes CH sont remplacés par N, $O^+$ et $S^+$ et/ou dans lequel deux groupes CH contigus (avec formation d'un noyau aromatique à cinq chaînons) sont remplacés par S, NH ou O; qui peut être substitué par jusqu'à six radicaux $R^{14}$ ou $-(CH_2)_{n-1}-(CHR^6-CH_2)_{o-1}-R^{15}$, identiques ou différents;

p) $R^{14}$ représente
1. un atome d'halogène,
2. le groupe oxo,
3. le groupe nitroso,
4. le groupe nitro,
5. le groupe amino,
6. le groupe cyano,
7. le groupe hydroxy,
8. un groupe alkyle en $C_1$-$C_6$,
9. un groupe alcanoyle en $C_1$-$C_4$,
10. un groupe alcanoyloxy en $C_1$-$C_4$,
11. $CO_2R^3$,
12. le groupe méthanesulfonylamino,
13. le groupe trifluorométhanesulfonylamino,
14. $-CO-NH-OR^9$,
15. $-SO_2-NR^6R^7$,
16. $-CH_2-OR^7$,
17. un groupe hétéroaryl($C_1$-$C_9$)-$(CH_2)_q$-, le fragment hétéroaryle étant tel que défini en g) 6.;
18. un groupe aroyle en $C_7$-$C_{13}$,
19.

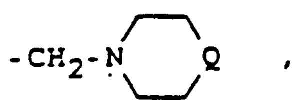

20.

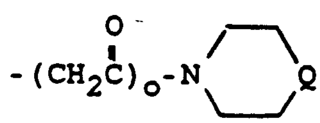

ou
21. un groupe aryle en $C_6$-$C_{12}$;

q) $R^{15}$ représente
1. un atome d'hydrogène,
2. un groupe alkyle en $C_1$-$C_6$,
3. un groupe cycloalkyle en $C_3$-$C_8$,
4. un groupe aryle en $C_6$-$C_{12}$,
5. un groupe aroyle en $C_7$-$C_{13}$,
6. un groupe alcoxy en $C_1$-$C_4$,
7. un groupe alcanoyloxy en $C_1$-$C_4$,
8. un groupe hétéroaryle en $C_1$-$C_9$ qui est tel que défini en h) 6.,
9. $CO_2R^3$,
10. un atome d'halogène,
11. le groupe cyano,
12. le groupe nitro,
13. $NR^6R^7$,
14. le groupe hydroxy,

15. $-CO-NH-CHR^5-CO_2R^3$,
16. le groupe sulfo,
17. $-SO_3R^3$,
18. $-SO_2-NR^7-CO-NR^6R^9$,
19. $-NR^7-CO-NR^6-SO_2-CH_2-R^5$,
20. $-C(CF_3)_2OH$,
21. le groupe phosphono-oxy,
22. $-PO_3H_2$,
23. $-NH-PO(OH)_2$,
24. $-S(O)_rR^6$,
25. $-CO-R^8$,
26. $-CO-NR^6R^9$,
27. $-CR^{20}(OH)-PO(OH)_2$,
28. le radical défini en p) 20.,
29.

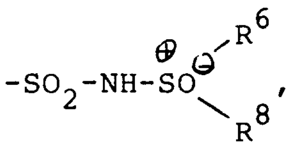

30.

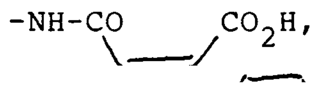

31.

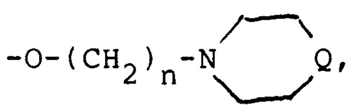

32. le groupe 5-tétrazolyl-NH-CO,
33. $-CO-NH-NH-SO_2CF_3$,
34.

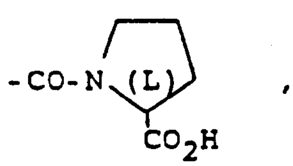

35.

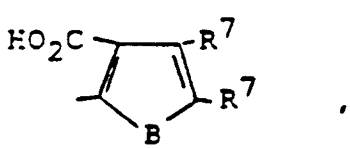

36.

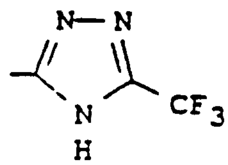 ,

37.

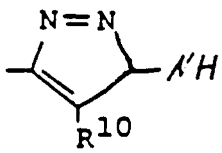 ,

38.

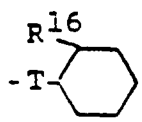 ,

39.

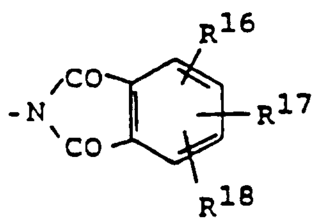 ,

40. $-CO-NH-SO_2-R^{19}$

41. le radical défini en q) 4., substitué par 1 ou 2 atomes ou radicaux identiques ou différents choisis parmi des atomes d'halogène et des groupes cyano, nitro, $NR^6R^7$ et hydroxy;

r) B représente O, $NR^7$ ou S;

s) W représente O ou S;

t) L représente un groupe alcanediyle en $C_1$-$C_3$;

u) $R^{16}$ représente $CO_2R^3$ ou $CH_2CO_2R^3$;

v) $R^{17}$ représente un atome d'hydrogène ou d'halogène ou un groupe alkyle en $C_1$-$C_4$ ou alcoxy en $C_1$-$C_4$;

w) $R^{18}$ représente un atome d'hydrogène ou un groupe alkyle en $C_1$-$C_4$ ou phényle;

x) $R^{19}$ représente

1. un groupe alkyle en $C_1$-$C_8$,
2. un groupe cycloalkyle en $C_3$-$C_8$,
3. le groupe phényle,
4. le groupe benzyle ou
5. le radical défini en x) 1., dans lequel de 1 à l'ensemble des atomes d'hydrogène sont remplacés par des atomes de fluor ou de chlore;

y) T représente

1. une simple liaison,
2. -CO-,
3. $-CH_2-$,
4. -O-,
5. -S-,

6. $-NR^{21}-$,
7. $-CO-NR^{21}-$,
8. $-NR^{21}-CO-$,
9. $-O-CH_2-$,
10. $-CH_2-O-$,
11. $-S-CH_2-$,
12. $-CH_2-S-$,
13. $-NH-CR^{20}R^{22}-$,
14. $-NR^{21}-SO_2-$,
15. $-SO_2-NR^{21}-$,
16. $-CR^{20}R^{22}-NH-$,
17. -CH = CH-,
18. -CF = CF-,
19. -CH = CF-,
20. -CF = CH-,
21. $-CH_2-CH_2-$,
22. $-CF_2-CF_2-$,
23. $-CH(OR^3)-$,
24. $-CH(OCOR^5)-$,
25.

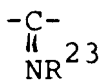

ou
26.

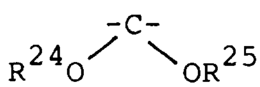
;

z) $R^{20}$ et $R^{22}$ sont identiques ou différents et représentent un atome d'hydrogène ou un groupe alkyle en $C_1$-$C_5$, phényle, allyle ou benzyle;
a') $R^{21}$ représente un atome d'hydrogène ou un groupe alkyle en $C_1$-$C_6$, benzyle ou allyle,
b') $R^{23}$ représente
1. $NR^{20}R^{21}$,
3. le groupe uréido,
3. le groupe thiouréido,
4. le groupe toluène-4-sulfonyle ou
5. le groupe benzènesulfonylamino;

c') $R^{24}$ et $R^{25}$ sont identiques ou différents et représentent un groupe alkyle en $C_1$-$C_4$, ou forment ensemble un groupe $-(CH_2)_q-$;
d') Q représente $CH_2$, NH, O ou S;
e') m est un nombre entier allant de 0 à 5;
f') n est un nombre entier allant de 1 à 5;
g') o est un nombre entier allant de 1 à 10;
h') q est 0 ou 1;
i') r est 0, 1 ou 2; ou
j') v est un nombre entier allant de 1 à 6;

caractérisé en ce que l'on fait réagir un composé de formule II

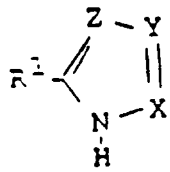

(II)

dans laquelle $R^1$, X, Y et Z sont tels que définis plus haut, avec un composé de formule III

U-L-$(O)_q$-A (III)

dans laquelle L, A et q sont tels que définis plus haut, et U représente un groupe partant, éventuellement on élimine des groupes protecteurs temporairement introduits, et éventuellement on convertit les composés de formule I obtenus en leurs sels physiologiquement acceptables.

**2.** Procédé selon la revendication 1, dans lequel, dans la formule I,

a) $R^1$ représente
1. un groupe alkyle en $C_3$-$C_{10}$,
2. un groupe alcényle en $C_3$-$C_{10}$,
3. un groupe alcynyle en $C_3$-$C_{10}$,
4. un groupe cycloalkyle en $C_3$-$C_8$,
5. le groupe benzyle ou
6. un groupe benzyle qui est substitué comme défini dans la revendication 1;

b) $R^2$ représente
1. un atome d'hydrogène,
2. un atome d'halogène,
3. le groupe nitro,
4. $C_vF_{2v+1}$,
5. le groupe pentafluorophényle,
6. le groupe cyano,
7. le groupe phényle,
8. un groupe phényl-alkyle($C_1$-$C_3$),
9. un groupe alkyle en $C_1$-$C_{10}$,
10. un groupe alcényle en $C_3$-$C_{10}$,
11. un groupe phényl-alcényle($C_2$-$C_6$),
12. un groupe 1-imidazolyl-$(CH_2)_m$-,
13. un groupe 1,2,3-triazolyl-$(CH_2)_o$-,
14. un groupe tétrazolyl-$(CH_2)_m$-,
15. -$(CH_2)_{o-1}$-$CHR^7$-$OR^5$,
16. -$(CH_2)_o$-O-$COR^3$,
17. -$COR^8$,
18. -$(CH_2)_o$-CO-$R^8$,
19. -$S(O)_rR^6$,
20. -CH = CH-$(CH_2)_m$-$CHR^3$-$OR^6$,
21. -$CH_2$ = CH-$(CH_2)_m$-CO-$R^8$,
22. -$(CH_2)_o$-NH-CO-$OR^9$,
23. -$(CH_2)_o$-NH-$SO_2$-$R^9$,
24. -$(CH_2)_nF$,
25. -$(CH_2)_o$-$SO_3R^9$,
26. -$(CH_2)_n$-$SO_2$-NH-CO-$NR^6R^9$ ou
27. un radical défini comme en b) 7., 8., 9., 10. ou 13., qui est substitué comme décrit pour un tel radical dans la revendication 1 en c) 46., 45. ou 44.;

c) $R^8$ représente un atome d'hydrogène ou un groupe alkyle en $C_1$-$C_5$, $OR^5$, $NR^{11}R^{12}$ ou morpholino;

d) T représente
1. une simple liaison,
2. -CO-,
3. -$CONR^{21}$,

4. $-CH_2-CH_2-$,
5. $-NR^{21}-CO-$,
6. $-O-CH_2-$,
7. $-CH_2-O-$,
8. $-S-CH_2-$,
9. $-CH_2-S-$,
10. $-NH-CH_2-$,
11. $-CH_2-NH-$ ou
12. $-CH=CH-$

et les autres radicaux et variables sont tels que définis dans la revendication 1, ainsi que pour la préparation des sels physiologiquement acceptables d'un tel composé.

**3.** Procédé selon la revendication 1 ou 2, caractérisé en ce que les symboles dans la formule I ont les significations suivantes:

a) $R^1$ représente un groupe alkyle en $C_3$-$C_7$, alcényle en $C_3$-$C_7$ ou alcynyle en $C_3$-$C_7$;

b) $R^2$ représente

1. un atome de chlore,
2. un atome de brome,
3. $C_vF_{2v+1}$, avec v = 1, 2 ou 3,
4. le groupe pentafluorophényle,
5. $-S(O)_rR^6$,
6. $(CH_2)_{o-1}-CHR^7-OR^5$,
7. $(CH_2)_o-O-CO-R^3$,
8. $-COR^8$,
9. $-(CH_2)_o-CO-R^8$,
10. $-CH_2-NH-CO-R^8$,
11. $-(CH_2)_o-NH-SO_2-R^9$,
12. $-CH=CH-CHR^3-OR^6$,
13. un groupe tétrazolyl-$(CH_2)_m$-,
14. $-(CH_2)_nSO_2-NH-CO-NR^6R^9$,
15. $-(CH_2)_o-SO_3R^9$ ou un radical alkyle en $C_1$-$C_6$ éventuellement substitué par le groupe hydroxy;

c) $R^3$ représente un atome d'hydrogène ou un groupe alkyle en $C_1$-$C_4$;

d) $R^6$ représente un atome d'hydrogène ou un groupe alkyle en $C_1$-$C_4$, alcanoyle en $C_1$-$C_4$, un radical tel que défini plus haut dans la revendication 1 en g) 4., 6. ou 9., substitué par 1 ou 2 atomes ou radicaux identiques ou différents, choisis parmi des atomes d'halogène et des groupes hydroxy, méthoxy, nitro, cyano, $CO_2R^3$ et trifluorométhyle, ou un radical hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,

e) $R^7$ représente un atome d'hydrogène ou un groupe alkyle en $C_1$-$C_4$, hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote, ou aryl($C_6$-$C_{12}$)-alkyle($C_1$-$C_4$);

f) $R^8$ représente un atome d'hydrogène ou un groupe alkyle en $C_1$-$C_4$, $OR^5$ ou morpholino;

g) $R^9$ représente $CF_3$, un groupe alkyle en $C_1$-$C_6$ ou phényle;

h) $R^{14}$ représente

1. un groupe alkyle en $C_1$-$C_4$,
2. un groupe alcoxy en $C_1$-$C_4$,
3. le groupe cyano,
4. le groupe amino,
5. le groupe nitroso,
6. le groupe nitro,
7. un atome de fluor,
8. un atome de chlore,

9. un atome de brome,
10. le groupe hydroxy,
11. $CH_2OR^7$,
12. un groupe hétéroaryl($C_1$-$C_9$)-$CH_2$-, le fragment hétéroaryle dérivant du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,
13. un groupe alcanoyloxy en $C_1$-$C_4$,
14. un groupe alcanoyle en $C_1$-$C_4$,
15. le groupe benzoyle,
16.

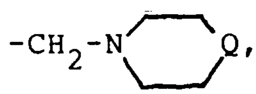

17. -NH-CO-$R^7$ ou
18. le groupe tétrazolyle;

i) $R^{15}$ représente

1. un groupe alkyle en $C_1$-$C_4$,
2. un groupe aryle en $C_6$-$C_{12}$,
3. un groupe alcanoyloxy en $C_1$-$C_3$,
4. un groupe alcoxy en $C_1$-$C_4$,
5. un groupe hétéroaryle en $C_1$-$C_9$ qui dérive du groupe phényle ou naphtyle, dans lequel un ou plusieurs groupes CH sont remplacés par N et/ou dans lequel au moins deux groupes CH contigus (avec formation d'un noyau aromatique à cinq chaînons) sont remplacés par S, NH ou O, un atome ou les deux du point de condensation de radicaux bicycliques (comme dans le radical indolizinyle) pouvant également être un(des) atome(s) d'azote,
6. le groupe cyano,
7. le groupe nitro,
8. le groupe hydroxy,
9. -$S(O)_rR^6$,
10. -$SO_3R^3$,
11. un atome de chlore,
12. un atome de brome,
13. le groupe benzoyle,
14. -$CO_2R^3$,
15. -CO-NH-$R^6$,
16. -$NR^6R^7$,
17. -CO-$R^8$,
18. -$SO_2$-$NR^6R^7$,
19.

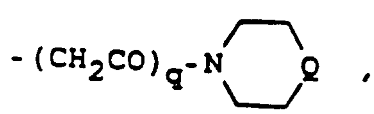

20.

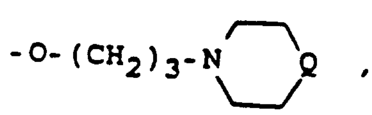

21. -$SO_2$-NH-CO-$NR^6R^9$,

22. $-PO_3H$,
23. $-CO-CHR^5-CO_2H$,
24. $-NH-CO-SO_2-CH_2-R^5$,
25. le groupe 5-tétrazolyl-NH-CO-,
26.

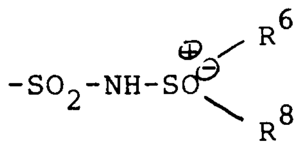

27.

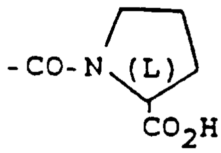

,

28.

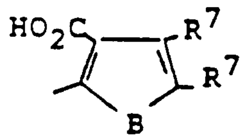

,

29.

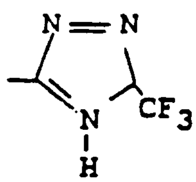

,

30.

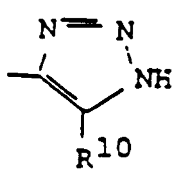

,

31.

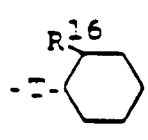

,

32.

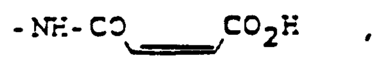

33.

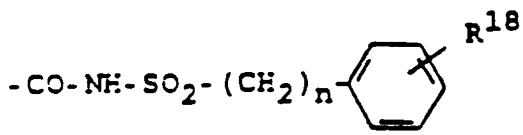

ou

34. le radical défini en i) 2., substitué comme défini dans la revendication 1;

j) Q représente $CH_2$, NH ou O;

k) $R^{18}$ représente un atome d'hydrogène ou le groupe méthyle ou éthyle;

l) T représente une simple liaison, -O-, -CO-, -NHCO- ou $-OCH_2-$

et les autres radicaux et variables sont tels que définis dans la revendication 1.

**4.** Procédé selon l'une des revendications 1 à 3, dans lequel, dans la formule (I), les symboles ont les significations suivantes:

a) X représente N, Y représente $CR^2$ et Z représente $CR^2$;

b) X représente $CR^2$, Y représente N et Z représente $CR^2$;

c) X représente $CR^2$, Y représente $CR^2$ et Z représente N; ou

d) X, Y et Z représentent chacun N.

**5.** Procédé selon l'une des revendications 1 à 4, dans lequel

a) $R^2$ représente un atome de chlore ou de brome, ou un groupe $-S(O)_rR^6$, $-COR^8$ ou $-(CH_2)_nSO_2-NH-CO-NR^6R^9$;

b) $R^9$ représente un groupe alkyle en $C_1-C_6$;

c) $R^{14}$ représente le groupe tétrazolyle;

d) $R^{15}$ représente un groupe $-CO_2-R^3$, $-SO_2-NR^6R^7$, $-SO_2-NH-CO-NR^6R^9$ ou $-NH-CO-NH-SO_2-CH_2-R^5$;

e) Z est N ;

f) X et Y représentent l'un et l'autre $CR_2$;

g) q est zéro;

h) L représente $CH_2$;

i) A représente un reste de benzothiophène, benzofuranne, indole, isoindole, indazole, benzimidazole, quinoléine, isoquinoléine, phtalazine, quinoxaline, quinazoline, cinnoline, benzothiazole, benzothiazol-1,1-dioxyde, coumarine, chromane, benzoxazole, benzisothiazole, benzodiazines, benzotriazole, benzotriazine, benzoxazine, imidazopyridine, imidazopyrimidine, imidazopyrazine, imidazopyridazine, imidazothiazole, pyrazolopyridine, thiénopyridine ou pyrrolopyrimidine, qui peut être substitué par jusqu'à 6 radicaux $R^{14}$ ou $-(CH_2)_{n-1}-(CHR^6-CH_2)_{o-1}-R^{15}$, identiques ou différents,

et les autres radicaux et variables sont tels que définis dans la revendication 3.

**6.** Procédé pour la fabrication d'une composition pharmaceutique, caractérisé en ce que l'on met sous une présentation appropriée un composé préparé selon l'une des revendications 1 à 5, conjointement avec un véhicule physiologiquement acceptable et éventuellement d'autres additifs ou adjuvants.

**7.** Procédé pour la préparation d'un composé de formule (II')

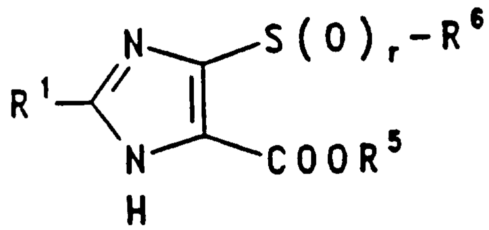

(II')

dans laquelle

a) $R^1$ représente le groupe n-butyle,
b) $R^6$ représente le groupe méthyle,
c) $R^5$ représente un atome d'hydrogène ou le groupe éthyle et
d) r est 0, 1 ou 2,

caractérisé en ce que l'on convertit en un composé de formule (II') un composé de formule (III)

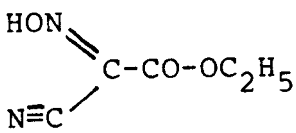

(III)

par, successivement,

- réduction en l'aminonitrile,
- introduction d'un groupe alkyle par acylation,
- addition d'un groupe alkylmercaptan sur le groupe cyano et cyclisation, et éventuellement oxydation supplémentaire du groupe thioalkyle et/ou hydrolyse du groupe ester.

**8.** Procédé pour la préparation d'un composé de formule (II') selon la revendication 7, dans lequel r est égal à 0 et $R^5$ représente le groupe éthyle.

**9.** Composé de formule (II')

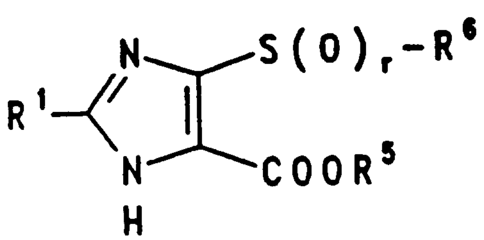

(II')

dans laquelle

a) $R^1$ représente le groupe n-butyle,
b) $R^6$ représente le groupe méthyle,
c) $R^5$ représente un atome d'hydrogène ou le groupe éthyle et
d) r est 0, 1 ou 2.

**10.** Composé de formule (II') selon la revendication 9, dans lequel r est égal à 0 et $R^5$ représente le groupe éthyle.